# EUROPÄISCHE PATENTSCHRIFT

(11) **EP 1 737 868 B1**
(45) Veröffentlichungstag und Bekanntmachung des Hinweises auf die Patenterteilung: **27.02.2008**
(21) Anmeldenummer: 04803495.3
(22) Anmeldetag: 03.12.2004
(51) Int. Cl.: C07D 495/04, C07D 333/20, C07C 211/08, C07C 209/68

(54) **STEREOSELEKTIVES VERFAHREN ZUR HERSTELLUNG VON CLOPIDOGREL**
STEREOSELECTIVE METHOD FOR THE PRODUCTION OF CLOPIDOGREL
PROCEDE STEREOSELECTIF POUR LA PRODUCTION DE CLOPIDOGREL

(30) Priorität: 20.04.2004 EP 04009280
(43) Veröffentlichungstag der Anmeldung: 03.01.2007
(73) Patentinhaber: Ratiopharm GmbH, 89070 Ulm (DE)
(72) Erfinder: STOHANDL, Jiri, 592 55 Bobrova (CZ); FRANTISEK, Jaroslav, 618 00 Brno (CZ); NESS, Winfried, 89077 Ulm (DE)
(74) Vertreter: Best, Michael
(86) Internationale Anmeldenummer: PCT/EP2004/013773
(87) Internationale Veröffentlichungsnummer: WO 2005/113559

(56) Entgegenhaltungen:
- WO-A-99/18110
- WO-A-03/035652
- M.M-L. CHAM AND J.B. ROBINSON: "Stereoisomeric Lactoyl-beta-methylcholine Iodides. Interaction with Cholinesterase and Acetylcholinesterase" J. MED. CHEM., Bd. 17, Nr. 10, 1974, Seiten 1057-60, XP002297328

## Beschreibung

Die vorliegende Erfindung betrifft ein stereoselektives Verfahren zur Herstellung von optisch reinen (2-Halogenphenyl)(6,7-dihydro-4H-thieno[3,2-c]pyridin-5-yl)essigsäuremethylestern, insbesondere von Clopidogrel, und Zwischenverbindungen zur Verwendung in diesem Verfahren. Das Verfahren ermöglicht es überraschenderweise, die optisch reinen Endverbindungen einfacher und in besserer Ausbeute zu erhalten.

(2-Halogenphenyl)(6,7-dihydro-4H-thieno[3,2-*c*]pyridin-5-yl)essigsäuremethylester und die Salze davon sind bekannte Thrombozytenaggregationshemmer. Insbesondere die Verbindung Clopidogrel, die beispielsweise in der EP-A 99 802 offenbart ist und die die Formel 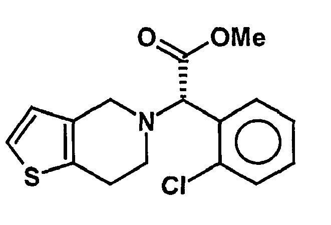 aufweist, ist ein hochwirksamer Arzneistoff. Clopidogrel ist die rechtsdrehende Verbindung (+)-[(S)-(2-Chlorphenyl)-(6,7-dihydro-4H-thieno[3,2-c]pyridin-5-yl)essigsäuremethylester, die in der Form des Hydrogensulfatsalzes vermarktet wird.

Eine Reihe von Verfahren zur Herstellung von Clopidogrel sind bekannt. In älteren Verfahren, die beispielsweise in der EP-A 99 802 und EP-A 420 706 beschrieben werden, wird die Verbindung 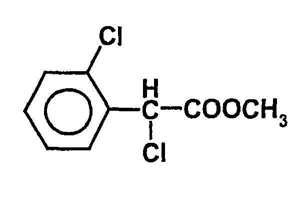 bzw. 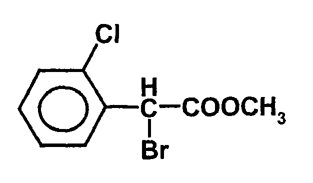 mit dem Thienopyridinrest 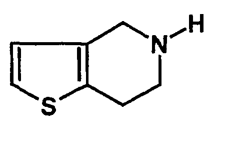 umgesetzt.

Diese Verfahren wurden bei nachfolgenden Entwicklungen jedoch als nachteilig angesehen, und nach den angegebenen Verfahrensbedingungen konnten nur verhältnismäßig niedrige Ausbeuten erzielt werden.

Dementsprechend gibt es eine Reihe von Druckschriften, die eine Verbesserung der Clopidogrelsynthese zum Gegenstand haben. Beispielsweise offenbart die WO 98/51681 ein Verfahren, das über die offenkettigen Zwischenverbindungen 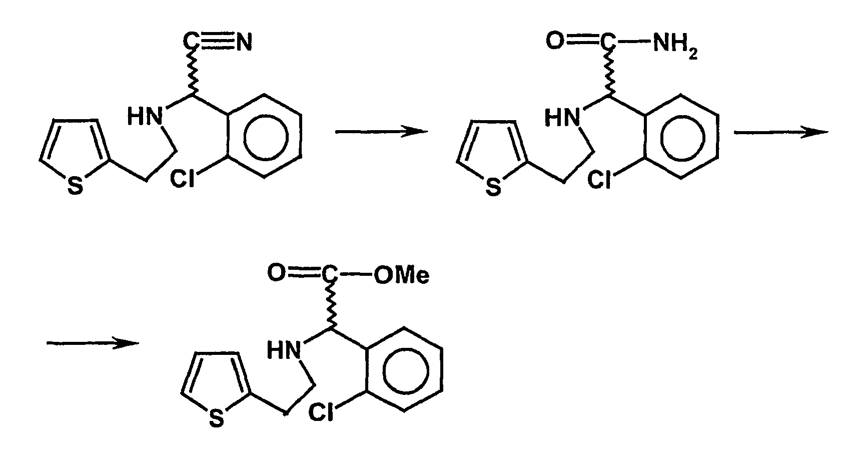 verläuft.

Da das Zielmolekül ein rechtsdrehendes Molekül ist, muß bei allen Verfahren zur Herstellung von Clopidogrel in einer bestimmten Verfahrensstufe eine Enantiomerentrennung erfolgen, und weitere Verfahrensstufen müssen enantiomerenrein erfolgen. Bei der Clopidogrelsynthese wird die Enantiomerentrennung in der Regel erst in einem relativ späten Verfahrensstadium durchgeführt, häufig erst nach dem letzten Syntheseschritt, und sie ist mit Schwierigkeiten verbunden. Insbesondere wenn die Enantiomerentrennung durch Umsetzung eines racemischen Gemisches mit einer enantiomerenreinen Verbindung und anschließender Ausfällung eines der gebildeten Diastereomeren erfolgt, tritt häufig keine selektive Ausfällung auf. Entweder wird das gewünschte Enantiomer nur in geringer Ausbeute oder in schlechter optischer Reinheit erhalten, so daß weitere Reinigungsschritte erforderlich sind. Dementsprechend wird in den Beispielen der WO 98/51681 bei der Enantiomerentrennung häufig ein optisch verunreinigtes Material erhalten, das erst nach weiterer Aufarbeitung die gewünschte hohe optische Reinheit aufweist.

Die Racemattrennung ist insbesondere dann schwierig, wenn sie an einer Verbindung erfolgt, bei der der Pyridinring bereits geschlossen ist. Dementsprechend erfolgt bei dem Verfahren der WO 98/51681 die Enantiomerentrennung bei einem offenkettigen Zwischenprodukt, z.B. dem Zwischenprodukt

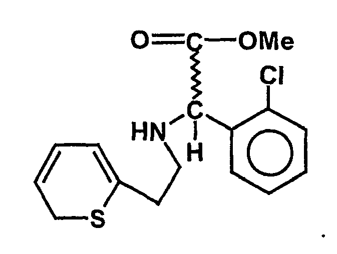

Viele neuere Verfahren schlagen vor, den Pyridinring erst in der letzten Verfahrensstufe nach der Enantiomerentrennung zu schließen.

Die WO 03/035652 offenbart ein Verfahren zur Herstellung von Clopidogrel, das über die Herstellung einer Zwischenverbindung der Formel 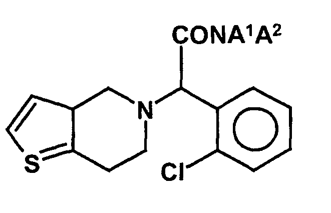 verläuft, wobei A¹ und A² unabhängig voneinander Wasserstoff oder C₁-C₄-Alkylgruppen bilden können oder einen Ring bilden können. Die Verbindung wird zuerst als Racemat hergestellt und anschließend in die einzelnen Enantiomere gespalten. Aus dem optisch aktiven Säureamid wird dann durch Umsetzung z.B. mit Methanol und Schwefelsäure Clopidogrel erhalten.

WO99/18110 offenbart ein Verfahren zur Herstellung von Clopidogrel worin 2-(Thien-2-yl) ethylamin mit einem Ester von (R)-Sulfonylessigsäure zu Methyl-(+)-(S)-Alpha-(2-(Thien-zyl)-ethylamino)-alpha-(2-chlorophenyl)-ersigsäure umgesetzt wird. Diese letzte Verbidung wird formyliert und anschließend zu Clopidogrel in Anwesendheid von Säure cyclisiert.

Der Erfindung liegt die Aufgabe zugrunde, eine neue Synthese für Verbindungen der Formel 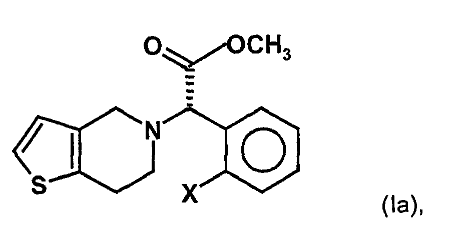 in der X ein Halogenatom darstellt, zur Verfügung zu stellen, die wirtschaftlich und mit wenigen Stufen durchgeführt werden kann.

Diese Aufgabe wird durch ein Verfahren zur Herstellung einer Verbindung der allgemeinen Formel (la)

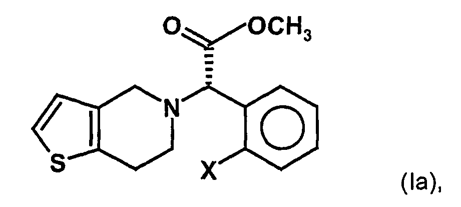

in der X ein Halogenatom darstellt, oder eines pharmazeutisch verträglichen Salzes davon, umfassend den Verfahrensschritt, daß eine Verbindung der Formel (II) 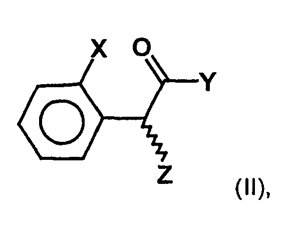 in der X wie vorstehend definiert ist und Y und Z unabhängig voneinander jeweils eine Abgangsgruppe darstellen, mit einem optisch aktiven Aminoalkohol zu einem ersten Gemisch aus Diastereomeren umgesetzt wird, gelöst.

Erfindungsgemäß wurde überraschend gefunden, daß bei der Umsetzung der Formel (II) 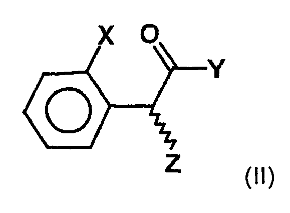 mit einem optisch aktiven Aminoalkohol ein erstes Gemisch aus Diastereomeren entsteht, bei dem eines der beiden Diastereomere angereichert ist, insbesondere entsteht ein Gemisch aus Diastereomeren, bei dem das Verhältnis des einen Diastereomeren zu dem zweiten Diastereomeren 2:1 oder höher ist. Bevorzugt ist das Verhältnis des einen Diastereomeren zu dem anderen Diastereomeren sogar 3:1 oder höher, insbesondere etwa 4:1 oder höher. Es ist erfindungsgemäß möglich, bereits von dem ersten Diastereomerengemisch das gewünschte Diastereomer abzutrennen und die weitere Clopidogrelsynthese nur mit diesem einen Diastereomer durchzuführen. Erfindungsgemäß bevorzugt wird jedoch das erste Diastereomerengemisch als solches weiterverarbeitet.

Überraschend wurde ebenfalls gefunden, daß bei der Umsetzung dieses ersten Gemischs aus Diastereomeren mit einer Verbindung der Formel (V) 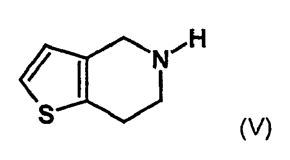 oder mit einer Verbindung der Formel (VII) 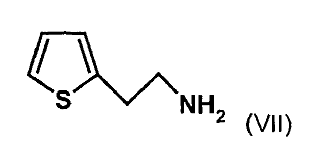 ein zweites Gemisch aus Diastereomeren entsteht, bei dem eines der beiden Diastereomeren noch stärker angereichert ist, als in dem ersten Gemisch aus Diastereomeren. Bevorzugt ist bei dem zweiten Gemisch aus Diastereomeren das Verhältnis des einen Diastereomeren zu dem zweiten Diastereomeren 3:1 oder höher, insbesondere 4:1 oder höher, am stärksten bevorzugt 9:1 oder höher. In einer besonders bevorzugten Ausführungsform enthält das zweite Gemisch aus Diastereomeren das gewünschte Diastereomer in einem Anteil von 95% oder mehr und das unerwünschte Diastereomer in einem Anteil von nur 5% oder weniger, stärker bevorzugt ist das gewünschte Diastereomer in einem Anteil von 98% oder mehr und das unerwünschte Diastereomer in einem Anteil von nur 2% oder weniger vorhanden, und es kann ein Verhältnis von gewünschtem Diastereomer zu unerwünschtem Diastereomer von etwa 99,5:0,5 oder besser erhalten werden.

Das Verhältnis von gewünschtem Diastereomer zu unerwünschtem Diastereomer ist in dem zweiten Diastereomerengemisch bevorzugt höher als in dem ersten Diastereomerengemisch.

Durch geeignete Wahl der optischen Aktivität des optisch aktiven Aminoalkohols kann entweder das eine oder das andere Diastereomer im Überschuß erhalten werden.

Durch die Umsetzung der Verbindung der Formel (II) 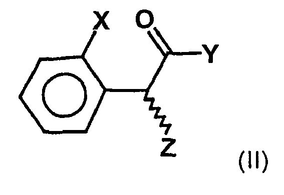 mit einem optisch aktiven Aminoalkohol kann also bereits in einem sehr frühen Verfahrensstadium ein Gemisch aus Diastereomeren erhalten werden, das das gewünschte Diastereomer im Überschuß enthält, das dann weiter zu der Verbindung der Formel (Ia) 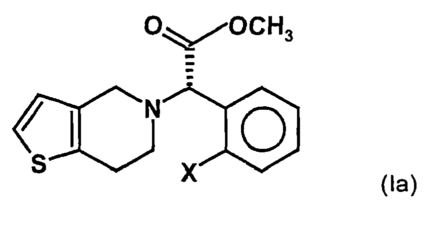 umgesetzt werden kann. Am Ende der Umsetzung erhält man entweder Clopidogrel in der gewünschten sterischen Anordnung oder ein Gemisch aus Enantiomeren, in dem das gewünschte Enantiomer (Clopidogrel) bereits in hohem Überschuß vorliegt. Entweder ist keine Racematspaltung mehr erforderlich oder die Racematspaltung kann einfacher und in besserer Ausbeute durchgeführt werden.

Erfindungsgemäß stellt der Rest X ein Halogenatom dar, insbesondere ein Fluor-, Chlor-, Brom- oder lodatom, besonders bevorzugt ein Chloratom.

Erfindungsgemäß stellen die Reste Y und Z übliche Abgangsgruppen dar, wie sie im Stand der Technik bekannt sind, und beispielsweise in Peter Sykes, Reaktionsmechanismen der organischen Chemie, 9. Auflage, Weinheim 1988 beschrieben werden. Bevorzugte Abgangsgruppen sind die Halogenatome, insbesondere lod-, Chlor-, Brom- oder Fluoratome, ein Tosylatrest, ein Triflatrest oder ein Brosylatrest.

Der optisch aktive Aminoalkohol ist nicht besonders eingeschränkt. Bevorzugt sind optisch aktive Aminoalkohole, bei denen zumindest ein optisch aktives Zentrum in räumlicher Nähe zu der Hydroxylgruppe angeordnet ist, die an die Verbindung der Formel II koppelt, bevorzugt nicht weiter als eine, zwei oder drei Bindungen von der Hydroxylgruppe entfernt.

Besonders bevorzugt wird ein optisch aktiver Aminoalkohol der Formel (III) 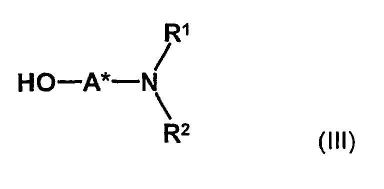 verwendet, wobei A* einen Kohlenwasserstoffrest mit 1 bis 30 Kohlenstoffatomen darstellt, der bis zu 5 Heteroatome enthalten kann, ausgewählt aus Stickstoff-, Sauerstoff-, Schwefel- und Halogenatomen, der mit bis zu 5 Substituenten substituiert sein kann, ausgewählt aus Hydroxylgruppen, Oxogruppen, Cyanogruppen und Nitrogruppen, und der eine oder mehrere optisch aktive Einheiten aufweist, und
R¹ und R² jeweils unabhängig voneinander Wasserstoffatome oder Kohlenwasserstoffreste mit 1 bis 20 Kohlenstoffatomen darstellen, die jeweils bis zu 4 Heteroatome, ausgewählt aus Stickstoff-, Sauerstoff-, Schwefel- und Halogenatomen, und bis zu 5 Substituenten, ausgewählt aus Hydroxylgruppen, Oxogruppen, Cyanogruppen und Nitrogruppen, aufweisen können, oder
einer oder beide der Reste R¹ und R² mit einem Kohlenstoffatom oder einem Heteroatom des Rests A* einen 5- bis 10-gliedrigen gesättigten oder ungesättigten Ring bildet, der neben dem Stickstoffatom gegebenenfalls noch 1 bis 3 weitere Heteroatome, ausgewählt aus Stickstoff-, Sauerstoff- und Schwefelatomen, als Ringglieder enthält, und der mit bis zu 5 Substituenten, ausgewählt aus C₁-C₆-Alkylresten, C₂-C₆-Alkenylresten, C₁-C₆-Alkoxyresten, C₅-C₁₀-Arylresten (bevorzugt C₆-C₁₀-Arylresten), C₅-C₁₀-Heteroarylresten, C₃-C₈-Cycloalkylresten, C₂-C₈-Heterocycloalkyfresten, Halogenatomen, Hydroxylgruppen, Oxogruppen, Cyanogruppen und Nitrogruppen, substituiert sein kann.

Bevorzugt weisen die optisch aktiven Aminoalkohole, die erfindungsgemäß verwendet werden können, nur eine einzige Hydroxylgruppe auf, damit Konkurrenzreaktionen bei der Umsetzung mit der Verbindung der Formel (II) vermieden werden.

Die Reste R¹ und R² stellen erfindungsgemäß bevorzugt unabhängig voneinander einen C₁-C₆-Alkylrest, einen C₆-C₁₀-Arylrest (bevorzugt C₆-C₁₀-Arylrest), einen C₅-C₁₀-Heteroarylrest, einen C₃-C₈-Cycloalkylrest oder C₂-C₈-Heterocycloalkylrest dar oder bilden zusammen mit dem Stickstoffatom, an das sie gebunden sind, einen gesättigten oder ungesättigten Ring mit 2 bis 8 Kohlenstoffatomen, der gegebenenfalls mit einer C₁-C₆-Alkylgruppe oder einem Halogenatom substituiert sein kann und neben dem Stickstoffatom 1 oder 2 weitere Heteroatome, ausgewählt aus Schwefelatomen, Stickstoffatomen und Sauerstoffatomen, enthalten kann.

Im Rahmen dieser Anmeldung bedeutet ein * an einer Molekülgruppierung, daß diese Molekülgruppierung optische Aktivität aufweist.

Im Rahmen der Anmeldung sind folgende Reste bevorzugt:

Bevorzugte C₁-C₆-Alkylreste sind Methyl-, Ethyl-, Isopropyl-, n-Propyl-, n-Butyl-, Pentyl- und Hexylreste.

Bevorzugte Arylreste sind Phenyl- oder Naphthylreste, insbesondere Phenylreste, die gegebenenfalls mit 1 bis 3 C₁-C₃-Alkylresten substituiert sein können.

Bevorzugte C₅-C₁₀-Heteroarylreste weisen ein oder mehrere, bevorzugt 1 oder 2, Heteroatome auf, insbesondere Stickstoff-, Sauerstoff- und/oder Schwefelatome. Beispiele sind Imidazolyl-, Oxazolyl-, Isoxazolyl-, Thiazolyl-, 1,2,3-Triazolyl-, Pyridinyl-, Pyridazinyl-, Pyrimidinyl-, Pyrazinyl- und 1-H-Pyrazonylreste.

Bevorzugte C₃-C₈-Cycloalkylreste sind Cyclopropyl-, Cyclobutyl-, Cyclopentyl-, Cyclohexyl-, Cycloheptyl- und Cyclooctylreste.

Bevorzugte C₂-C₈-Heterocycloalkylreste sind Cycloalkylreste mit 2 bis 8 Kohlenstoffatomen, die ein oder mehrere Heteroatome aufweisen. Bevorzugte Heterocycloalkylreste weisen ein oder mehrere, bevorzugt 1 oder 2, Heteroatome auf, insbesondere Stickstoff-, Sauerstoff- und/oder Schwefelatome. Beispiele sind Oxiranyl-, Aziridinyl-, Azetidinyl-, Tetrahydrofuranyl-, Pyrrolyl- und Pyranylreste.

Bevorzugte C₂-C₆-Alkenylreste sind Ethenyl-, Propenyl-, Butenyl- und Pentenylreste.

Bevorzugte C₁-C₆-Alkoxyreste sind Methoxy-, Ethoxy- und Propoxyreste, insbesondere Methoxyreste.

Arylreste, Heteroarylreste, Cycloalkylreste und Heterocycloalkylreste können unsubstituiert oder substituiert sein. Falls diese Reste substituiert sind, sind sie bevorzugt mit 1 bis 3 Substituenten substituiert, ausgewählt aus C₁-C₃-Alkyl-, C₁-C₃-Alkoxyresten und Halogenatomen.

Cycloalkylreste und Heterocycloalkylreste können gesättigt oder ungesättigt sein. Ungesättigte Reste weisen bevorzugt eine oder zwei Doppelbindungen auf.

Die Umsetzung der Verbindung der Formel (II) 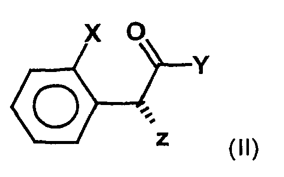 mit dem bevorzugten optisch aktiven Aminalkohol der Formel (III) 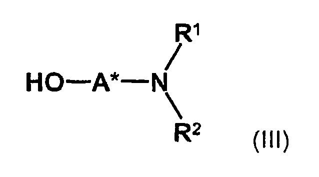 ergibt ein Gemisch aus den Diastereomeren (IVa) 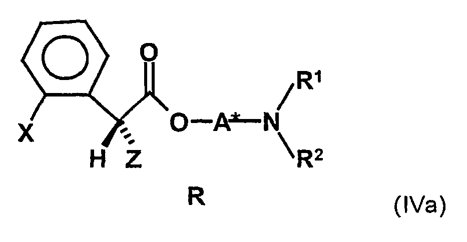 und (IVb)

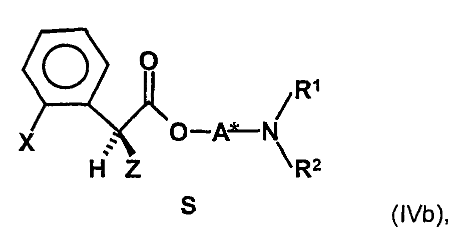

in dem X, Z, A*, R¹ und R² wie vorstehend definiert sind, das als erstes Diastereomerengemisch bezeichnet wird.

Das erste Diastereomerengemisch enthält einen Überschuß bevorzugt an dem Diastereomeren der Formel (IVa) 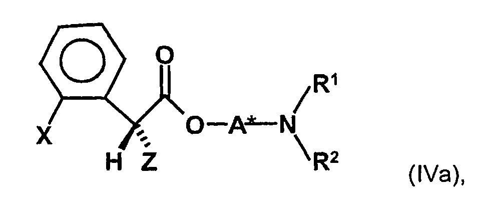 und der Überschuß ist in der Regel etwa 2:1 oder höher.

Das erste Diastereomerengemisch wird bevorzugt mit der Verbindung der Formel (V) 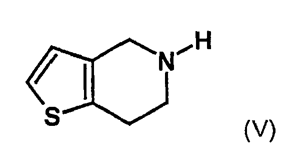 oder der Verbindung der Formel (VII) 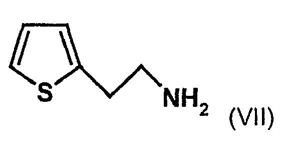 umgesetzt, wodurch ein Gemisch aus Diastereomeren der Formel (VIa) 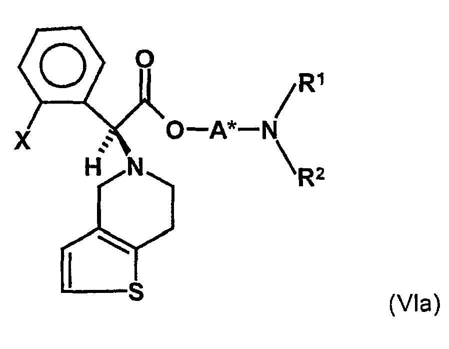 und (VIb) 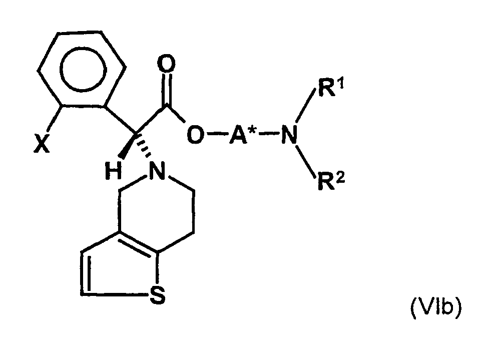 bzw. der Formel (VIIIa) 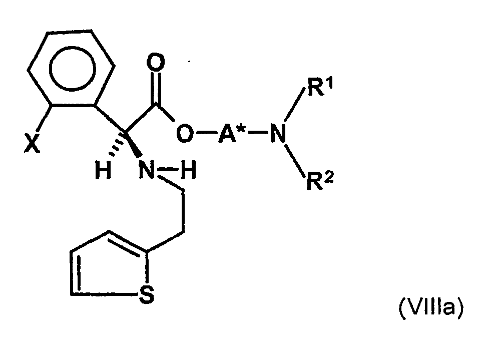 und der Formel (VIIIb) 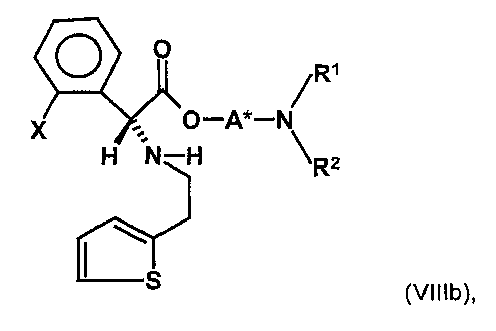 erhalten wird, und diese Diastereomerengemische werden jeweils als zweites Diastereomerengemisch bezeichnet. Das Verhältnis des Diastereomers der Formel (VIa) zu dem Diastereomer der Formel (Vlb) bzw. des Diastereomers der Formel (VIIIa) zu dem Diastereomer der Formel (VIIIb) beträgt bevorzugt etwa 3 oder mehr, stärker bevorzugt etwa 4 oder mehr und kann besonders bevorzugt bis zu 9 oder sogar noch darüber liegen.

In einer besonders bevorzugten Ausführungsform enthält das zweite Gemisch aus Diastereomeren das gewünschte Diastereomer in einem Anteil von 95% oder mehr und das unerwünschte Diastereomer in einem Anteil von nur 5% oder weniger, stärker bevorzugt ist das gewünschte Diastereomer in einem Anteil von 98% oder mehr und das unerwünschte Diastereomer in einem Anteil von nur 2% oder weniger vorhanden, und es kann ein Verhältnis von gewünschtem Diastereomer zu unerwünschtem Diastereomer von etwa 99,5:0,5 oder besser erhalten werden.

In den vorstehenden Formeln sind die Reste X, Z, A*, R¹ und R² wie vorstehend definiert.

In einer bevorzugten Ausführungsform ist der Rest der Formel HO-A* ein Rest der Formel 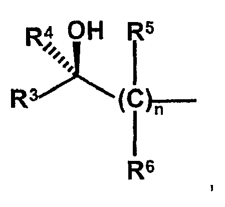 wobei die Reste R³ bis R⁶ jeweils unabhängig voneinander Wasserstoffatome oder Kohlenwasserstoffreste mit 1 bis 20 Kohlenstoffatomen darstellen, die jeweils bis zu 4 Heteroatome, ausgewählt aus Stickstoff-, Sauerstoff-, Schwefel- und Halogenatomen, und bis zu 5 Substituenten, ausgewählt aus Hydroxylgruppen, Oxogruppen, Cyanogruppen und Nitrogruppen, aufweisen können, oder
einer oder zwei der Reste R³ bis R⁶ mit dem Rest R¹ bzw. dem Rest R² einen 5- bis 10-gliedrigen gesättigten oder ungesättigten Ring bilden, der neben dem Stickstoffatom gegebenenfalls noch 1 bis 3 weitere Heteroatome, ausgewählt aus Stickstoff-, Sauerstoff- und Schwefelatomen, als Ringglieder enthält, und der mit bis zu 5 Substituenten, ausgewählt aus C₁-C₆-Alkyfresten, C₂-C₆-Alkenylresten, C₁-C₆-Alkoxyresten, C₅-C₁₀-Arylresten (bevorzugt C₆-C₁₀-Arylresten), C₃-C₈-Cycloalkylresten, C₂-C₈-Heterocycloalkylresten, C₅-C₁₀-Heteroarylresten, Halogenatomen, Hydroxylgruppen, Oxogruppen, Cyanogruppen und Nitrogruppen, substituiert sein kann, und n eine ganze Zahl von 1 bis 3 darstellt.

In der vorstehenden Formel sind die Reste R⁵ und R⁶ bevorzugt unabhängig voneinander aus Wasserstoffatomen und C₁-C₆-Alkylresten ausgewählt, wobei die bevorzugten C₁-C₆-Alkylreste die gleichen sind, wie vorstehend ausgeführt. Am stärksten bevorzugt ist nur einer der Reste R⁵ und R⁶ von einem Wasserstoff verschieden, und in der am stärksten bevorzugten Ausführungsform sind alle Reste R⁵ und R⁶ Wasserstoffatome. Der Index n ist bevorzugt 1 oder 2, stärker bevorzugt 1. Der Rest R³ ist bevorzugt ein C₁-C₆-Alkyl-, C₁-C₆-Alkoxy-, C₅-C₁₀-Aryl- (bevorzugt C₆-C₁₀-Aryl-), C₅-C₁₀-Heteroaryl-, C₂-C₈-Heterocycloalkyl- oder C₃-C₈-Cycloalkylrest, wobei die Alkyl-, Alkoxy-, Aryl-, Heteroaryl-, Heterocycloalkyl- und Cycloalkylreste stärker bevorzugt die gleiche Bedeutung haben, wie vorstehend ausgeführt. Am stärksten bevorzugt ist der Rest R³ ein C₁-C₆-Alkylrest.

Erfindungsgemäß ebenfalls bevorzugt ist der Rest R³ mit dem Rest R² zu einem Ring verbunden, der 5 bis 10 Ringatome aufweist und der neben dem Stickstoffatom, an das der Rest R² gebunden ist, noch 1 bis 3 weitere Heteroatome, ausgewählt aus Sauerstoff-, Stickstoff- und Schwefelatomen, aufweisen kann, und der gesättigt oder einfach oder zweifach ungesättigt sein kann und der 1 bis 3 Substituenten aufweisen kann, ausgewählt aus C₁-C₆-Alkylresten, C₁-C₆-Alkoxyresten, C₅-C₁₀-Arylresten (bevorzugt C₆-C₁₀-Arylresten), C₃-C₈-Cycloalkylresten, C₂-C₈-Heterocycloalkylresten, C₅-C₁₀-Heteroarylresten und Halogenatomen.

Der Rest R⁴ ist besonders bevorzugt ein Wasserstoffatom oder ein C₁-C₆-Alkylrest, am stärksten bevorzugt ein Wasserstoffatom.

Die Reste R³ und R⁴ sind bevorzugt verschieden, so daß das optisch aktive Zentrum an dem Kohlenstoffatom sitzt, das auch die Hydroxylgruppen trägt. Es ist aber auch möglich, daß die Reste R³ und R⁴ gleich sind, z.B. beide Wasserstoffatome darstellen und das optisch aktive Zentrum an einem anderen Kohlenstoffatom sitzt und z.B. dadurch bewirkt wird, daß an diesem Kohlenstoffatom die Reste R⁵ und R⁶ verschieden sind. Bevorzugt ist das Substitutionsmuster so gewählt, daß der vorstehend angegebene Rest HO-A*, nämlich

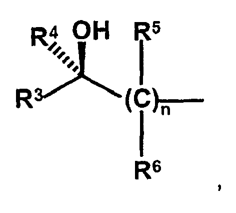

in der R-Konfiguration vorliegt, da sich hierdurch in den späteren Reaktionsschritten Diastereomerengemische ergeben, in denen dasjenige Diastereomer im Überschuß vorliegt, das Clopidogrel in der gewünschten sterischen Konfiguration ergibt.

Wird die Verbindung der Formel (II) mit dem erfindungsgemäß bevorzugten optisch aktiven Aminoalkohol der Formel (III) umgesetzt, ergibt sich ein erstes Gemisch aus den Diastereomeren der Formel (IVa) 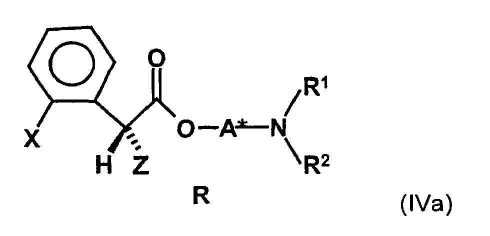 und der Formel (IVb)

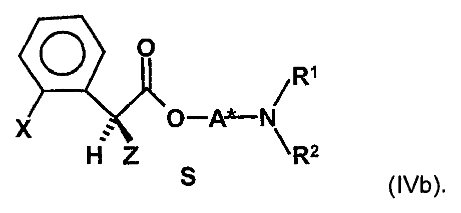

Für die besonders bevorzugte Ausführungsform, in der der Rest HO-A* ein Rest der Formel 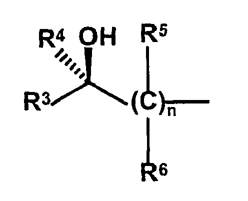 ist, ergibt sich ein Diastereomerengemisch aus den Verbindungen der Formel 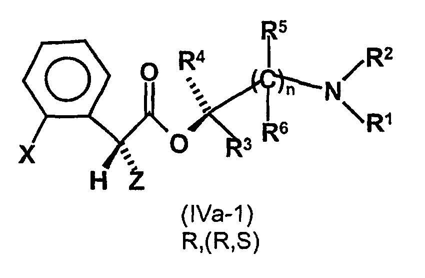 und 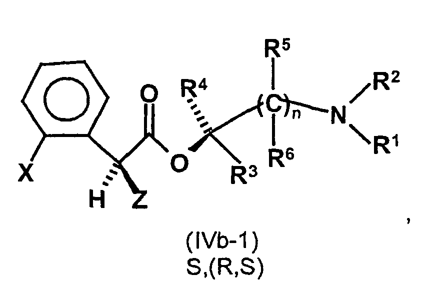 wobei die Reste X, Z, R¹ bis R⁶ und der Index n wie vorstehend definiert sind und das als erstes Diastereomerengemisch bezeichnet wird.

Erfindungsgemäß wird bei Einsatz eines optisch aktiven Aminoalkohols mit der sterischen Konfiguration (in der R-Konfiguration) 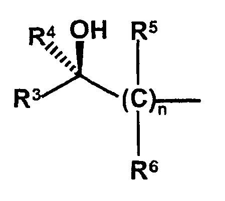 die R,(R,S)-Verbindung der Formel (IVa-1) in einem Überschuß zu der S,(R,S)-Verbindung der Formel (IVb-1) gebildet, und zwar ist das Verhältnis zwischen der R,(R,S)- und der S,(R,S)-Verbindung bevorzugt 2:1 oder höher, stärker bevorzugt 3:1 oder höher und insbesondere etwa 4:1 oder höher.

Es ist erfindungsgemäß möglich, bereits eine Enantiomerentrennung bei dem ersten Diastereomerengemisch durchzuführen und die weitere Clopidogrelsynthese nur mit dem R,(R,S)-Diastereomeren der Formel (IVa-1) weiterzuführen. Die Trennung der Diastereomeren kann auf sich bekannte Art und Weise durch Kristallisation oder Chromatographieverfahren erfolgen. Erfindungsgemäß bevorzugt wird jedoch das erste Diastereomerengemisch als solches mit einer Verbindung der Formel 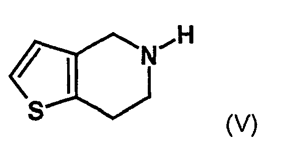 oder mit einer Verbindung der Formel (VII) 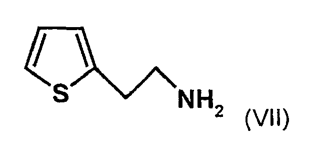 umgesetzt. Bei einer derartigen Umsetzung mit der Verbindung der Formel (V) 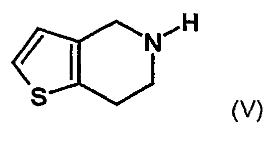 ergibt sich das Gemisch aus den Diastereomeren 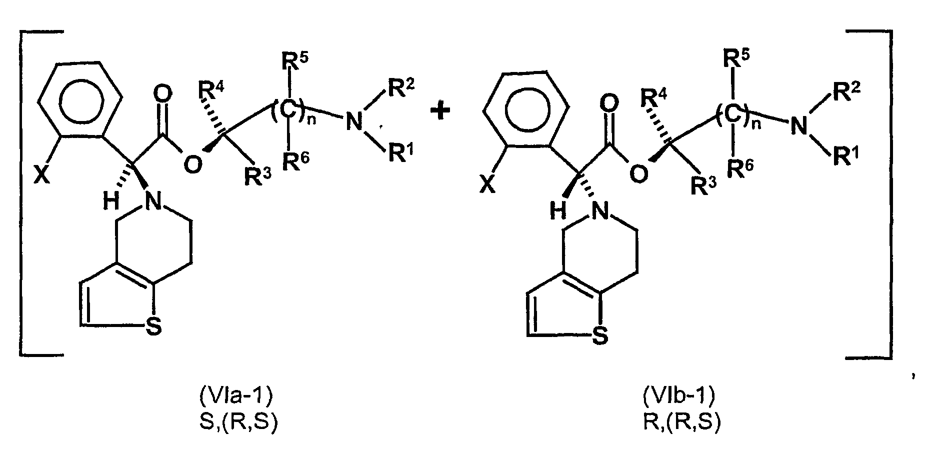 wobei die Reste X, R¹ bis R⁶ und der Index n wie vorstehend definiert sind und das als zweites Diastereomerengemisch bezeichnet wird. Hierbei handelt es sich um das Gemisch aus den Diastereomeren 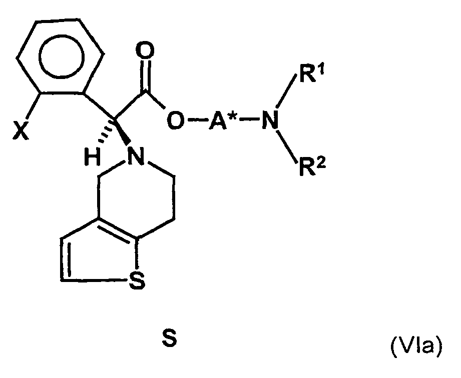 und 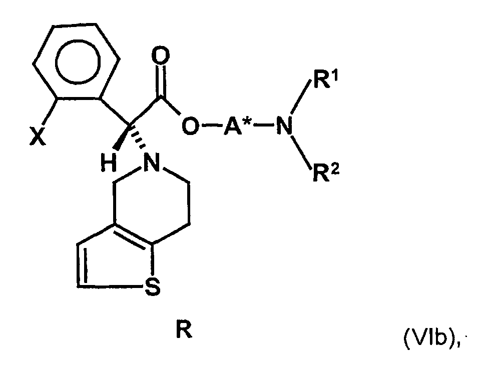 in denen der Rest -O-A* ein Rest der Formel 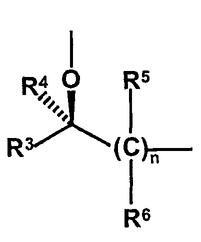 darstellt. Die S,(R,S)-Verbindung der Formel (VIa-1) liegt bevorzugt in einem Überschuß zu der R,(R,S)-Verbindung der Formel (VIb-1) vor, und überraschenderweise hat sich der diastereomere Überschuß durch die Umsetzung mit der Verbindung der Formel (V) 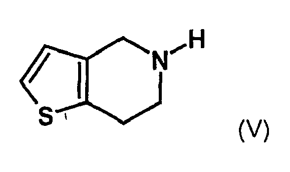 nochmals erhöht, so daß das Verhältnis der Verbindung der Formel (VIa-1) zu der Verbindung der Formel (VIb-1) bevorzugt 3:1 oder höher ist, stärker bevorzugt 4:1 oder höher ist. Gemäß der am stärksten bevorzugten Ausführungsform der Erfindung wird sogar ein Verhältnis der Diastereomeren von 9:1 oder höher erreicht. In einer besonders bevorzugten Ausführungsform enthält das zweite Gemisch aus Diastereomeren das gewünschte Diastereomer in einem Anteil von 95% oder mehr und das unerwünschte Diastereomer in einem Anteil von nur 5% oder weniger, stärker bevorzugt ist das gewünschte Diastereomer in einem Anteil von 98% oder mehr und das unerwünschte Diastereomer in einem Anteil von nur 2% oder weniger vorhanden, und es kann ein Verhältnis von gewünschtem Diastereomer zu unerwünschtem Diastereomer von etwa 99,5:0,5 oder besser erhalten werden.

Wir das Gemisch aus Diastereomeren (erstes Diastereomerengemisch) 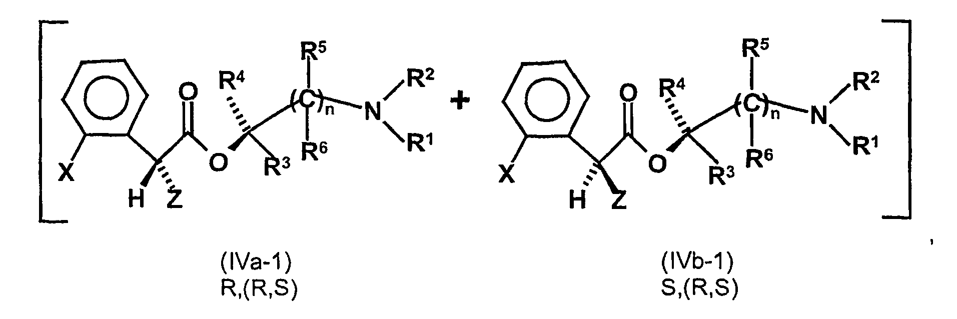 mit einer Verbindung der Formel (VII) 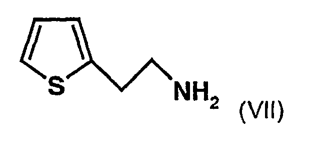 umgesetzt, entsteht entsprechend ein Gemisch aus den Diastereomeren (zweites Diastereomerengemisch)

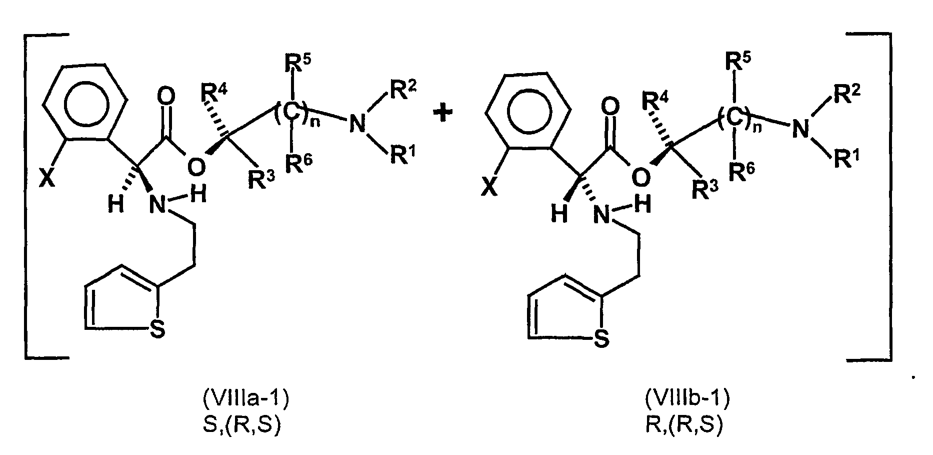

In diesem Diastereomerengemisch ist das Diastereomere S,(R,S) der Formel (VIIIa-1) bevorzugt in einem Überschuß zu dem Diastereomeren R,(R,S) der Formel (VIIIb-1) vorhanden (sofern das "richtige" Enantiomer des Aminoalkohols eingesetzt wird, andernfalls ist das Verhältnis der Diastereomeren vertauscht), und auch hier hat sich überraschenderweise der Überschuß des Diastereomeren der Formel (Vllla-1) gegenüber dem Diastereomeren der Formel (VIIIb-1) erhöht und ist bevorzugt 3:1 oder höher, stärker bevorzugt 4:1 oder höher bzw. 9:1 oder höher. In einer besonders bevorzugten Ausführungsform enthält das zweite Gemisch aus Diastereomeren das gewünschte Diastereomer in einem Anteil von 95% oder mehr und das unerwünschte Diastereomer in einem Anteil von nur 5% oder weniger, stärker bevorzugt ist das gewünschte Diastereomer in einem Anteil von 98% oder mehr und das unerwünschte Diastereomer in einem Anteil von nur 2% oder weniger vorhanden, und es kann ein Verhältnis von gewünschtem Diastereomer zu unerwünschtem Diastereomer von etwa 99,5:0,5 oder besser erhalten werden.

Das Gemisch aus den Diastereomeren (zweites Diastereomerengemisch) 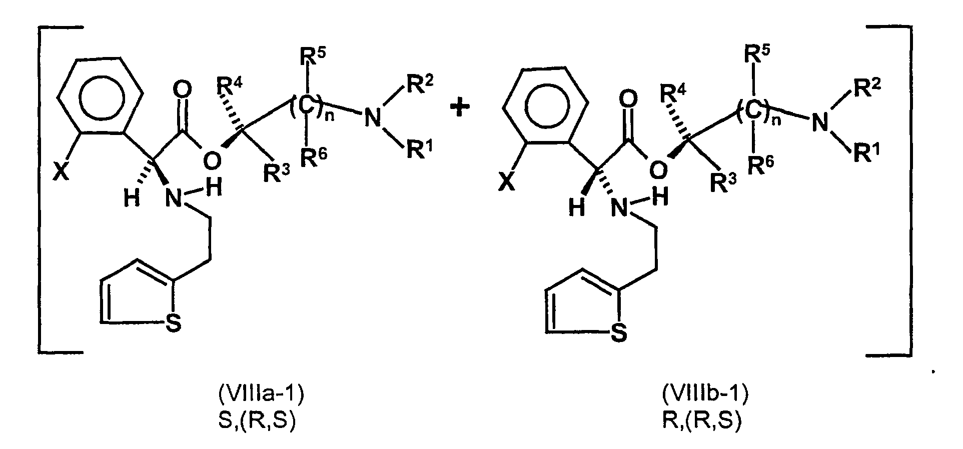 kann auf übliche Art und Weise zu dem Gemisch aus Diastereomeren (zweites Diastereomerengemisch) 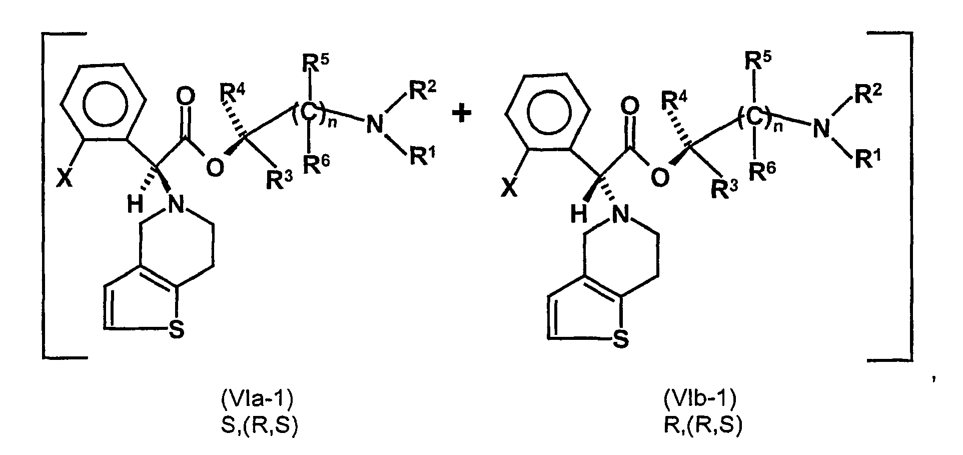 umgesetzt werden, wie es im Stand der Technik prinzipiell bekannt ist.

Bezüglich dieser Reaktion kann auf die in der Einleitung diskutierten Druckschriften und die nachstehenden Ausführungen zur Ringschlußreaktion des Tetrahydrothienopyridins verwiesen werden.

Der am stärksten bevorzugte optisch aktive Aminoalkohol ist (R)-1-(Dimethylamino)-2-propanol ((R)-Dimepranol). Dieser optisch aktive Aminoalkohol ist kommerziell erhältlich, erfindungsgemäß wurde jedoch gefunden, daß er auch auf einfache Art und Weise durch optische Auflösung des entsprechenden racemischen Aminoalkohols mit Di-O-Benzoyl-L-(-)-weinsäure erhalten werden kann. Dies erhöht die Wirtschaftlichkeit des erfindungsgemäßen Verfahrens. Auch andere optisch aktive Alkohole, die in dem erfindungsgemäßen Verfahren eingesetzt werden können, können auf diese Art und Weise aus dem entsprechenden racemischen Alkohol gewonnen werden.

Mit dem erfindungsgemäß bevorzugten optisch aktiven Aminoalkohol (R)-Dimepranol ergeben sich für das erste Diastereomerengemisch und das zweite Diastereomerengemisch die folgenden Formeln:

Erstes Diastereomerengemisch: 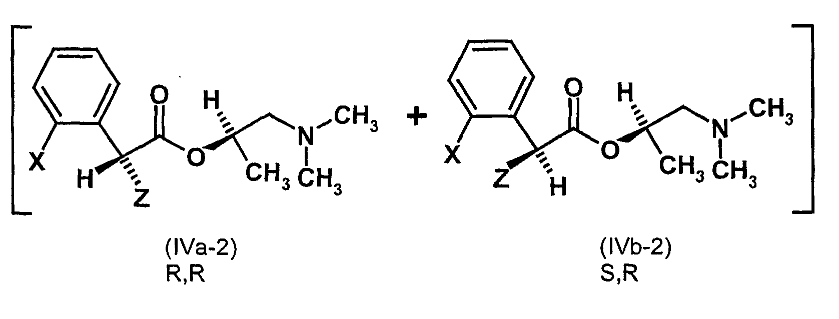 zweites Diastereomerengemisch: 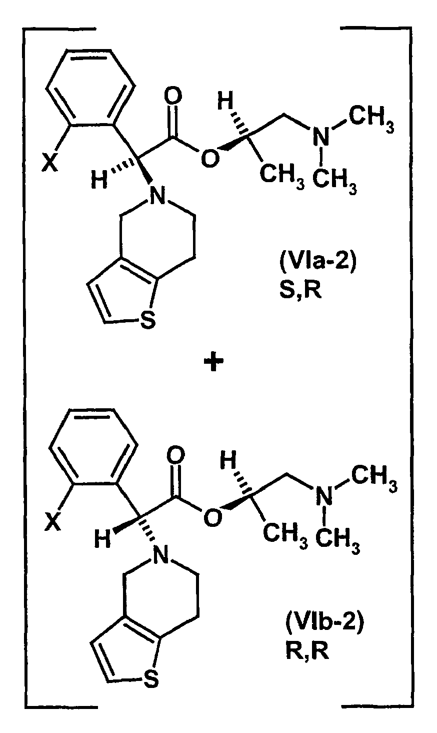 bzw. mit der offenkettigen Verbindung:

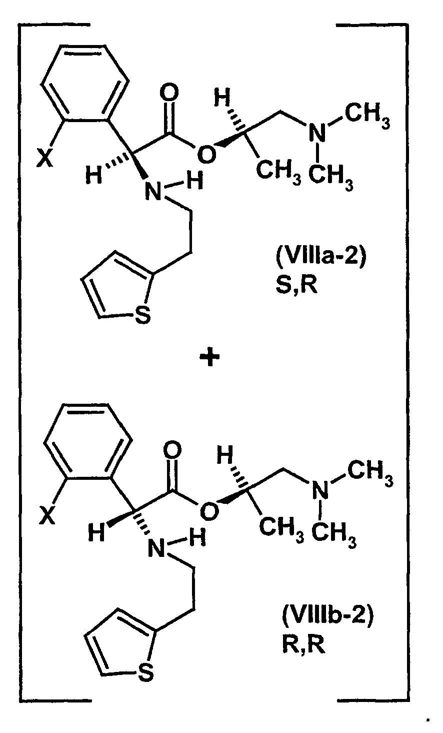

Weitere erfindungsgemäß bevorzugte optisch aktive Aminoalkohole sind die Verbindungen der Formeln (im folgenden dargestellt ohne Angabe der Stereochemie): 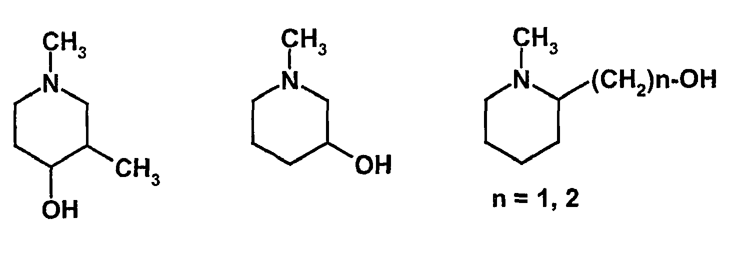 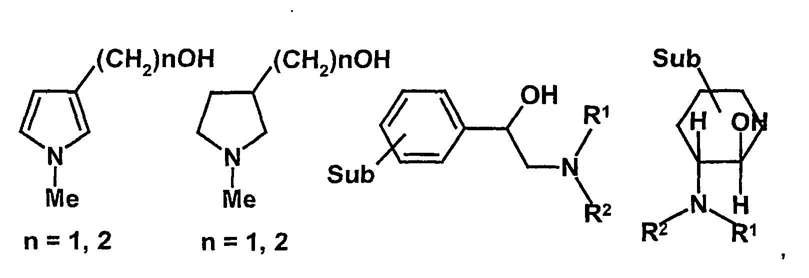 wobei die Reste R¹ und R² wie vorstehend definiert sind und "Sub" die Bedeutung hat, daß der Ring an einer beliebigen Stelle mit einem C₁-C₃-Alkylrest substituiert sein kann.

Die Verbindungen 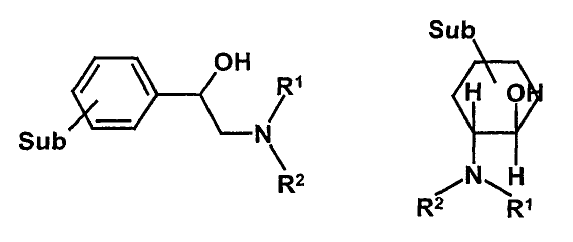 können leicht aus den entsprechenden Derivaten von substituiertem Styroloxid oder substituiertem Cyclohexenoxid hergestellt und in ihre einzelnen Enantiomere aufgetrennt werden, wie es in "Optical Resolution via Diastereoisomeric Salts Formation", David Kozma, Ed., CRC Press, London, New York, Washington D.C. beschrieben ist.

Erfindungsgemäß ebenfalls bevorzugt kann der Rest HO-A* ein Rest der Formel 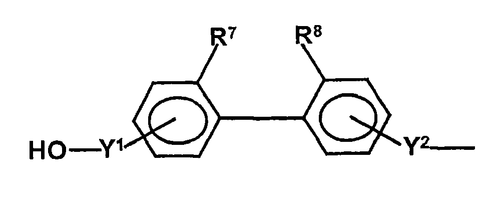 oder 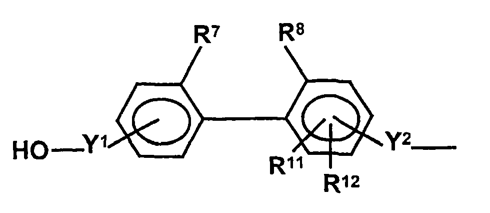 darstellen, wobei Y¹ einen Rest 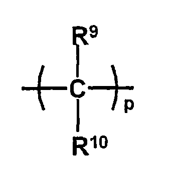 darstellt, und Y² einen Rest 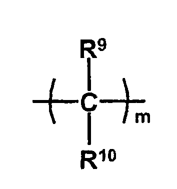 darstellt, in dem R⁹ und R¹⁰ unabhängig voneinander aus Wasserstoffatomen und C₁-C₆-Alkylresten ausgewählt sind und die Reste R⁷ und R⁸ Gruppen darstellen, die die freie Drehbarkeit der beiden Phenylgruppen relativ zueinander verhindern, R¹¹ und R¹² unabhängig voneinander Wasserstoffatome, C₁-C₄-Alkoxyreste, Halogenatome, Cyanoreste, C₁-C₆-Alkoxycarbonylreste oder C₁-C₆-Alkylreste darstellen oder R¹¹ und R¹² zusammen mit dem Benzolring, an den sie gebunden sind, eine kondensierte Ringstruktur bilden, die ausgewählt ist aus einer 1,3-Benzodioxolyl-, α-Naphthyl-, β-Naphthyl-, Tetrahydronaphthyl-, Benzimidazolyl- und Benztriazolylstruktur, m eine ganze Zahl von 0 bis 2 ist und p 1 oder 2 darstellt.

Besonders bevorzugt sind alle Reste R⁹ und R¹⁰ Wasserstoffatome oder nur ein Rest ist von einem Wasserstoffatom verschieden und ein C₁-C₆-Alkylrest, bevorzugt ein C₁-C₃-Alkylrest.

Besonders bevorzugt ist einer der Reste R⁷ und R⁸ ausgewählt aus einer C₁-C₆-Alkylgruppe, einem Halogenatom und einer Cyanogruppe, und der zweite Rest ist ausgewählt aus einer C₃-C₆-verzweigten Alkylgruppen, bevorzugt eine tertiäre Butylgruppe, einem Halogenatom und einer Cyanogruppe. Voluminöse Halogenatome wie lodatome sind bevorzugt.

Bei diesem Rest wird die optische Aktivität dadurch hergestellt, daß zumindest einer der Reste R⁷ und R⁸, bevorzugt beide Reste R⁷ und R⁸, großvolumige Reste darstellen, wie verzweigte Alkylgruppen (insbesondere eine verzweigte C₃-C₆-Alkylgruppe), Halogenatome oder Cyanogruppen und dadurch die Rotation der beiden Phenylringe verhindert wird.

In einer weiteren erfindungsgemäß bevorzugten Ausführungsform ist die Verbindung der Formel 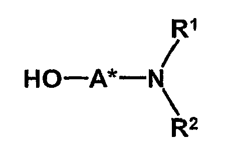 eine Verbindung der Formel

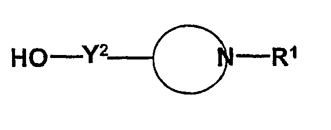

in der Y² wie vorstehend definiert ist, R¹ wie vorstehend definiert ist und bevorzugt ein Wasserstoffatom oder einen Kohlenwasserstoffrest mit 1 bis 20 Kohlenstoffatomen darstellt, der bis zu 4 Heteroatome, ausgewählt aus Stickstoff-, Sauerstoff-, Schwefel- und Halogenatomen, und bis zu 5 Substituenten, ausgewählt aus Hydroxylgruppen, Oxogruppen, Cyanogruppen und Nitrogruppen, aufweisen kann, und die Gruppe 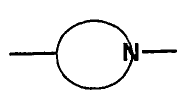 einen 5- bis 10-gliedrigen gesättigten oder ungesättigten Ring darstellt, der neben dem Stickstoffatom gegebenenfalls noch 1 bis 3 weitere Heteroatome, ausgewählt aus Stickstoff-, Sauerstoff- und Schwefelatomen, als Ringglieder enthält, und der mit bis zu 5 Substituenten, ausgewählt aus C₁-C₆-Alkylresten, C₁-C₆-Alkoxyresten, C₂-C₆-Alkenylresten, C₅-C₁₀-Arylresten (insbesondere C₆-C₁₀-Arylresten), C₅-C₁₀-Heteroarylresten, C₃-C₆-Cycloalkylresten, C₂-C₈-Heterocycloalkylresten, Halogenatomen, Hydroxylgruppen, Oxogruppen, Cyanogruppen und Nitrogruppen, substituiert sein kann.

Bevorzugt ist die Gruppe 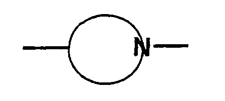 eine Gruppe

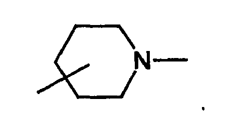

Die Umsetzungsprodukte der vorstehend genannten optisch aktiven Aminoalkohole mit den Verbindungen der Formel (II) zu dem ersten Diastereomerengemisch und die Umsetzungsprodukte aus dem ersten Diastereomerengemisch mit einer Verbindung der Formel (V) 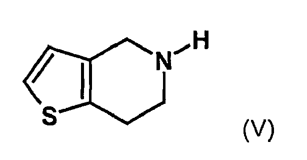 oder einer Verbindung der Formel (VII) 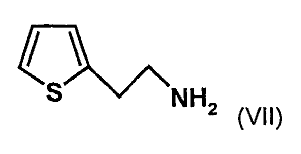 zu dem zweiten Diastereomerengemisch, sind neue Verbindungen, und die Erfindung betrifft auch die entsprechenden Diastereomerengemische mit beliebigen Anteilen der einzelnen Diastereomeren und die einzelnen isolierten Diastereomeren aus diesen Diastereomerengemischen.

Die Weiterverarbeitung des zweiten Diastereomerengemischs zu dem Endprodukt der Formel (la) 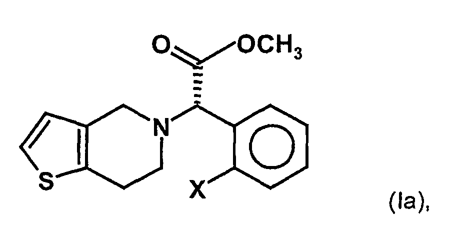 insbesondere zu Clopidogrel 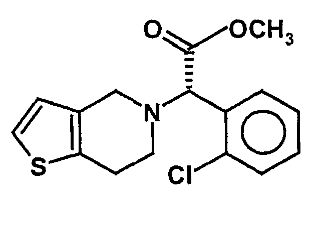 erfolgt auf dem Fachmann an und für sich bekannte Art und Weise. Folgende Verfahrensführungen sind dabei prinzipiell möglich, wobei die folgenden Formeln mit dem bevorzugten optisch aktiven Aminoalkohol der Formel (III) 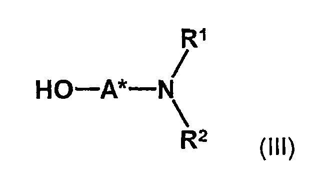 gezeigt sind, aber auch andere Aminoalkohole verwendet werden können. Insbesondere können auch die übrigen in der Anmeldung genannten Aminoalkohole eingesetzt werden.

Sofern ein Gemisch aus Diastereomeren der Formel (VIIIa) 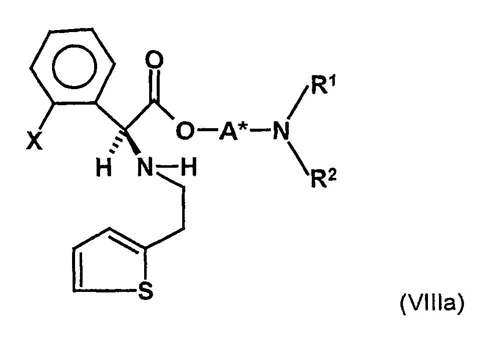 und (Vlllb) 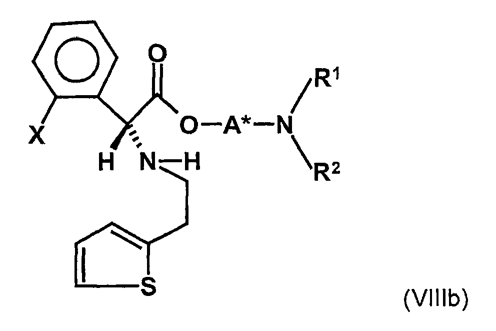 vorliegt, kann entweder zunächst eine Ringschlußreaktion zu einem Gemisch der Diastereomeren der Formeln (VIa) 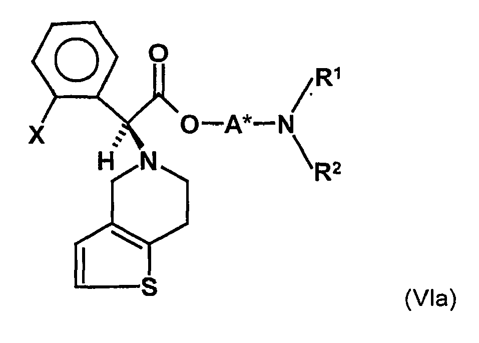 und (Vlb) 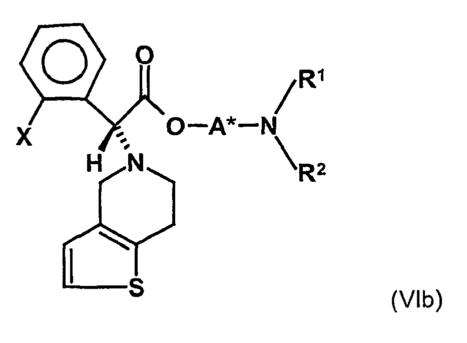 durchgeführt werden, das dann wie nachstehend erläutert weiter umgesetzt wird. Es ist auch möglich, daß an dem Gemisch der Diastereomeren (VIIIa) 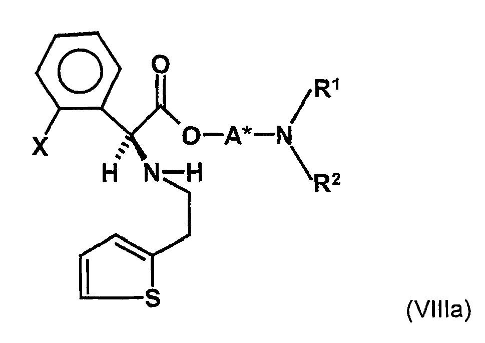 und (Vlllb) 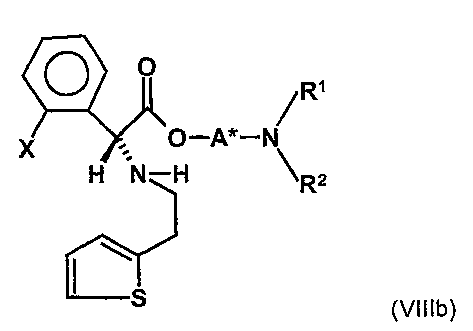 zunächst eine Diastereomerentrennung durchgeführt wird, beispielsweise durch Kristallisation (bevorzugt) oder durch Chromatographieverfahren und nur das Diastereomere der Formel (VIIIa) 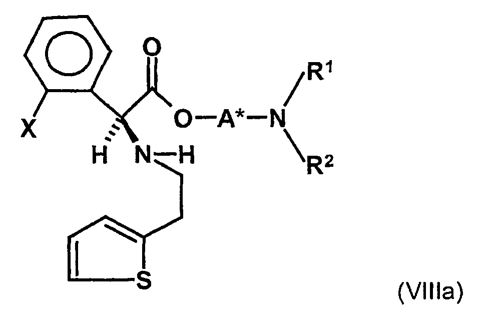 weiter umgesetzt wird. Das einzelne Diastereomere der Formel (VIIIa) 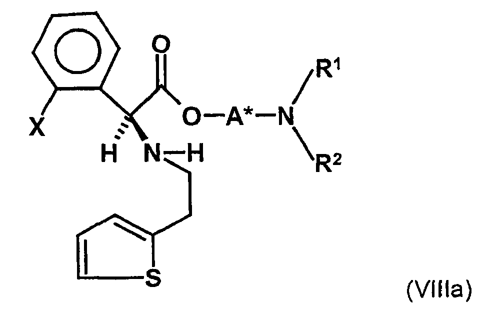 kann dann entweder zunächst durch eine Ringschlußreaktion unter Erhalt der Stereochemie in die Verbindung der Formel (VIa) 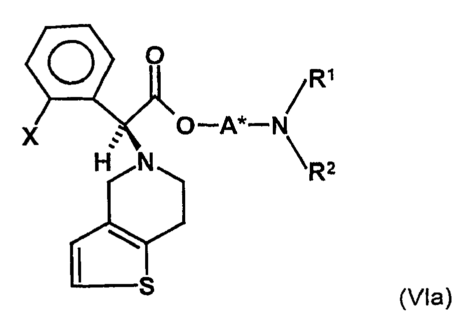 überführt werden, wie es im Stand der Technik für ähnliche Verbindungen gezeigt ist, und anschließend einer Umesterung unterzogen werden, wie es nachstehend gezeigt wird, oder die Verbindung der Formel (VIIIa) kann zunächst einer Umesterungsreaktion unterworfen werden, so daß die Verbindung der Formel 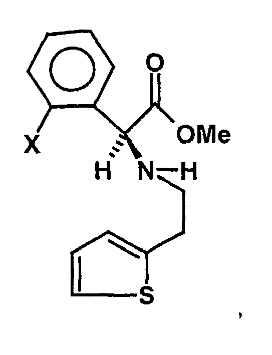 entsteht, die dann durch Ringschluß in die Verbindung der Formel (la) 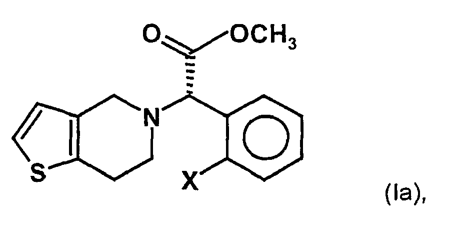 bevorzugt in Clopidogrel, überführt wird, wie es im Stand der Technik beschrieben ist, z.B. in der EP 466 569.

Das zweite Diastereomerengemisch mit den bereits geschlossenkettigen Diastereomeren der Formeln (VIa) 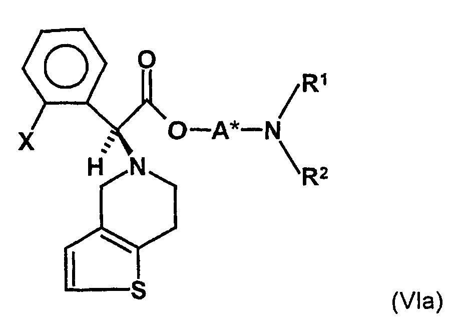 und (Vlb) 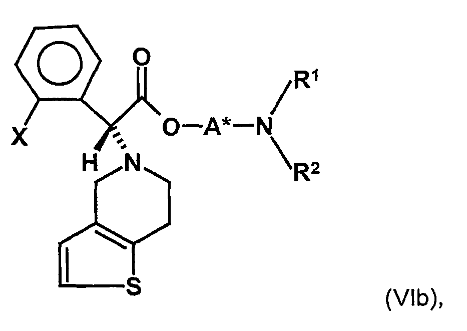 in dem das Diastereomer der Formel (VIa) 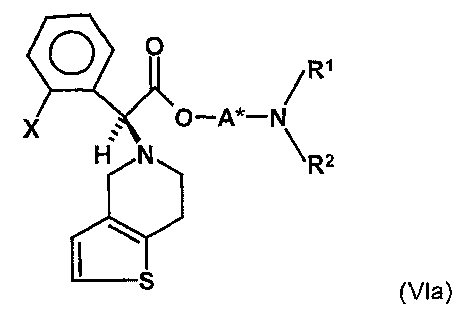 bevorzugt in einem Überschuß von zumindest 3:1 oder mehr, stärker bevorzugt 4:1 oder mehr, am stärksten bevorzugt 9:1 oder mehr vorliegt (bzw. indem das zweite Gemisch aus Diastereomeren das gewünschte Diastereomer in einem Anteil von 95% oder mehr und das unerwünschte Diastereomer in einem Anteil von nur 5% oder weniger, enthält, stärker bevorzugt indem das gewünschte Diastereomer in einem Anteil von 98% oder mehr und das unerwünschte Diastereomer in einem Anteil von nur 2% oder weniger vorhanden ist, und insbesondere ein Verhältnis von gewünschtem Diastereomer zu unerwünschtem Diastereomer von etwa 99,5:0,5 oder besser vorliegt), kann entweder zunächst einer Diastereomerentrennung unterworfen werden, was erfindungsgemäß bevorzugt ist, und hierdurch wird das Diastereomer der Formel (VIa) 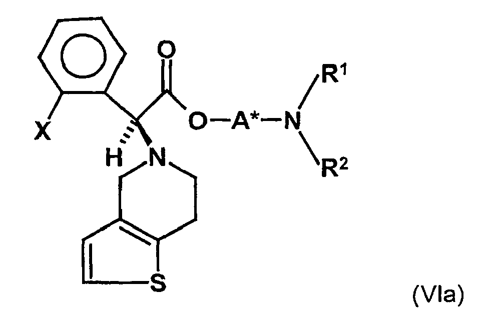 abgetrennt. Die Diastereomerentrennung kann auf an sich bekannte Art und Weise erfolgen, wie es im Stand der Technik beschrieben ist und wie es in den Beispielen näher erläutert wird. Anschließend wird das Diastereomer der Formel (VIa) 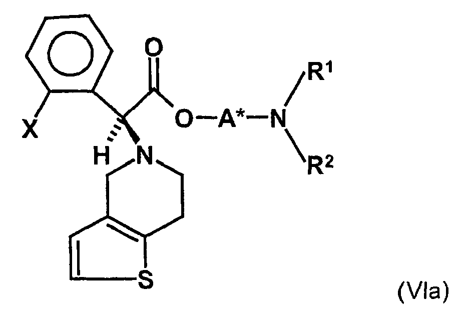 einer Umesterungsreaktion unterworfen unter Erhalt der Stereochemie zu der Verbindung der Formel (la)

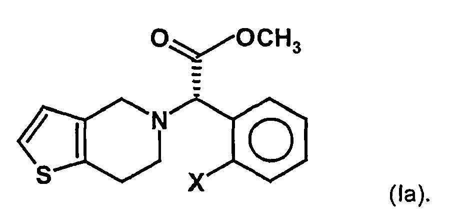

Alternativ dazu kann das Diastereomerengemisch aus den Verbindungen der Formel (Vla) 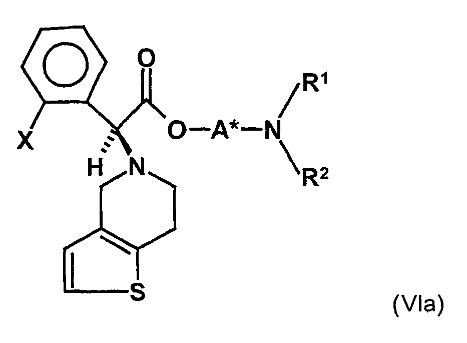 und (Vlb) 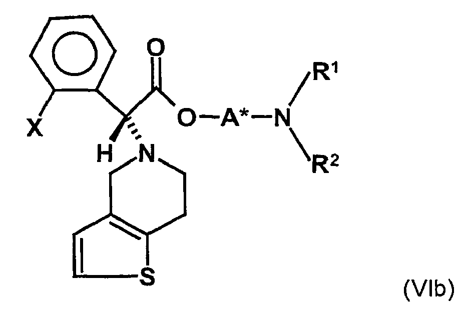 ohne vorherige Auftrennung einer Umesterungsreaktion unterworfen werden. Diese Variante ist insbesondere dann bevorzugt, wenn ein sehr hoher Überschuß an dem Diastereomer der Formel (VIa) 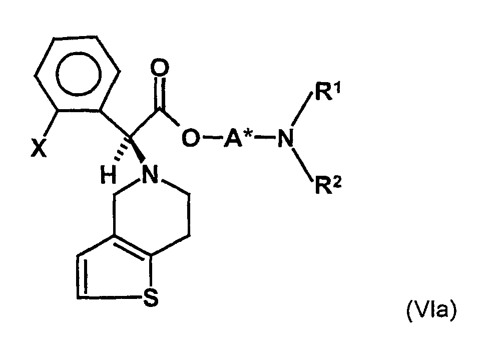 vorliegt oder wenn die anschließende Umesterung nicht vollständig unter Erhalt der Stereochemie durchgeführt werden kann. Bei der Umesterung entsteht dann ein Gemisch aus den Verbindungen (la) 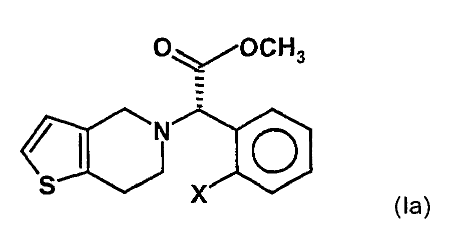 und (Ib) 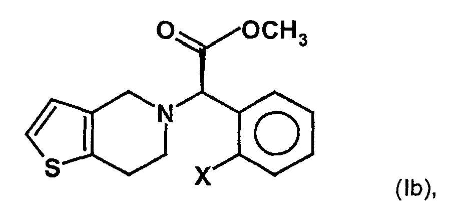 bei dem aber die Verbindung (la) in erheblichem Überschuß zu der Verbindung der Formel (Ib) vorliegt. Dieses Gemisch aus Enantiomeren kann dann, falls erforderlich, einer Enantiomerentrennung unterworfen werden, wie sie im Stand der Technik prinzipiell bekannt ist.

Die Umesterung erfolgt bevorzugt unter Verwendung eines Titan- oder Siliciumkatalysators, insbesondere bevorzugt unter Verwendung eines Titan(IV)alkoxids, insbesondere eines Titan(IV)isopropoxids (Tetraisopropylorthotitanats) oder eines Titan(IV)ethoxids, wie sie beispielsweise von der Firma Fluka kommerziell erhältlich sind. Das Titanalkoxid wird durch Umsetzung mit einem geeigneten Alkohol, z.B. mit Ethylenglycol, aktiviert. Auch ein anderer Katalysator kann verwendet werden, beispielsweise chloriertes Siliciumdioxid, das man z.B. durch Umsetzung von Siliciumdioxid mit Thionylchlorid gegebenenfalls in Phosphorpentachlorid erhalten kann, oder auch durch Umsetzung von Siliciumdioxid ausschließlich mit Phosphorpentachlorid in einem geeigneten inerten Lösemittel wie Hexan, Chloroform, Chlorbenzol oder Dichlormethan erhalten kann.

In einer besonders bevorzugten Ausführungsform, die sich insbesondere dann anbietet, wenn das Diastereomerengemisch bereits einen sehr hohen Überschuß an dem gewünschten Diastereomer aufweist, erfolgt die Umesterung unter Verwendung eines Halogenids eines Übergangsmetalls der ersten oder zweiten Nebengruppe des Periodensystems der Elemente. Geeignete Katalysatoren sind beispielsweise ZnX₂, Cu₂X₂, CuX₂, AgX, AuX, AuX₃, CdX₂, Hg₂X₂, HgX₂, CoX₂, wobei X für das Halogenidgegenion steht, insbesondere für ein Fluorid-, Chlorid-, Bromid- oder Iodidion, insbesondere für ein Chloridion. Am stärksten bevorzugt handelt es sich bei dem Übergangsmetall um Zink oder Kobalt, so daß der am stärksten bevorzugte Katalysator der Erfindung ZnX₂ oder CoX₂ ist.

Prinzipiell können auch Halogenide von anderen Metallen der dritten bis achten Übergangsmetallgruppe des Periodensystems der Elemente verwendet werden, diese müssen aber in der Regel in saurem Medium eingesetzt werden, was die Gefahr der Racemisierung erhöht und ihre katalytischen Eigenschaften sind häufig schwächer, so daß diese Katalysatoren erfindungsgemäß nicht so sehr bevorzugt sind.

Ein besonderer Vorteil der erfindungsgemäß besonders bevorzugten Übergangsmetallhalogenidkatalysatoren, insbesondere des Zinkchlorids, ist es, daß unter basischen Bedingungen gearbeitet werden kann und daß bei der Umesterung Racemisierung allenfalls in einem sehr geringen Ausmaß auftritt. Setzt man beispielsweise zur Umesterung eines der besonders bevorzugten zweiten Diastereomerengemische der Erfindung ein, das das gewünschte Diastereomer in einem Anteil von 98% oder mehr, stärker bevorzugt von 99% oder mehr, enthält und das nicht erwünschte Diastereomer nur in einem Anteil von 2% oder weniger, insbesondere von 1 % oder weniger, aufweist, so kann man bei sorgfältiger Verfahrensführung nach der Umesterung ein Produkt erhalten, das 80% oder mehr, insbesondere 90% oder mehr, bevorzugt etwa 95% oder mehr, stärker bevorzugt etwa 98% oder mehr, an dem gewünschten Enantiomer der Formel (la) 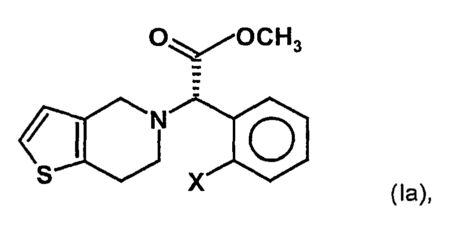 (d.h. des Clopidogrels wenn X gleich Cl ist) enthält, wobei der Rest das unerwünschte Enantiomer der Formel (Ib) 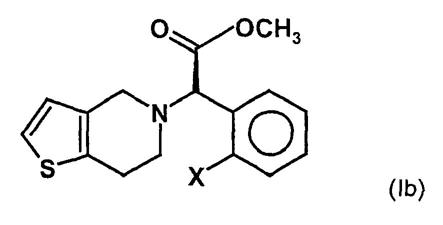 ausmacht. Eine weitere Aufreinigung des Clopidogrelgemischs zur Erhöhung des Anteils an dem gewünschten Enantiomer der Formel (la) 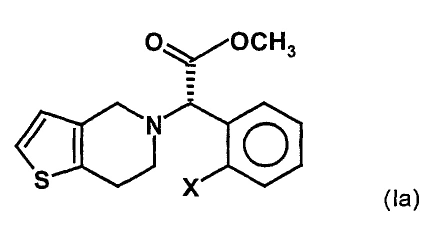 des Clopidogrels kann durch Behandlung mit einer Säure erfolgen, wie es in den Beispielen gezeigt wird.

Die Umesterung kann prinzipiell auch ohne Verwendung eines Katalysators durchgeführt werden, allerdings muss man dann höhere Reaktionszeiten in Kauf nehmen.

Zur Durchführung der Umesterung wird am besten in Methanol als Lösemittel gearbeitet, da ein Methylester hergestellt werden soll.

Im folgenden wird das erfindungsgemäße Verfahren am Beispiel der Umsetzung von 2-Chlorphenylessigsäure mit (R)-Dimepranol und anschließender Umsetzung mit 2-(2-Thienyl)ethylamin näher erläutert. Die Umsetzung erfolgt jedoch entsprechend, wenn anstelle des (R)-Dimepranols ein anderer optisch aktiver Aminoalkohol verwendet wird bzw. wenn statt der 2-Chlorphenylessigsäure eine andere 2-Halogenphenylessigsäure verwendet wird und wenn statt des 2-(2-Thienyl)ethylamins die entsprechende offenkettige Verbindung verwendet wird, die dann in einem späteren Verfahrensschritt einer Ringschlußreaktion unterworfen wird.

Die Umsetzung erfolgt prinzipiell nach folgendem Reaktionsschema:

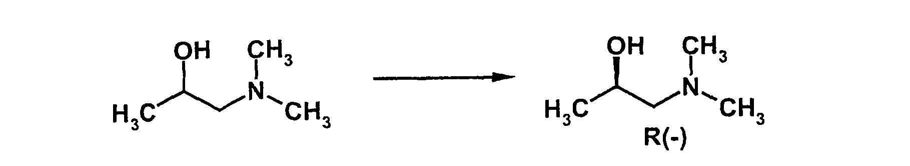

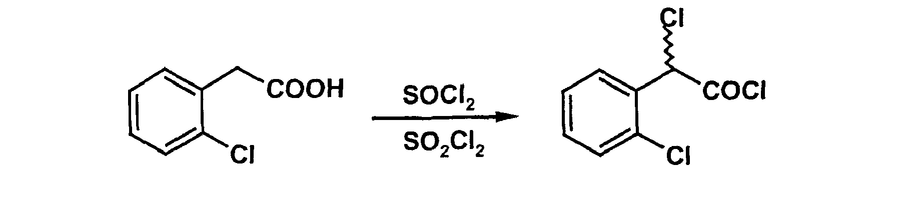

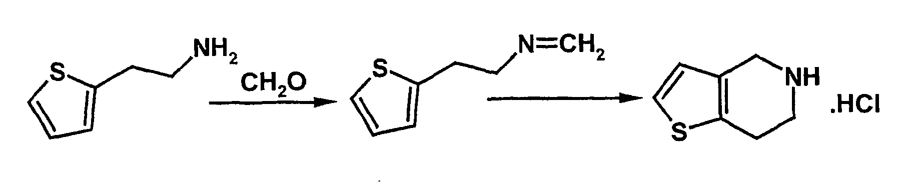

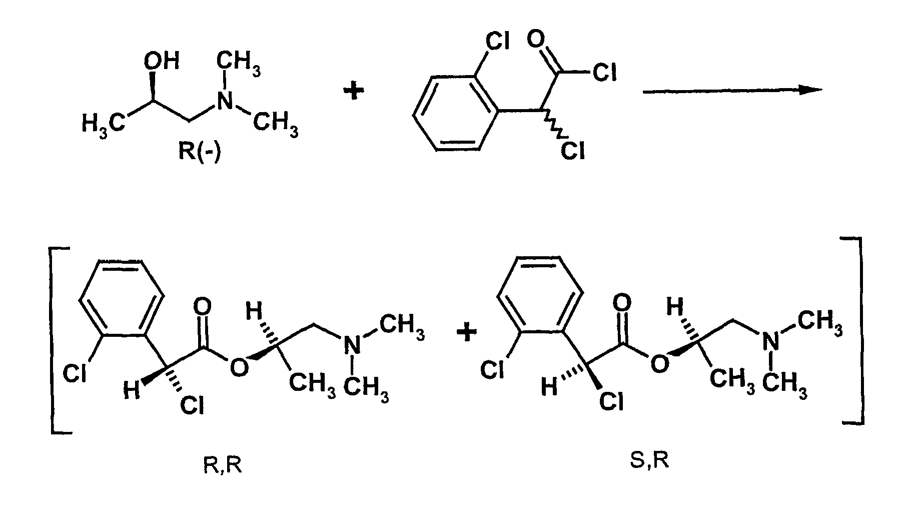

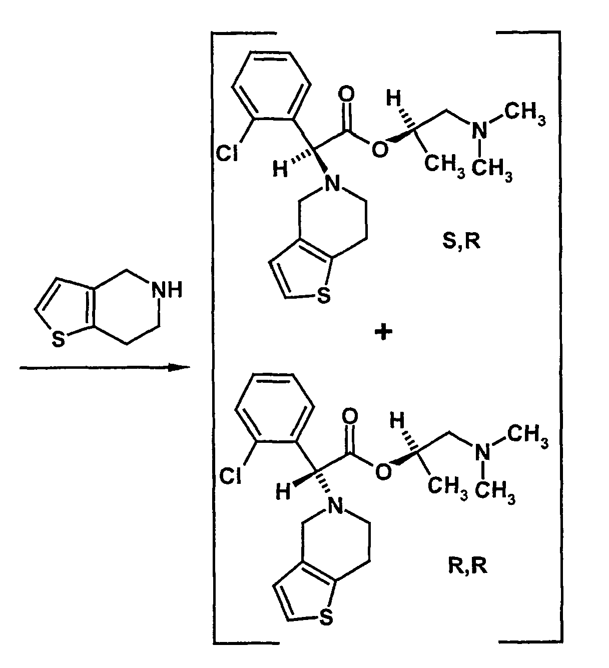

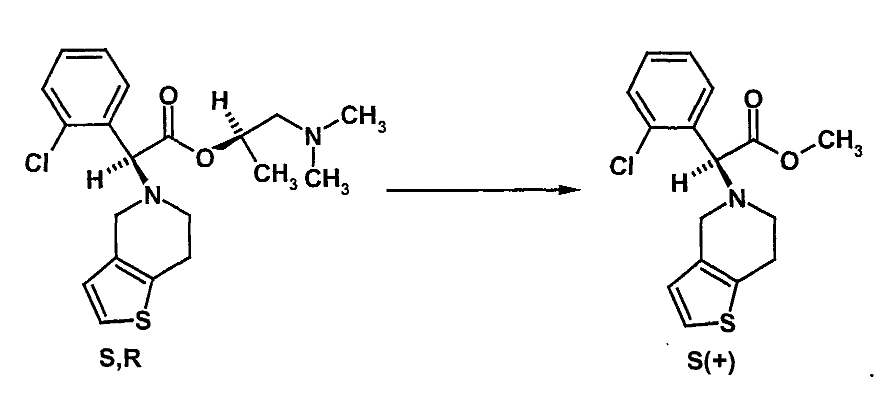

Der optisch aktive Aminoalkohol (R)-Dimepranol ist zwar kommerziell erhältlich, es wurde jedoch ein Verfahren gefunden, mit dem der optisch aktive Aminoalkohol leicht und kostengünstig aus dem racemischen Aminoalkohol hergestellt werden kann. Hierzu werden 0,5 Äquivalente Dibenzoyl-L-weinsäure mit einem Äquivalent des racemischen Alkohols in einem geeigneten Lösemittel, wie einem C₁-C₄-Alkanol, insbesondere Ethanol, stärker bevorzugt eine etwa 1:1 Mischung aus Methanol oder Ethanol mit Isopropanol, gelöst. Hierbei bildet sich das entsprechende Dibenzoyl-L-weinsäurederivat des R(-)Dimepranols. Die Lösung wird dann mit einer achiralen Mineralsäure, z.B. Salzsäure, angesäuert. Hierdurch bildet sich das Salz (z.B. das Hydrochloridsalz) mit dem entgegengesetzten Enantiomer des Dimepranols, dem S(+)Dimepranol, das dadurch in Lösung gehalten wird. Gegebenenfalls nach Animpfen fällt das Dibenzoyl-L-weinsäurederivat des R(-)Dimepranols als kristallines Produkt aus, während das Salz des S(+)Dimepranols in Lösung bleibt. Aus dem ausgefallenen Salz kann die freie (R)-Dimepranolbase durch Umsetzung mit einer geeigneten Base, wie Natrium- oder Kaliumhydroxid, in einem geeigneten Lösemittel, wie einem Alkohol, insbesondere in Ethanol, auf an sich bekannte Art und Weise erhalten werden. In einer alternativen Ausführungsform kann z.B. auch das Dibenzoyl-L-weinsäurederivat des R-Dimepranols durch Behandlung mit trockenem Chlorwasserstoff in das R-Dimepranol-Hydrochlorid überführt werden, das als solches oder nach Behandlung mit einer geeigneten Base in der basischen Form weiterverwendet werden kann.

Durch das erfindungsgemäße Verfahren wird die Menge an benötigter Dibenzoyl-L-weinsäure reduziert, was das Verfahren sehr wirtschaftlich macht. Ferner hat das erhaltene Produkt eine hohe optische Reinheit, und mehrfache Rekristallisationen sind in der Regel nicht erforderlich.

2,α-Dichlorphenylacetylchlorid 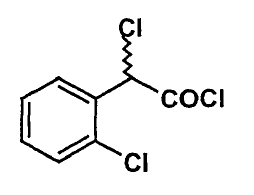 kann auf an sich bekannte Art und Weise, beispielsweise nach folgendem Reaktionsschema hergestellt werden:

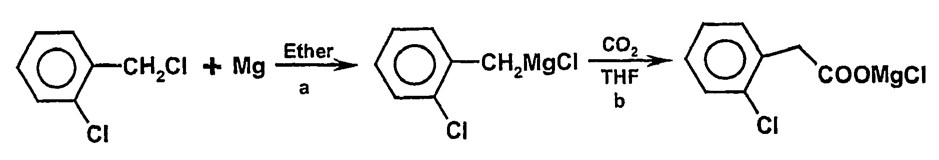

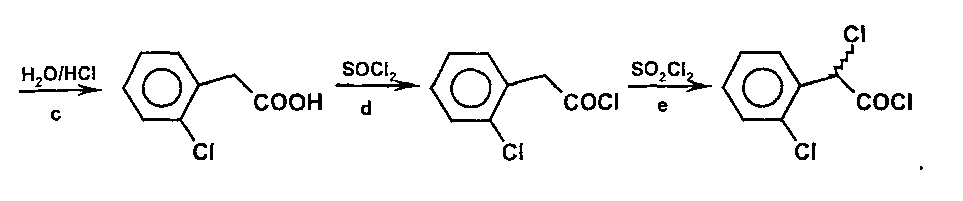

Schritt (a) wird durch Umsetzung der Verbindung 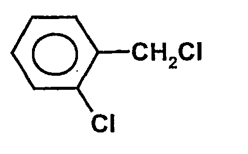 mit metallischem Magnesium in einem geeigneten Lösemittel wie beispielsweise Ether (d.h. Diethylether), Dibutylether oder höherem Ether, THF oder einem anderen cyclischen Ether, Toluol etc., vorzugsweise Ether, unter Rückfluß durchgeführt. Es handelt sich hierbei um eine übliche Grignard-Reaktion. Zu dem Reaktionsgemisch kann in Schritt (b) ohne Isolierung einer Zwischenverbindung CO₂, bevorzugt in Form von Trockeneis, entweder ohne Lösemittel oder bevorzugt mit einem Lösemittel, das CO₂ lösen kann, wie z.B. THF unter starkem Rühren zugegeben werden. Gasförmiges Kohlendioxid entweicht, und es entsteht die Verbindung

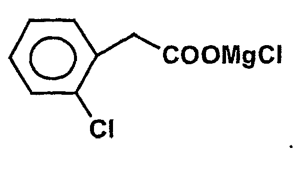

Anschließend wird in Schritt (c) eine Hydrolyse durchgeführt. Hierzu wird unter Kühlung und Rühren zu dem in Schritt (b) erhaltenen Reaktionsgemisch verdünnte Mineralsäure, z.B. verdünnte Salzsäure, zugegeben, und es entsteht ein 2-Phasen-Gemisch. Anschließend wird durch Zugabe einer geeigneten Base neutralisiert, beispielsweise mit einer wäßrigen Base wie einer NH₄OH-Lösung in Wasser oder einer Alkalicarbonatlösung oder Ammoniumcarbonatlösung in Wasser. Es entsteht eine wäßrige Lösung des Salzes der Halogenphenylessigsäure. Falls erforderlich, kann weiteres Wasser zugegeben werden. Anschließend wird die organische Phase abgetrennt, und die wäßrige Phase, die das Salz der 2-Halogenphenylessigsäure enthält, z.B. mit verdünnter Mineralsäure versetzt, bis die gewünschte 2-Halogenphenylessigsäure, insbesondere die 2-Chlorphenylessigsäure, ausfällt.

Die Säure wird abgetrennt und auf geeignete Art und Weise weiter aufgearbeitet, beispielsweise durch Auflösen in einem geeigneten Lösemittel wie Chloroform, Extraktion mit Wasser, Trocknen der Chloroformphase mit Natriumsulfat oder Magnesiumsulfat und Abdampfen des Lösemittels. Nach diesem Verfahren wird 2-Halogenphenylessigsäure in guter Ausbeute mit einer Reinheit von 95% oder höher, insbesondere von 98% oder höher, erhalten.

Die 2-Halogenphenylessigsäure wird auf übliche Art und Weise in das Säurechlorid überführt, z.B. wie es in Schritt (d) gezeigt ist unter Erwärmen in Thionylchlorid aufgelöst, wodurch die Verbindung der Formel 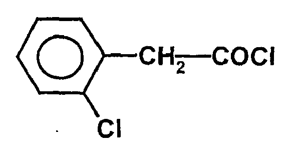 erhalten wird.

In Abhängigkeit des gewünschten Rests Y wird anschließend in Schritt (e) beispielsweise eine Bromierung oder eine Chlorierung durchgeführt. Die Bromierung kann durch Zugabe von flüssigem Brom in Gegenwart von rotem Phosphor unter Erhitzen zum Rückfluß erfolgen. Das Reaktionsgemisch wird über Nacht stehengelassen, anschließend wird nicht umgesetztes Thionylchlorid und Brom abgedampft, und man erhält die Verbindung der Formel

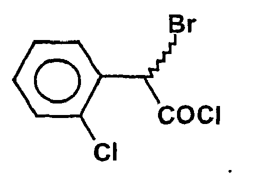

Für die Herstellung der chlorierten Verbindung wird in Schritt (e) zu dem Reaktionsgemisch Sulfurylchlorid (SO₂Cl₂) unter Rühren und Erwärmen zugegeben. Anschließend werden die Reste von SOCl₂ und SO₂Cl₂ abgedampft und man erhält die Verbindung der Formel

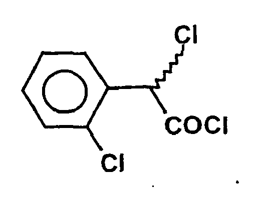

Die Verbindung der Formel 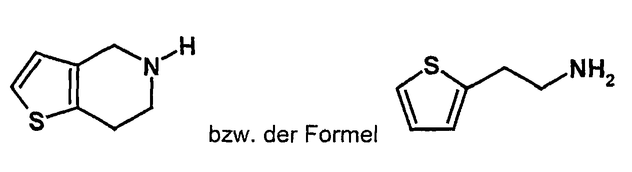 kann auf an sich bekannte Art und Weise erhalten werden, z.B. gemäß folgendem Reaktionsschema:

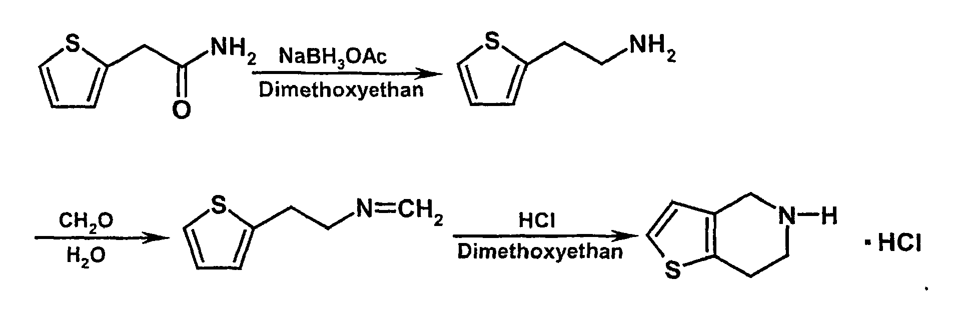

Die Ausgangsverbindung 2-Thienylacetamid kann beispielsweise aus 2-Thienylacetonitril oder 2-Thienylacetylchlorid auf bekannte Art und Weise hergestellt werden. Das 2-Thienylacetamid wird beispielsweise mit Dimethoxyethan unter Kühlung mit Natriumborhydrid versetzt. Anschließend wird unter weiterem Kühlen unter Rühren Essigsäure zugegeben. Das Gemisch wird unter Rühren erwärmt, und unter Zusatz von Wasser wird hydrolysiert, wobei gegebenenfalls gekühlt wird. Nach Abdampfen des Dimethoxyethans wird die Lösung z.B. durch Zugabe einer geeigneten Base, z.B. Kaliumhydroxid oder Natriumhydroxid, basisch gestellt, und ein geeignetes Lösemittel wie z.B. Ether wird zugegeben. Die wäßrige Phase wird abgetrennt. Die organische Phase wird gewaschen, angesäuert, beispielsweise mit einer Mineralsäure, und es wird erneut extrahiert. Die organische Phase wird abgetrennt. Die wäßrige Phase wird basisch gestellt, beispielsweise durch Zugabe von Kaliumhydroxid oder Natriumhydroxid, und es wird Ether zugegeben. Die organische Phase enthält das gewünschte 2-(2-Thienyl)ethylamin, und nach Abdampfen des Lösemittels erhält man das 2-(2-Thienyl)ethylamin in einer Reinheit von bevorzugt 95% oder höher, stärker bevorzugt von 98% oder höher.

Das so erhaltene 2-(2-Thienyl)ethylamin kann beispielsweise mit wäßrigem Formaldehyd unter Rühren erwärmt werden. Anschließend wird abgekühlt, es wird ein geeignetes organisches Lösungsmittel wie Dichlormethan zugegeben, und es wird nach Zusatz einer Base, beispielsweise Natriumhydroxid oder Kaliumhydroxid, wie einer 10%igen Natriumhydroxidlösung, extrahiert. Die wäßrige Phase wird abgetrennt, und die organische Phase wird zur Trockne abgedampft. Der Rückstand wird in z.B. Dimethoxyethan aufgelöst, und es wird angesäuert. Es folgt die Cyclisierung durch Schiffsche Base unter Rühren. Nach Abkühlung, Abfiltrieren und Waschen erhält man das gewünschte 4,5,6,7-Tetrahydro[3,2-c]thienopyridin.

Diese Umsetzung zur Herstellung der Verbindung 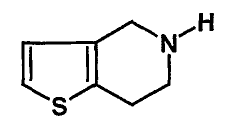 wird z.B. auch in der EP-A 439 404 und in Arkiv för kemi, 32, (19), 217-227 (1971) beschrieben, auf die insoweit vollinhaltlich Bezug genommen werden.

Die Ausgangsverbindungen der Synthesen sind dem Fachmann bekannt und sind entweder kommerziell erhältlich oder können leicht durch literaturbekannte Verfahren hergestellt werden.

Die Umsetzung der Verbindung der Formel (II) 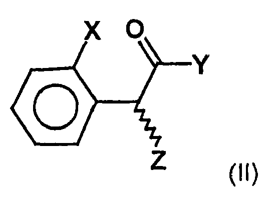 mit der Verbindung der Formel (III) 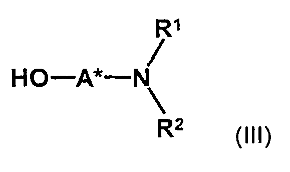 erfolgt auf an sich bekannte Art und Weise. Beispielsweise wird 2,α-Dichlorphenylacetylchlorid in einem geeigneten Lösemittel, insbesondere in einem dipolar aprotischen Lösemittel, z.B. einem Ether wie THF, mit (R)-1-(Dimethylamino)-2-propanol, bevorzugt in Gegenwart eines Amins, wie einer Pyridinverbindung, wie 4-(Dimethylamino)pyridin, insbesondere aber in Gegenwart eines tertiären Amins, wie Triethylamin, umgesetzt. Das kristalline Hydrochlorid des entstehenden Aminoesters fällt aus und kann abgetrennt werden. In einer besonders bevorzugten Ausführungsform wird das Gemisch zunächst noch mit einem geeigneten Keton, insbesondere mit Aceton, unter Rückfluß erhitzt, wodurch sich der Gehalt des gewünschten R,R-Diastereomers erhöht. Diese Ausführungsform ist daher besonders vorteilhaft zur Erzielung eines hohen Überschusses des gewünschten Diastereomers bereits in einem sehr frühen Verfahrensstadium.

Wird bei der Herstellung des ersten Diastereomerengemischs ein tertiäres Amin wie z.B. Triethylamin eingesetzt, enthält das abfiltrierte kristalline Produkt ein Gemisch aus dem gewünschten Diastereomerengemisch und Triethylamin. Dadurch, daß das Diastereomerengemisch noch ein tertiäres Amin enthält, läßt es sich in der Regel leichter abfiltrieren und überraschenderweise wird hierdurch die Racemisierung in dem folgenden Verfahrensschritt zur Herstellung des zweiten Diastereomerengemischs vermindert.

Die Aufarbeitung und gegebenenfalls Reinigung erfolgt auf bekannte Art und Weise, z.B. durch Umkristallisieren und/oder Chromatographie. Häufig kann aber auch das rohe Gemisch direkt weiterverarbeitet werden. Die verwendbaren Lösemittel sind nicht besonders eingeschränkt, allerdings sollten keine primären Alkohole wie Methanol oder Ethanol verwendet werden, um das Risiko von Transveresterungen zu vermeiden. Sekundäre oder tertiäre Alkohole wie 2-Propanol können verwendet werden, und insbesondere für die Umkristallisation kann die Verwendung derartiger sekundärer Alkohole wie 2-Propanol vorteilhaft sein.

Das Verhältnis der beiden Diastereomeren in dem entstehenden Gemisch 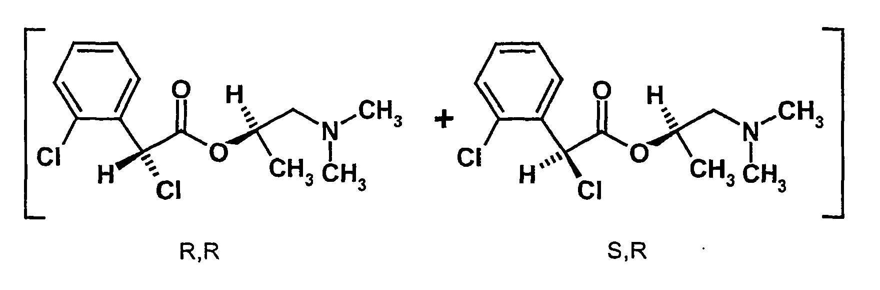 kann beispielsweise durch chirale HPLC ermittelt werden. Bevorzugt beträgt es etwa 2:1 oder mehr, stärker bevorzugt 3:1 oder mehr, insbesondere etwa 4:1 oder mehr.

Das Diastereomerengemisch 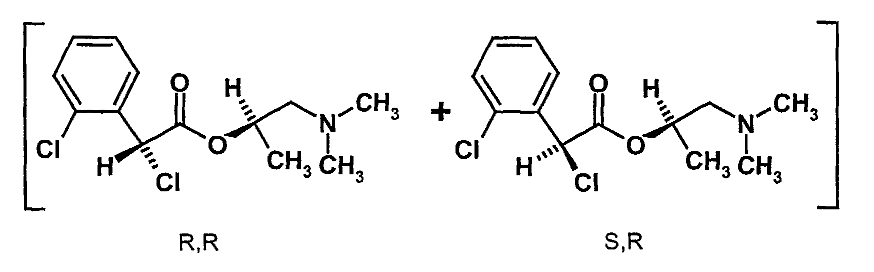 wird bevorzugt ohne Auftrennung in die einzelnen Diastereomeren mit der Verbindung der Formel (V) 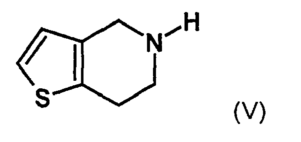 oder der Verbindung der Formel (VII) 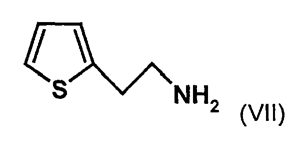 umgesetzt, bevorzugt mit der Verbindung der Formel (V).

Beispielsweise kann 4,5,6,7-Tetrahydro-[3,2-c]-thienopyridinhydrochlorid in einem geeigneten Lösemittel, insbesondere einem dipolar aprotischen Lösemittel, z.B. Dimethylformamid, DMSO oder 1,3-Dimethyl-3,4,5,6-tetrahydro-2-(1H)-pyrimidinon, in Gegenwart einer geeigneten Base, insbesondere eines Carbonats, Hydrogencarbonats oder Amins wie Triethylamin und/oder Lithiumcarbonat mit 2,α-Dichlorphenylessigsäure-(R)-1-(dimethylamino)-2-propylesterhydrochlorid vorzugsweise bei einer Temperatur im Bereich von 0 bis 100°C für einen geeigneten Zeitraum von in der Regel mehreren Stunden gerührt werden. In einer besonders bevorzugten Ausführungsform erfolgt dieser Umsetzungsschritt in Gegenwart von Siliciumdioxid. Es hat sich gezeigt, daß hierdurch die optische Reinheit des Endprodukts noch weiter erhöht werden kann.

Das Produkt kann durch Verteilen zwischen einer organischen Schicht und einer wäßrigen Schicht und anschließendem Abdampfen des Lösemittels isoliert werden. Durch Überführung in das Hydrochlorid oder das Hydrobromid mit der entsprechenden Mineralsäure kann ein kristallines Salz erhalten werden. Das entstehende Gemisch aus Diastereomeren 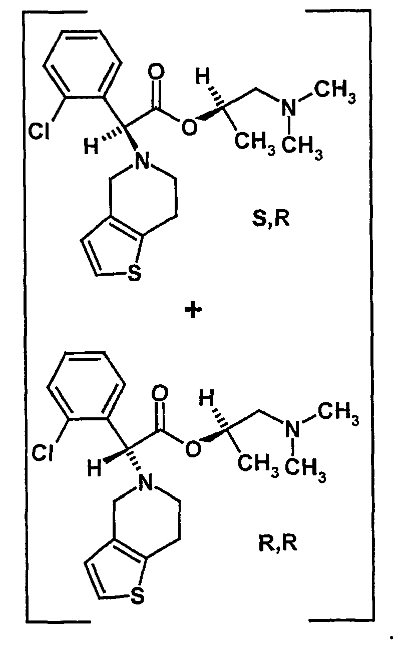 weist einen Überschuß an dem S,R-Diastereomeren von bevorzugt 4:1 oder mehr, stärker bevorzugt etwa 9:1 oder mehr auf.

Überraschenderweise hat sich auch gezeigt, daß statt Verwendung einer Mineralsäure zur Überführung der Diastereomeren in die entsprechenden Salze besonders vorteilhaft eine Dicarbonsäure verwendet werden kann. Durch Verwendung einer Dicarbonsäure anstelle der Mineralsäuren kann das Verhältnis des gewünschten Diastereomeren gegenüber dem nicht erwünschten Diastereomeren noch weiter erhöht werden, und man erhält Gemische aus Diastereomeren, die das gewünschte Diastereomer zu 95% oder mehr und das unerwünschte Diastereomer zu 5% oder weniger enthalten, insbesondere solche, bei denen das gewünschte Diastereomer in einem Anteil von 98% oder mehr und das nicht erwünschte Diastereomer in einem Anteil von 2% oder weniger vorhanden ist. Es können sogar Diastereomerengemische erhalten werden, die das gewünschte Diastereomer in einem Anteil von 99,5% oder mehr und das unerwünschte Diastereomer in einem Anteil von 0,5% oder weniger enthalten.

Die geeigneten Dicarbonsäuren sind nicht besonders eingeschränkt, bevorzugt sind aber Maleinsäure, Oxalsäure und Fumarsäure. Am stärksten bevorzugt ist Maleinsäure, so daß das zweite Diastereomerengemisch in der besonders bevorzugten Ausführungsform der Erfindung als Maleinsäuresalz vorliegt.

Falls das erhaltene Gemisch an Diastereomeren (Diastereomerengemisch 2) ein Verhältnis von (S,R)-Enantiomer zu (R,R)-Enantiomer aufweist, das für die folgende Umsetzung zu Clopidogrel noch nicht als ausreichend hoch angesehen wird, kann der Gehalt an gewünschtem (S,R)-Diastereomer erhöht werden, z.B. indem das Gemisch aus Diastereomeren in einem Überschuß eines geeigneten polar-protischen Lösungsmittels, beispielsweise eines geeigneten Alkohols, wie Isopropanol, unter Rückfluß erhitzt wird. Auf diese Art und Weise können Diastereomerengemische erhalten werden, die das gewünschte (S,R)-Diastereomer in einem Anteil von 98% oder mehr, insbesondere von 99,5% oder mehr, enthalten und die dementsprechend das unerwünschte (R,R)-Diastereomer in einer Menge von 2% oder weniger, bevorzugt 0,5% oder weniger, enthalten. Durch dieses Verfahren werden auch andere Verunreinigungen des Diastereomerengemischs verringert.

Das Gemisch aus Diastereomeren 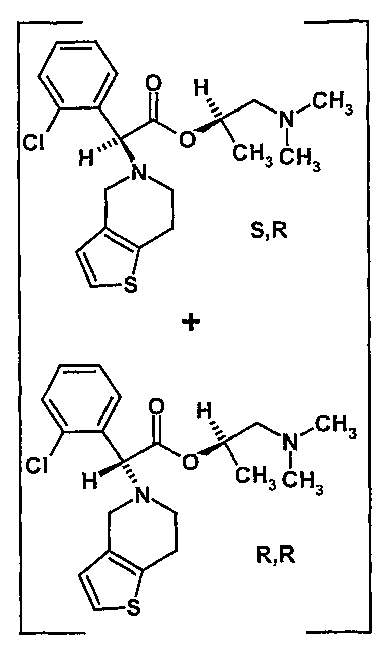 oder das abgetrennte Diastereomer 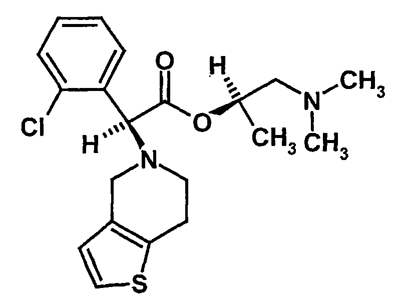 gegebenenfalls als Salz, insbesondere als Maleatsalz, Oxalatsalz oder Fumaratsalz, wird durch Umesterung in das Gemisch aus dem S- und R-Enantiomeren des Clopidogrels überführt, das an dem S-Enantiomeren angereichert ist, oder in reines Clopidogrel überführt. Hierbei werden bevorzugt die Titan- oder Siliciumdioxidkatalysatoren eingesetzt, wie vorstehend ausgeführt. Besonders bevorzugt erfolgt die Umesterung aber wie vorstehend ausgeführt im basischen Medium unter Verwendung eines Katalysators, bei dem es sich bevorzugt um das Halogenid eines Übergangsmetalls aus der ersten oder zweiten Nebengruppe des Periodensystems der Elemente, wie Cu₂X₂, CuX₂, AgX, AuX, AuX₃, CdX₂, Hg₂X₂, HgX₂, CoX₂ oder ZnX₂, insbesondere ZnX₂ oder CoX₂, handelt, wobei X ein Halogenidgegenion, insbesondere ein Chloridion, darstellt.

Die Umesterung erfolgt bevorzugt durch Erhitzen der Verbindung der Formel 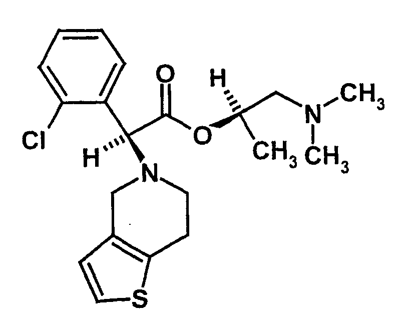 oder des Diastereomerengemischs 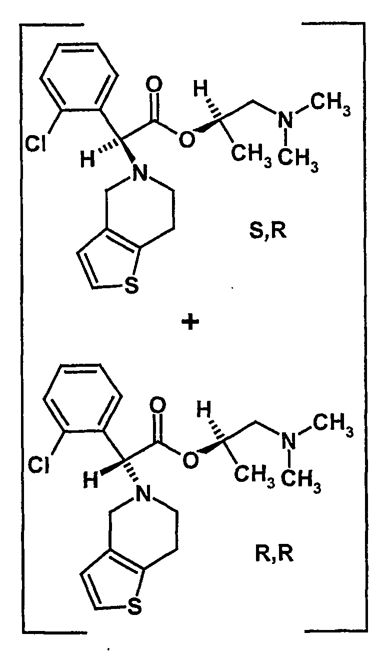 mit dem Katalysator in Methanol. Für die Umsetzung kann das Diastereomerengemisch bzw. die abgetrennte Verbindung als freie Base oder aber direkt in Form eines Salzes, insbesondere eines Salzes mit einer Diarbonsäure, besonders bevorzugt als Maleatsalz, eingesetzt werden. Bei Verwendung der freien Base ist die Reaktionsgeschwindigkeit höher als bei Verwendung des Salzes, allerdings führt die Verwendung des Salzes zu einer besonders geringen Racemisierung während der Umesterung. Die besonders bevorzugte Umesterung unter Verwendung eines Halogenids eines Übergangsmetalls, wie vorstehend ausgeführt, erfolgt bevorzugt unter schwach basischen Bedingungen unter Verwendung einer geeigneten anorganischen oder bevorzugt organischen Base, wie Diisopropylamin, Triethylamin oder eines Pyrolidins, beispielsweise von N-Methylpyrolidin. Bei Verwendung eines Salzes des eingesetzten Diastereomerengemischs bzw. des eingesetzten bevorzugten Diastereomers zur Umesterung kann es vorteilhaft sein, zur Erhöhung der Reaktionsgeschwindigkeit eine stärkere Base wie Triethylamin oder Diisopropylamin zu verwenden, hierdurch erhöht sich aber das Risiko der Racemisierung bei der Umesterung. Bevorzugt wird daher erfindungsgemäß ein geeignetes Salz des Diastereomerengemischs eingesetzt, wobei das Diastereomerengemisch bereits derart angereichert ist, daß es das gewünschte Diastereomer zu 95% oder mehr, stärker bevorzugt zu 98% oder mehr, insbesondere zu 99,5% oder mehr, enthält, wobei es sich bei dem Salz am stärksten bevorzugt um ein Oxalatsalz, ein Fumaratsalz oder Maleatsalz handelt, insbesondere um ein Maleatsalz, und die Umsetzung erfolgt unter schwach basischen Bedingungen, so daß die Racemisierung bei der Umesterung möglichst gering gehalten wird.

Nach Beendigung der Reaktion wird zu dem Umsetzungsgemisch Base zugegeben, wie es in den Beispielen gezeigt wird, abfiltriert und auf übliche Art und Weise aufgearbeitet, wie es in den Beispielen gezeigt wird. Falls das Endprodukt noch die Verbindung der Formel 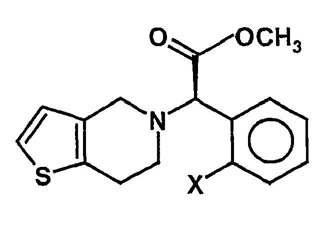 enthält, kann auf übliche Art und Weise das gewünschte Clopidogrelenantiomer abgetrennt werden. Hierzu kann auf die eingangs erwähnten Druckschriften verwiesen werden.

Besonders bevorzugt wird das unerwünschte Clopidogrelenantiomer aber abgetrennt, indem das Gemisch aus Enantiomeren in einem geeigneten Lösemittel, bevorzugt einem dipolar-aprotischen Lösemittel, wie einem Keton, insbesondere Aceton, aufgelöst wird und die Lösung angesäuert wird, beispielsweise durch konzentrierte Schwefelsäure. Das racemische Clopidogrelhydrogensulfat fällt aus der Lösung aus, und das gewünschte Enantiomer des Clopidogrels verbleibt in der Mutterlauge und kann hieraus in großer Reinheit gewonnen werden. Falls der Gehalt des unerwünschten Enantiomers gering ist, erfolgt die Kristallisation des racemischen Clopidogrelhydrogensulfats nicht unmittelbar nach Zusetzung der stöchiometrischen Menge der Schwefelsäure, und die Menge an Schwefelsäure muß erhöht werden. Falls auch dies nicht zur unmittelbaren Kristallisation führt, kann es sich empfehlen, mit racemischem Clopidogrelhydrogensulfat anzuimpfen.

Soweit im Rahmen dieser Beschreibung nichts anderes angegeben oder aus dem Zusammenhang ersichtlich ist, beziehen sich "Teile" und "Prozent" immer auf das Gewicht.

Die folgenden Beispiele erläutern die Erfindung.

### Beispiel 1

### 2,α-Dichlorphenylacetylchlorid

2-Chlorphenylessigsäure (171 g) wurde zu 300 ml Thionylchlorid gegeben, und das Gemisch wurde 30 Minuten bei 60°C gerührt. Ein lodkristall gefolgt von 300 ml Sulfurylchlorid wurden verteilt auf mehrere Male zugegeben. Das Gemisch wurde insgesamt 7 Stunden unter Rückfluß erhitzt. Überschüssige Reagenzien wurden unter vermindertem Druck abdestilliert. Der Rückstand (228 g) enthielt 82-84% 2,α-Dichlorphenylacetylchlorid, 5-6% nicht-α-chloriertes 2-Chlorphenylacetylchlorid und etwa 10% 2,4,(2,6),α-Trichlorphenylacetylchlorid. Das rohe Acylchlorid wurde ohne weitere Reinigung in den weiteren Umsetzungen verwendet.

### Beispiel 2

### (R)-1-(Dimethylamino)-2-propanol ((R)-Dimepranol)

Eine Lösung aus 190 g Dibenzoyl-L-weinsäure in 1200 ml Ethanol wurde mit 103 g 1-(Dimethylamino)-2-propanol gemischt. Die entstandene Lösung wurde mit 36 ml 36%-iger Salzsäure angesäuert und angeimpft. Nachdem es über Nacht stehengelassen wurde, wurde das kristalline Produkt abfiltriert, mit kaltem Ethanol und Diethylether gewaschen und getrocknet. Rohes (R)-1-(Dimethylamino)-2-propanoldibenzoyl-L-tartrat wurde aus 2700 ml heißem Ethanol umkristallisiert, und die Ausbeute betrug 187 g reines diastereomeres Salz. Das Salz wurde in 1000 ml kalter 20%-iger Natriumhydroxidlösung gelöst und in Dichlormethan extrahiert. Der Extrakt wurde getrocknet, filtriert, abgedampft, und das Restöl wurde durch Destillation bei Atmosphärendruck gereinigt. Das Produkt destillierte bei 122-124°C ab, und die Ausbeute betrug 46 g (*R*)-Dimepranol (45% bezogen auf das Ausgangsracemat), [α_{D}²⁰] -27°.

### Beispiel 3

### 2,α-Dichlorphenylessigsäure-(R)-1-(dimethylamino)-2-propylester

2,α-Dichlorphenylacetylchlorid (8,6 g) wurde in 30 ml Tetrahydrofuran gelöst, 1,2 g 4-(Dimethylamino)-pyridin wurden zugegeben und gemischt. Anschließend erfolgte die schnelle Zugabe einer Lösung aus 4,1 g (R)-Dimepranol in 30 ml Tetrahydrofuran unter Rühren und Kühlen. Das kristalline Aminoesterhydrochlorid wurde abgetrennt. Das Gemisch wurde anschließend auf 55°C erhitzt und 20 Minuten bei dieser Temperatur gerührt, abkühlen gelassen und dann weitere 2 Stunden bei Umgebungstemperatur gerührt. Diethylether (2 ml) wurde zugegeben, und das kristalline Produkt wurde abfiltriert, gewaschen und bei Raumtemperatur getrocknet. Rohes Esterhydrochlorid wurde aus Isopropanol umkristallisiert. Die Ausbeute betrug 10,0 g. Das gereinigte Esterhydrochlorid wurde zwischen einer gesättigten wäßrigen Lösung aus Natriumhydrogencarbonat und Diethylether verteilt. Nach dem Abdampfen erhielt man den Aminoester als freie Base. Die Ausbeute betrug 8,1 g (72,5%) des Produkts, wobei das (*R,R*)-Diastereomer (65-80%) überwog.

### Beispiel 4

### 2,α-Dichlorphenylessigsäure-(R)-1-(dimethylamino)-2-propylesterhydrochlorid (bevorzugtes Verfahren)

2,α-Dichlorphenylacetylchlorid (19,2 g, 86 mMol) wurde in 80 ml Tetrahydrofuran gelöst, und die Lösung wurde über 45 Minuten tropfenweise unter Rühren und Kühlen mit einem Eiswasserbad auf 10 bis 15°C zu einer Lösung von 12 g (86 mmol) (R)-1-(Dimethylamino)-2-propanolhydrochlorid in 80 ml Tetrahydrofuran, die 12 ml (8,9 g, 88 mMol) Triethylamin enthielt, zugegeben. Das Gemisch wurde 3 Stunden bei Raumtemperatur gerührt, und innerhalb von 2 Stunden fiel das kristalline Hydrochlorid des Aminoethers aus. Das Gemisch wurde 2 Stunden unter Rückfluß erhitzt. Anschließend wurde auf 50°C abgekühlt, 60 ml Aceton wurden zugefügt, und das Gemisch wurde weitere 2 Stunden unter Rückfluß erhitzt. Nach dem Abkühlen des Gemischs wurde das Gemisch über Nacht gekühlt (Kühlschrank). Das kristalline Produkt wurde unter Stickstoff auf einer Glasfritte abfiltriert und mit 20 ml Aceton gewaschen. Anschließend wurde bei Raumtemperatur getrocknet. Man erhielt ein Gemisch aus rohem Esterhydrochlorid und Triethylaminhydrochlorid, das etwa 24 g der diastereomeren Ester enthielt. Der Gehalt der Titelverbindung im Gemisch der diastereomeren Ester wurde durch HPLC bestimmt, und das Gemisch der diastereomeren Ester enthielt etwa 80% der Titelverbindung, was einem Verhältnis des gewünschten R,R-Diastereomeren zu dem unerwünschten Diastereomeren von 4:1 entspricht.

Das so erhaltene Diastereomerengemisch, das Triethylaminhydrochlorid enthielt, wurde ohne weitere Reinigung für die weiteren Verfahrensschritte verwendet. Gegebenenfalls kann das Gemisch aber auch aufgereinigt werden, in dem das Rohgemisch der Hydrochloride der diastereomeren Aminoester und des Triethylamins in der 2,5-fachen Menge (v/w) kochenden 2-Propanols gelöst wird, das gleiche Volumen heißen Propylacetats zugegeben wird und das Gemisch abgekühlt und über Nacht im Kühlschrank stehengelassen wird. Am folgenden Tag kann das kristalline Produkt unter Stickstoff abfiltriert werden, mit 25 ml kaltem (ca. 5°C) 2-Propylacetat gewaschen und getrocknet werden.

### Beispiel 5

### 1-Phenyl-2-(1-pyrrolidinyl)-ethanolhydrochlorid

Wasser (10 ml) wurde unter Rühren zu einem Gemisch aus 24,0 g Styroloxid und 21,3 g Pyrrolidin gegeben. Die Temperatur des Gemischs stieg auf fast 100°C an. Wasser und überschüssiges Pyrrolidin wurden aus der entstandenen klaren Lösung abdestilliert. Der Rückstand wurde in 60 ml 1,2-Dimethoxyethan gelöst, und die Lösung wurde mit einer 5,5 M Lösung aus Chlorwasserstoff in 1,2-Dimethoxyethan angesäuert. Das ausgefällte Hydrochloridsalz wurde abfiltriert, mit 1,2-Dimethoxyethan gewaschen und getrocknet. Das Filtrat wurde über Nacht in einem Kühlschrank stehengelassen, und eine zweite Ausbeute des Produkts wurde erhalten. Die Gesamtausbeute an kristallinem 1-Phenyl-2-(1-pyrrolidinyl)-ethanolhydrochlorid betrug 35,9 g.

### Beispiel 6

### (R)-1-Phenyl-2-(1-pyrrolidinyl)-ethanol

Lösungen aus 35,8 g Di-O-benzoyl-L-weinsäure in 150 ml Methanol und 19,1 g 1-Phenyl-2-(1-pyrrolidinyl)-ethanol in 100 ml Methanol wurden gemischt, und das Gemisch wurde 2 Tage im Kühlschrank stehengelassen. Das kristalline Produkt wurde abfiltriert, mit einer geringen Menge kaltem Methanol und Diethylether gewaschen und getrocknet. Das Produkt wurde wiederholt aus heißem Ethanol umkristallisiert, und es ergab sich eine Ausbeute an optisch reinem diastereomeren Salz von 15,0 g. Die freie Base wurde durch Lösung des Salzes in 100 ml kaltem 20%-igem wäßrigem Natriumhydroxid freigesetzt und in Dichlormethan extrahiert. Nach Abdampfen des Lösungsmittels erhielt man 7,2 g (38%, bezogen auf das Ausgangsracemat) des öligen (R)-Enantiomers von 1-Phenyl-2-(1-pyrrolidinyl)-ethanol, das sich nach Aufbewahrung im Kühlschrank zu einer kristallinen Masse verfestigte. Das Produkt zeigte einen [α_{D}²⁰]-Wert von -40° (Methanol).

### Beispiel 7

### 2,α-Dichlorphenylessigsäure-(R)-2-(1-pyrrolidinyl)-1-phenylethylester

Eine Lösung aus 5,7 g (R)-1-Phenyl-2-(1-pyrrolidinyl)-ethanol in 25 ml Tetrahydrofuran wurde zu einer Lösung aus 6,5 g 2,α-Dichlorphenylacetylchlorid in 25 ml Tetrahydrofuran, die 0,9 g 4-Dimethylaminpyridin enthielt, gegeben. Nach einer ähnlichen Vorgehensweise wie in Beispiel 3 erhielt man ein Gemisch aus diastereomeren Estern, das 72% (R,R) und 28% (R,S) des Produkts enthielt, in einer Ausbeute von 8,4 g (74%).

### Beispiel 8

### 2-Dimethylamino-1-phenylethanol

Eine Lösung aus 12 g Natriumhydroxid in 60 ml Wasser wurde mit 100 ml Ethanol und 24,0 g Dimethylaminhydrochlorid gemischt. Styroloxid (24,0 g) wurde zugegeben, und das Gemisch wurde 2 Stunden bei Raumtemperatur gerührt. Das ausgefällte Natriumchlorid wurde abfiltriert, Ethanol wurde bei vermindertem Druck abgedampft, und 8,0 g Natriumhydroxid wurden zu der verbleibenden wäßrigen Lösung gegeben. Der Aminoalkohol wurde in Diethylether extrahiert, die Extrakte wurden getrocknet und verdampft, und man erhielt 26,7 g eines öligen Produkts, das laut HPLC-Analyse 87% 2-Dimethylamino-1-phenylethanol enthielt.

### Beispiel 9

### (R)-2-Dimethylamino-1-phenylethanol

Racemisches 2-Dimethylamino-1-phenylethanol wurde entsprechend dem 2-Pyrrolidinyl-1-phenylethanol (siehe Beispiel 6) unter Verwendung von 16,5 g rohem Aminoalkohol, wie in Beispiel 8 beschrieben, aufgespalten. Die Ausbeute an öligem (R)-2-Dimethylamino-1-phenylethanol mit einem [α_{D}²⁰]-Wert von -45,5° (Methanol) betrug 8,9 g.

### Beispiel 10

### 2,α-Dichlorphenylessigsäure-(R)-2-dimethylamino-1-phenylethylester

Ein Gemisch aus diastereomeren Estern, in dem die (R,R)-Ester überwogen, wurde wie in Beispiel 3 und 7 aus 5,0 g (*R*)-2-Dimethylamino-1-phenylethanol hergestellt, die Ausbeute betrug 7,7 g.

### Beispiel 11

### trans-2-Dimethylaminocyclohexan-1-ol

Der Aminoalkohol wurde wie in Beispiel 8 beschrieben unter Verwendung von Cyclohexenoxid (19,6 g) anstelle von Styroloxid hergestellt. Öliges *trans*-2-Dimethylaminocyclohexan-1-ol wurde in einer Ausbeute von 22,0 g (76%) erhalten.

### Beispiel 12

### trans-2-(S)-Dimethylamino-1-(R)-hydroxycyclohexan

Der racemische Aminoalkohol des Beispiels 11 wurde entsprechend den Beispielen 3, 5 und 8 unter Verwendung von Di-O-Benzoyl-L-weinsäure in einem Molverhältnis von 1:1 (35,8 g Di-O-Benzoyl-L-weinsäure und 14,5 g racemisches *trans*-2-Dimethylaminocyclohexan-1-ol) unter Verwendung von Aceton als Lösemittel aufgespalten. Man erhielt öliges *trans*-(*S*,*R*)-2-Dimethylaminocyclohexan-1-ol in einer Ausbeute von 5,2 g. Der [α_{D}²⁰]-Wert betrug -23° (Methanol).

### Beispiel 13

### 2,α-Dichlorphenylessigsäure-trans-(S,R)-2-dimethylaminocyclohexylester

Der Ester des *trans*-2-(S)-Dimethylamino-1-(*R*)-hydroxycyclohexans mit 2,α-Dichlorphenylessigsäure wurde aus 4,4 g des Aminoalkohols und 6,5 g Acylchlorid in Tetrahydrofuran in einer Ausbeute von 6,7 g erhalten. Laut HPLC-Analyse enthielt das Produkt 68% des Diastereomeren des Esters der α-(*R*)-Chlorsäure.

### Beispiel 14

### (S)-(2-Chlorphenyl)-4,5,6,7-tetrahydro-[3,2-c]thienopyridin-5-yl-essigsäure-(R)-1-(dimethyl-amino)-2-propylesterdihydrochlorid

Die freie Base des 2,α-Dichlorphenylessigsäure-(*R*)-1-(dimethylamino)-2-propylesters, hergestellt wie in Beispiel 3 (5,8 g), wurde in 30 ml Dimethylformamid gelöst, und die Lösung wurde mit 3,6 g 4,5,6,7-Tetrahydro-[3,2-c]-thienopyridinhydrochlorid und 3,4 g festem Lithiumhydrogencarbonat gemischt. Das Gemisch wurde anschließend 45 Minuten bei 80°C gerührt. Nach Abkühlen auf Raumtemperatur wurde die Suspension mit 100 ml Chloroform und 100 ml Wasser gemischt. Die organische Schicht wurde abgetrennt und mit Wasser gewaschen, getrocknet, und das Chloroform wurde abgedampft. Der Rückstand wurde in 40 ml 2-Propanol gelöst und mit Aktivkohle entfärbt. Gasförmiger Chlorwasserstoff (1,5 g) wurde in die Lösung eingebracht, und das kristalline Hydrochloridsalz fiel aus. Das Salz wurde abfiltriert und mit 10 ml kaltem Isopropanol gewaschen. Nach dem Trocknen erhielt man ein Produkt, das 85% des gewünschten (R,S) diastereomeren Esters enthielt, in einer Ausbeute von 7,7 g (83%).

### Beispiel 15

### (S)-(2-Chlorphenyl)-4,5,6,7-tetrahydro-[3,2-c]thieno-pyridin-5-yl-essigsäure-(R)-1-(dimethyl-amino)-2-propylesterdihydrobromid

Ein etwa 3:1 Gemisch aus den (R,S)- und (R,R)-Diastereomeren des 2,α-Dichlorphenylessigsäure-1-(dimethylamino)-2-propylesterhydrochlorids (20,0 g) wurde in 4 Teilen unter Rühren zu einer Suspension aus 10,5 g 4,5,6,7-Tetrahydro-[3,2-c]-thienopyridinhydrochlorid in 150 ml Dimethylformamid, das 40 ml Triethylamin enthielt, gegeben. Das Gemisch wurde auf 45°C erwärmt und 2 Stunden bei dieser Temperatur gerührt. Dann wurden 150 ml Methyl-*tert*.butylether und 300 ml Wasser zugegeben und gründlich gemischt. Die organische Schicht wurde abgetrennt und zweimal mit Wasser gewaschen, getrocknet und verdampft, und man erhielt eine Ausbeute von 20,0 g eines Gemischs der freien Basen der diastereomeren Aminoester, das 85% (*R,S*)-Diastereomer (HPLC) enthielt. Der Rückstand wurde in einem 15-fachen Überschuß an Isopropylacetat gelöst, und die Lösung wurde im Eisbad auf 5°C abgekühlt. Gasförmiger Bromwasserstoff wurde bei 5-10°C unter Rühren in das Gemisch eingebracht, bis 8,5 g HBr absorbiert worden waren. Es wurde weitere 2 Stunden bei 10°C und dann ohne Kühlung gerührt, bis Raumtemperatur erreicht worden war. Das Gemisch wurde angeimpft und über Nacht in einem Kühlschrank stehengelassen. Die Kristalle des Aminoesterdihydrobromids wurden abfiltriert, mit kaltem Isopropylacetat gewaschen und getrocknet. Die Ausbeute betrug 20,2 g, der Gehalt an (R,S)-Diastereomer war 92%.

### Beispiel 16

### Maleinsäuresalz des (S)-(2-Chlorphenyl)-4,5,6,7-tetrahydro-[3,2-c]thienopyridin-5-yl-essigsäure-(R)-1-(dimethylamino)-2-propylesters (bevorzugtes Verfahren)

In einen 1 l Kolben wurden 180 ml (191,4 g) 1,3-Dimethyl-3,4,5,6-tetrahydro-2-(1H)-pyrimidinon (Kurzbezeichnung: N,N'-dimethylpropylenharnstoff), 18,0 g (100 mMol) 4,5,6,7-Tetrahydro-[3,2-c]thienopyridinhydrochlorid, 60 ml (43,5 g, 425 mMol) Triethylamin und 21 g (250 mMol) Lithiumcarbonat gegeben. Ein Rohprodukt, das entsprechend Beispiel 4, hergestellt worden war und das 31,38 g (96 mMol) der Aminoester enthielt, wurde unter Rühren zugegeben. Anschließend wurden 12,0 g Siliziumdioxid (Silica, 60 Å) zugegeben. Das Gemisch wurde auf 35°C erwärmt und 4 Stunden bei dieser Temperatur gerührt. Anschließend wurde die Temperatur auf 50°C erhöht, und es wurde weitere 30 Minuten zur Vervollständigung der Umsetzung gerührt. Das Gemisch wurde auf Raumtemperatur abgekühlt, 300 ml Diisopropylether wurden zugegen, und das Gemisch wurde 15 Minuten gerührt. Anschließend wurde über eine Glasfritte abfiltriert. Das Siliziumdioxid und die festen Salze wurden in 300 ml Diisopropylether resuspendiert (im Filter), und das Lösemittel wurde abgesaugt. Die Filtrate wurden vereinigt, 2 x mit jeweils 150 ml Wasser extrahiert und 2 x mit jeweils 150 ml Boratpuffer, pH 6,5, extrahiert. Die organische Phase wurde abgetrennt und mit wasserfreiem Natriumsulfat getrocknet. Das Trockenmittel wurde abfiltriert, es wurde 2 x mit 15 ml Diisopropylether gewaschen und eingedampft. Man erhielt 37,4 g der freien Base als öliges Produkt. Die Reinheit des Produkts war gemäß HPLC 95%, und das Produkt enthielt 82 bis 85% des gewünschten (R,S)-Diastereomers.

Das Rohprodukt wurde vorsichtig erwärmt und mit einer Lösung von 11,1 g (96 mmol) Maleinsäure in 300 ml 2-Propanol gemischt. Das Gemisch wurde unter Rühren auf 50°C erwärmt, bis eine klare Lösung erhalten wurde. Das Gemisch wurde dann auf Raumtemperatur abgekühlt und gerührt. Nach kurzer Zeit kristallisiert das gewünschte Produkt aus. Es wird weitere 4 Stunden gerührt, und das Produkt wird auf einem gesinterten Glasfilter abgetrennt, 2 x mit 10 ml 2-Propanol gewaschen und an der Luft getrocknet.

Die Ausbeute betrug 38,8 g (97,5%, bezogen auf den Gehalt des (S)-(2-Chlorphenyl)-4,5,6,7-tetrahydro-[3,2-c]thienopyridin-5-ylessigsäure-(*R*)-1-(dimethylamino)-2-propylesters in dem rohen Ausgangsprodukt und 80%, bezogen auf die Gesamtmenge des Diastereomerengemischs in dem Ausgangsprodukt. Das Produkt hatte einen über DSC bestimmten Schmelzpunkt von 173°C und enthielt mehr als 98% (HPLC) der Titelverbindung. Der Gehalt des unterwünschten (R,R)-Diastereomers war unter 1%. Die Mutterlauge und die Waschlösung enthielten beide Diastereomere in einem Verhältnis von 15:85. Das Verhältnis der Diastereomeren wurde durch chirale Chromatographie bestimmt.

Der Gehalt des gewünschten (R,S)-Diastereomers in dem Endprodukt war derart hoch, daß das Produkt ohne weitere Reinigung und ohne weitere Anreicherung des gewünschten (R,S)-Diastereomers direkt weiter zu Clopidogrel umgesetzt werden konnte.

### Beispiel 17

### (2-Chlorphenyl)-α-2-[(2-thieno)ethyl]aminoessigsäure-(R)-1-(dimethylamino)-2-propylester

Eine Lösung aus 2,9 g 2,α-Dichlorphenylessigsäure-1-(dimethylamino)-2-propylester als freie Base, enthaltend etwa 80% *(R,R)-* und 20% (*R,S*)-Diastereomere in 20 ml Acetonitril wurde mit 1,3 g 2-Thienylethylamin und 4 ml Triethylamin gemischt, und das Gemisch wurde unter Rühren 2 Stunden unter Rückfluß erhitzt. Acetonitril und überschüssiges Triethylamin wurden abgedampft, und der Rückstand wurde zwischen Chloroform und Wasser verteilt. Die organische Phase wurde abgetrennt, mit Wasser gewaschen, getrocknet, und das Lösemittel wurde abgedampft. Der Rückstand (3,2 g) enthielt ein Gemisch aus 85% (R,S)- und 15% (R,R)-diastereomeren Estern, das ohne weitere Aufreinigung für den nächsten Schritt verwendet wurde.

### Beispiel 18

### (2-Chlorphenyl)-α-4,5,6,7-tetrahydro-[3,2-c]thieno-pyridin-5-yl-essigsäure-(R)-1-(dimethyl-amino)-2-propylesterdihydrobromid

Ein rohes Gemisch aus diastereomerem (*R*,*S*)- und (*R*,*R*)-α-(2-Chlorphenyl)-α-2-[(2-thieno)ethyl]aminoessigsäure-(1-(dimethylamino)-2-propylester (3,1 g), das wie in Beispiel 17 hergestellt worden war, wurde in 10 ml 2 M Salzsäure aufgenommen, und 5 ml 36%-iges wäßriges Formaldehyd wurde unter Rühren zugegeben. Das Gemisch wurde 2 Stunden bei 50°C gerührt. Das Reaktionsgemisch wurde zwischen Diisopropylether und 10% wäßrigem Natriumhydrogencarbonat verteilt. Die organische Schicht wurde abgetrennt, mit Wasser gewaschen, getrocknet und unter vermindertem Druck abgedampft. Der Rückstand wurde in 30 ml eines 1:1 Gemischs aus 2-Propanol und 2-Propylacetat gelöst. Gasförmiger Bromwasserstoff (1,6 g) wurde unter Rühren und Abkühlen auf 10°C in das Gemisch eingebracht. Das ausgefällte Salz wurde abfiltriert, mit 2-Propylacetat gewaschen und getrocknet, und man erhielt 2,85 g eines kristallinen Produkts, das (chirale HPLC) etwa 92% (R,S)- und 8% (R,R)-Diastereomer enthielt.

### Beispiel 19

### Transveresterung des Hydrochlorids des (S)-(2-Chlorphenyl)-4,5,6,7-tetrahydro-[3,2-c]thienopyridin-5-yl-essigsäure-(R)-1-(dimethylamino)-2-propylesters durch Katalyse mit Orthotitanat

Ethylenglykol (1,36 g) wurde zu 12,6 g Titan(IV)isopropoxid gegeben, und beide Bestandteile wurden gründlich gemischt. Es erfolgte eine exotherme Reaktion unter Bildung von Ethylen-bis-(triisopropyl)orthotitanat, das nach Abkühlen des Gemischs als Transveresterungskatalysator verwendet wurde. Der Katalysator wurde zu einer Lösung aus 7,43 g (*S*)-(2-Chlorphenyl)-4,5,6,7-tetrahydro-[3,2-c]-thienopyridin-5-yl-essigsäure-(*R*)-1-(dimethylamino)-2-propylesterhydrochlorid in 70 ml Methanol gegeben, das Gemisch wurde 48 Stunden unter Rückfluß erhitzt und dann auf Raumtemperatur abkühlen gelassen. Anschließend wurde Natriumhydrogencarbonat (6,3 g) zugegeben, und das Gemisch wurde 30 Minuten gerührt. Die Feststoffe wurden abfiltriert, und das Filtrat wurde abgedampft. Der Rückstand wurde in 200 ml Diethylether suspendiert, und die Suspension wurde 30 Minuten gerührt. Der Katalysator wurde durch langsame Zugabe von 1,6 ml Wasser zersetzt. Festes wasserfreies Natriumsulfat (10 g) wurde der Suspension aus voluminösem weißem Niederschlag des gebildeten hydrierten Titanoxids zugesetzt, und das Gemisch wurde weitere 20 Minuten gerührt. Anschließend wurden die Feststoffe abfiltriert und auf dem Filter mit mehreren Portionen Diethylether gewaschen. Die Filtrate und das Waschwasser wurden abgedampft, und man erhielt 3,9 g Clopidogrel als freie Base, das etwa 15% (R)-Enantiomer und 85% (S)-Enantiomer enthielt.

### Beispiel 20

### Transveresterung von (S)-(2-Chlorphenyl)-4,5,6,7-tetrahydro-[3,2-c]thienopyridin-5-yl-essigsäure-(R)-1-(dimethylamino)-2-propylester durch Katalyse mit chloriertem Siliciumdioxid

Getrocknetes (120°C, 20 Torr, 2 Stunden) Siliciumdioxid mit einem durchschnittlichen Porendurchmesser von 60 Å (20 g) wurde in 60 ml Thionylchlorid suspendiert, und die Suspension wurde 46 Stunden unter Rückfluß erhitzt. Das modifizierte Siliciumdioxid wurde abfiltriert, 2 Stunden in einem Vakuumtrockner bei 125°C unter vermindertem Druck getrocknet und anschließend durch 30-minütiges Erhitzen auf 360°C aktiviert. Die freie Base des (*S*)-(2-Chlorphenyl)-4,5,6,7-tetrahydro-[3,2-c]thienopyridin-5-yl-essigsäure-(*R*)-1-(dimethylamino)-2-propylesters (10,0 g) wurde in 150 ml Methanol gelöst, 20 g aktiviertes modifiziertes Siliciumdioxid wurden zugegeben, und das Gemisch wurde 48 Stunden unter Rühren unter Rückfluß erhitzt. Das Siliciumdioxid wurde abfiltriert, mit 100 ml Methanol in mehreren Portionen gewaschen, und die vereinigten Filtrate und das Waschwasser wurden abgedampft. Der Verdampfungsrückstand (10,0 g enthaltend 7,1 g (*S*)-Clopidogrel) wurde in ein pharmazeutisch annehmbares Salz des (S)-Clopidogrels überführt.

### Beispiel 21

### Transveresterung des Dihydrochlorids des (S)-(2-Chlorphenyl)-4,5,6,7-tetrahydro-[3,2-c]thienopyridin-5-yl-essigsäure-(R)-1-(dimethylamino)-2-propylesters durch Katalyse mit chloriertem Siliciumdioxid

Getrocknetes (120°C, 20 Torr, 2 Stunden) Siliciumdioxid mit einem durchschnittlichen Porendurchmesser von 100 Å (20 g) wurde in einer Lösung aus 14 g Phosphorpentachlorid in 80 ml Hexan suspendiert und 10 Stunden unter Rückfluß erhitzt. Nach Abkühlen wurde die Suspension über Nacht stehengelassen, dann abfiltriert, zweimal mit 20 ml Hexan gewaschen und unter Stickstoffzufuhr 2 Stunden getrocknet. Anschließend wurde 2 Stunden in einem Vakuumtrockner bei 120°C und unter vermindertem Druck getrocknet, und der Katalysator wurde dann durch 30-minütiges Erhitzen auf 360°C aktiviert. Der Katalysator behält seine Leistung über mehrere Monate bei, wenn er in einem gut verschlossenen Behälter gelagert wird.

(*S*)-(2-Chlorphenyl)-4,5,6,7-tetrahydro-[3,2-c]thienopyridin-5-yl-essigsäure-(*R*)-1-(dimethyl-amino)-2-propylesterdihydrochlorid (10,0 g) wurden in 150 ml Methanol gelöst, 20 g aktiviertes modifiziertes Siliciumdioxid wurden zugegeben, und das Gemisch wurde 46 Stunden unter Rühren unter Rückfluß erhitzt. Das Siliciumdioxid wurde abfiltriert, mit drei 25 ml Ladungen Methanol gewaschen, und die vereinigten Filtrate und das Waschwasser wurden abgedampft. Das verbleibende Produkt wurde zwischen einer kalten gesättigten wäßrigen Lösung aus Natriumhydrogencarbonat und Methyl-*tert*.butylether aufgeteilt. Die organische Schicht wurde abgetrennt, und das freigesetzte Dimepranol wurde in Wasser extrahiert. Nach Verdampfen des Methyl-*tert*.butylethers aus der organischen Phase enthielt der Rückstand 6,5 g (*S*)-Clopidogrel, freie Base, das in ein pharmazeutisch annehmbares Salz überführt und durch Kristallisation gereinigt wurde.

### Beispiel 22

### (S)-Clopidogrel (Reinigung und enantiomere Anreicherung des Transveresterungsprodukts - Variante A)

Die rohe freie Base des Clopidogrels, die nach der Transveresterung erhalten wurde (16,0 g) und die 8% (R)-Enantiomer und 92% (S)-Enantiomer enthielt, wurde in 240 ml Aceton gelöst. Die Lösung wurde auf 15°C abgekühlt, und 0,43 ml (0,78 g) 96%-ige Schwefelsäure in 10 ml Aceton wurde unter Rühren zugegeben. Nach etwa 2,5 Stunden bildeten sich Kristalle des racemischen Clopidogrelsulfatdihydrats, die abfiltriert wurden, während das (S)-Enantiomer in Lösung blieb. Das Filtrat wurde unter vermindertem Druck abgedampft, und der Rückstand wurde zwischen Methyl-*tert*.butylether und gesättigter wäßriger Natriumhydrogencarbonatlösung verteilt. Die organische Schicht wurde getrocknet und abgedampft. Man erhielt (*S*)-Clopidogrel als freie Base mit einer optischen Reinheit von 99.0% in einer Ausbeute von 11,2 g.

### Beispiel 23

### (S)-Clopidogrel (Reinigung und enantiomere Anreicherung des Transveresterungsprodukts - Variante B)

Die Reinigung des Transveresterungsprodukts wurde wie in Beispiel 20 durchgeführt, jedoch wurde vor der Ausfällung des racemischen Clopidogrelsulfats Triethylamin oder ein anderes geeignetes flüchtiges tertiäres Amin in einer dem enantiomeren Überschuß von (*S*)-Clopidogrel entsprechenden Menge zugegeben. Das Amin bildet ein Doppelsulfatsalz mit (*S*)-Clopidogrel mit erhöhter Löslichkeit, was zur Erhöhung der optischen Reinheit des Produkts beiträgt. Das flüchtige Amin kann nach der Salzzersetzung, der Extraktion der freien Base in ein organisches Lösungsmittel und dem Abdampfen leicht entfernt werden.

### Beispiel 24

### Herstellung von Clopidogrel

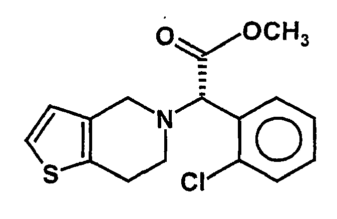

durch Transveresterung des Maleats des (S)-(2-Chlorphenyl)-4,5,6,7-tetrahydro-[3,2-c]thienopyridin-5-ylessigsäure-(R)-1-(dimethylamino)-2-propylesters durch Katalyse mit Zinkchlorid im basischen Medium (bevorzugtes Verfahren)

25,2 g (0,185 Mol) wasserfreies Zinkchlorid und 27 ml (21,6 g 0,25 Mol) N-Methylpyrolidin wurden in 550 ml heißem Methanol (maximaler Wassergehalt 0,1%) gelöst. Zu der Lösung wurden unter Rückfluß 65 g (0,128 Mol) des gemäß Beispiels 16 hergestellten Produktes mit einem Gehalt an (S)-(2-Chlorphenyl)-4,5,6,7-tetrahydro-[3,2-c]thienopyridin-5-ylessigsäure-(R)-1-(dimethylamino)-2-propylestermaleatsalz von ca. 98,0% und einem Gehalt an entsprechendem (R,R)-Enantiomeren von ca. 0,5% zugegeben (die restlichen 1,5% sind nicht bestimmte Verunreinigungen). Das Gemisch wurde auf einem Wasserbad zum Rückfluß erhitzt, bis die Umsetzung im wesentlichen abgeschlossen war (Transveresterung von 97,5 bis 98,5% gemäß HPLC). Nach Beendigung der Transveresterung wurde das Gemisch abgekühlt, das Methanol wurde in einem Rotationsverdampfer unter vermindertem Druck entfernt, und der Rückstand wurde mit 160 ml einer 10%-igen wäßrigen Natriumhydrogencarbonatlösung und 200 ml Diisopropylether vermischt. 25%-iger wäßriger Ammoniak wurde schrittweise zugegeben, bis die Suspension aus Zinkhydroxid und basischem Zinkcarbonat aufgelöst war (ca. 70 ml). Die organische Phase wurde abgetrennt, und die wäßrige Phase wurde 2 x mit 40 ml Diisopropylether extrahiert. Während der Extraktion wurde die Temperatur auf 12 bis 15°C gehalten, und der Gehalt an Clopidogrel in der wäßrigen Schicht wurde über HPLC kontrolliert. Die organischen Schichten wurden vereint und 3 x mit 60 ml Kochsalzlösung gewaschen. Die organische Schicht wurde über Natriumsulfat getrocknet, filtriert und abgedampft. Die Ausbeute an roher Clopidogrelbase war quantitativ (41 g).

Das erhaltene Clopidogrel enthielt 93 bis 96% des gewünschten S-(+)-Clopidogrels und 4 bis 7% des unerwünschten S-(-)-Clopidogrels und wurde in 4 bis 8 ml/g Aceton gelöst und 20% seines molaren Äquivalents an konzentrierter Schwefelsäure wurde schrittweise unter Kühlung (15°C) zugegeben. Das racemische Clopidogrelhydrogensulfat fiel langsam aus der Lösung aus. Das Umsetzungsgemisch wurde über Nacht gerührt, und der racemische Feststoff wurde durch Filtration abgetrennt. Das gewünschte S-(+)-Enantiomer des Clopidogrelhydrogensulfats verblieb in der Mutterlauge. Seine Konzentration wurde auf etwa 25% eingestellt, und es wurde weiter tropfenweise unter Kühlen auf 15°C konzentrierte Schwefelsäure bis zu einem Gehalt von 1,05 Äquivalenten zugegeben. Nach Animpfen der Lösung mit dem gewünschten S-(+)-Enantiomer des Clopidogrels erfolgte schnelle Kristallisation. Das Gemisch wurde 5 bis 6 Stunden bei Raumtemperatur gerührt und dann durch einen Sinterfilter S2 filtriert, mit 50 ml Aceton gewaschen, zunächst an der Luft und dann in einem Vakuumtrockner bei 50°C und 22 Tor getrocknet. Falls zu Beginn der Schwefelsäurezugabe eine Trübung auftritt, die sich zu einem braunen amorphen Feststoff zusammenballt, muß dieses amorphe Material aus dem Umsetzungsgemisch entfernt werden. Es handelt sich hierbei um amorphe Sulfate von Verunreinigungen bzw. des Ausgangsprodukts, die die folgende Kristallisation des polymorphen Hydrogensulfats beeinträchtigen würden.

Die Ausbeute des Verfahrens betrug 78 bis 63% (bezogen auf reines Ausgangsmaterial), der Schmelzpunkt 182 bis 184°C, und der Gehalt an unerwünschtem R-(-)-Clopidogrel war 0,5 bis 1,2%.

## Patentansprüche

1. Verfahren zur Herstellung einer Verbindung der allgemeinen Formel (la) 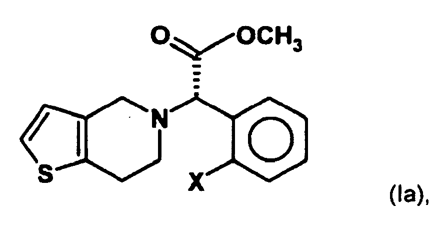 in der X ein Halogenatom darstellt, oder eines pharmazeutisch verträglichen Salzes davon, umfassend den Verfahrensschritt, daß eine Verbindung der Formel (II) 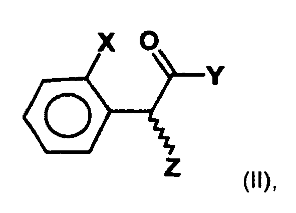 in der X wie vorstehend definiert ist und Y und Z unabhängig voneinander jeweils eine Abgangsgruppe darstellen, mit einem optisch aktiven Aminoalkohol zu einem ersten Gemisch aus Diastereomeren umgesetzt wird.

2. Verfahren nach Anspruch 1, bei dem das erste Gemisch aus Diastereomeren weiter mit einer Verbindung der Formel (V) 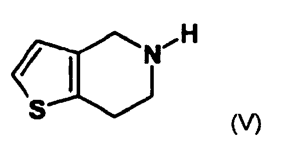 oder einer Verbindung der Formel (VII) 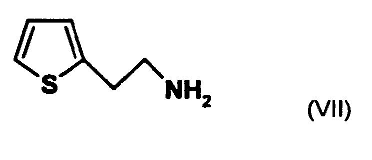 zu einem zweiten Gemisch aus Diastereomeren umgesetzt wird, das gegebenenfalls nach Abtrennung eines Diastereomeren weiter zu der Verbindung der Formel (Ia) umgesetzt wird.

3. Verfahren nach Anspruch 2, bei dem die Umsetzung des zweiten Gemisches aus Diastereomeren zu der Verbindung der Formel (Ia) eine Umesterung in Gegenwart eines Ti- oder Si-Katalysators umfaßt.

4. Verfahren nach Anspruch 3, bei dem der Ti-Katalysator ein Umsetzungsprodukt aus Ethylenglycol und einem Titan(IV)alkoxid und der Si-Katalysator ein chloriertes Siliciumdioxid ist.

5. Verfahren nach Anspruch 2, bei dem die Umsetzung des zweiten Gemischs aus Diastereomeren zu der Verbindung der Formel (Ia) eine Umesterung in Gegenwart eines Katalysators umfaßt, bei dem es sich um ein Halogenid eines Übergangsmetalls der ersten oder zweiten Nebengruppe des Periodensystems der Elemente handelt.

6. Verfahren nach Anspruch 5, wobei es sich bei dem Katalysator um einen Katalysator handelt, der ausgewählt ist aus ZnX₂, Cu₂X₂, CuX₂, AgX, AuX, AuX₃, CdX₂, Hg₂X₂, CoX₂ und HgX₂, wobei X ein Halogenidion darstellt, ausgewählt aus Fluorid, Chlorid, Bromid und lodid.

7. Verfahren nach Anspruch 6, wobei es sich bei dem Katalysator um Zinkchlorid handelt.

8. Verfahren nach einem der Ansprüche 5 bis 7, bei dem die Umesterung in Gegenwart einer organischen Base stattfindet.

9. Verfahren nach einem der Ansprüche 1 bis 8, bei dem in dem ersten Gemisch aus Diastereomeren das Verhältnis des einen Diastereomeren zu dem zweiten Diastereomeren 2:1 oder höher ist.

10. Verfahren nach Anspruch 9, bei dem in dem ersten Gemisch aus Diastereomeren das Verhältnis des einen Diastereomeren zu dem zweiten Diastereomeren 3:1 oder höher ist.

11. Verfahren nach Anspruch 9 oder 10, bei dem das Reaktionsprodukt aus der Verbindung der Formel II 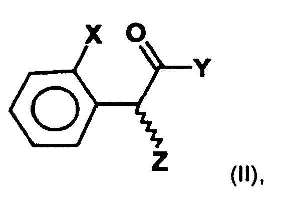 und dem optisch aktiven Aminoalkohol mit einem Keton unter Rückfluss erhitzt wird.

12. Verfahren nach einem der Ansprüche 2 bis 11, bei dem in dem zweiten Gemisch aus Diastereomeren das Verhältnis des einen Diastereomeren zu dem zweiten Diastereomeren 3:1 oder höher ist.

13. Verfahren nach Anspruch 12, bei dem in dem zweiten Gemisch aus Diastereomeren das Verhältnis des einen Diastereomeren zu dem zweiten Diastereomeren 9:1 oder höher ist.

14. Verfahren nach einem der Ansprüche 2 bis 13, bei dem von dem zweiten Gemisch aus Diastereomeren ein Diastereomer abgetrennt wird, das dann weiter zu der Verbindung der Formel (la) umgesetzt wird.

15. Verfahren nach einem der Ansprüche 2 bis 13, bei dem das zweite Gemisch aus Diastereomeren ohne vorherige Abtrennung eines Diastereomeren zu einem Gemisch der Verbindungen (la) 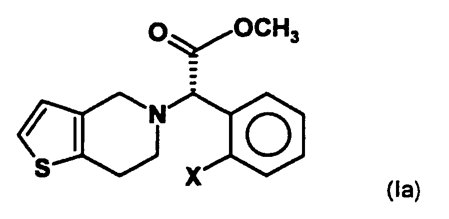 und (Ib) 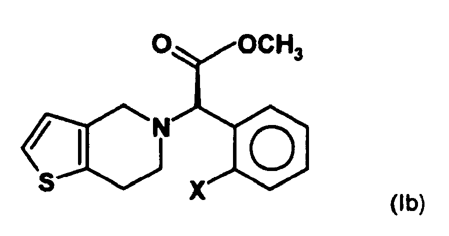 umgesetzt wird, wobei X wie vorstehend definiert ist, und anschließend aus dem Gemisch der Verbindungen der Formeln (Ia) und (Ib) die Verbindung der Formel (Ia) isoliert wird, die gegebenenfalls in ein pharmazeutisch verträgliches Salz davon überführt wird.

16. Verfahren nach Anspruch 15, wobei die Isolierung der Verbindung der Formel (Ia) aus dem Gemisch der Verbindung der Formel (Ia) und (Ib) den Zusatz einer anorganischen Säure zu dem Gemisch, das Ausfällen und Abtrennen des Salzes des Racemats der Verbindungen der Formel (Ia) und (Ib), den Zusatz von weiterer anorganischer Säure zu der Mutterlauge und das Ausfällen und Abtrennen des Salzes der Verbindung der Formel (la) umfasst.

17. Verfahren nach einem der Ansprüche 2 bis 16, bei dem die Diastereomeren des zweiten Diastereomerengemischs in das Salz einer Dicarbonsäure überführt werden, das dann einer Umesterung zu einem Gemisch aus den Verbindungen der Formel (Ia) und (Ib) unterworfen wird.

18. Verfahren nach Anspruch 17, wobei die Dicarbonsäure Maleinsäure ist.

19. Verfahren nach einem der Ansprüche 1 bis 18, wobei X ein Chloratom darstellt.

20. Verfahren nach einem der Ansprüche 1 bis 19, wobei der optisch aktive Aminoalkohol die Formel (III) 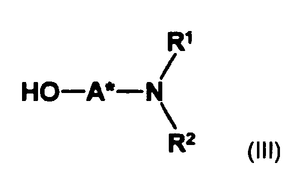 aufweist, wobei A* einen Kohlenwasserstoffrest mit 1 bis 30 Kohlenstoffatomen darstellt, der bis zu 5 Heteroatome enthalten kann, ausgewählt aus Stickstoff-, Sauerstoff-, Schwefel- und Halogenatomen, mit bis zu 5 Substituenten substituiert sein kann, ausgewählt aus Hydroxylgruppen, Oxogruppen, Cyanogruppen und Nitrogruppen, und der eine oder mehrere optisch aktive Einheiten aufweist, und
R¹ und R² jeweils unabhängig voneinander Wasserstoffatome oder Kohlenwasserstoffreste mit 1 bis 20 Kohlenstoffatomen darstellen, die jeweils bis zu 4 Heteroatome, ausgewählt aus Stickstoff-, Sauerstoff-, Schwefel- und Halogenatomen, und bis zu 5 Substituenten, ausgewählt aus Hydroxylgruppen, Oxogruppen, Cyanogruppen und Nitrogruppen, aufweisen können, oder
einer oder beide der Reste R¹ und R² mit einem Kohlenstoffatom oder einem Heteroatom des Rests A* einen 5- bis 1 0-gliedrigen gesättigten oder ungesättigten Ring bildet, der neben dem Stickstoffatom gegebenenfalls noch 1 bis 3 weitere Heteroatome, ausgewählt aus Stickstoff-, Sauerstoff- und Schwefelatomen, als Ringglieder enthält, und der mit bis zu 5 Substituenten, ausgewählt aus C₁-C₆-Alkylresten, C₂-C₆-Alkenylresten, C₁-C₆-Alkoxyresten, C₅-C₁₀-Arylresten, C₅-C₁₀-Heteroarylresten, C₃-C₈-Cycloalkylresten, C₂-C₈-Heterocycloalkylresten, Halogenatomen, Hydroxylgruppen, Oxogruppen, Cyanogruppen und Nitrogruppen, substituiert sein kann.

21. Verfahren nach Anspruch 20, bei dem die Reste R¹ und R² unabhängig voneinander einen C₁-C₆-Alkylrest, einen C₅-C₁₀-Arylrest, einen C₅-C₁₀-Heteroarylrest, einen C₃-C₈-Cycloalkylrest oder einen C₂-C₈-Heterocycloalkylrest darstellen oder zusammen mit dem Stickstoffatom, an das sie gebunden sind, einen gesättigten oder einfach ungesättigten Ring mit 3 bis 8 Kohlenstoffatomen darstellen, der gegebenenfalls mit einer C₁-C₆-Alkylgruppe oder einem Halogenatom substituiert ist und neben dem Stickstoffatom 1 oder 2 weitere Heteroatome, ausgewählt aus Schwefelatomen, Stickstoffatomen und Sauerstoffatomen, enthalten kann.

22. Verfahren nach Anspruch 20 oder 21, bei dem der Rest HO-A* einen Rest der Formel 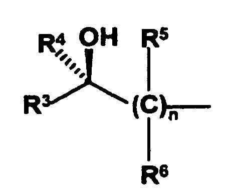 darstellt, wobei die Reste R³ bis R⁶ jeweils unabhängig voneinander Wasserstoffatome oder Kohlenwasserstoffreste mit 1 bis 20 Kohlenstoffatomen darstellen, die jeweils bis zu 4 Heteroatome, ausgewählt aus Stickstoff-, Sauerstoff-, Schwefel- und Halogenatomen, und bis zu 5 Substituenten, ausgewählt aus Hydroxylgruppen, Oxogruppen, Cyanogruppen und Nitrogruppen, aufweisen können, oder
einer oder zwei der Reste R³ bis R⁶ mit dem Rest R¹ bzw. dem Rest R² einen 5- bis 10-gliedrigen gesättigten oder ungesättigten Ring bilden, der neben dem Stickstoffatom gegebenenfalls noch 1 bis 3 weitere Heteroatome, ausgewählt aus Stickstoff-, Sauerstoff- und Schwefelatomen, als Ringglieder enthält, und der mit bis zu 5 Substituenten, ausgewählt aus C₁-C₆-Alkylresten, C₂-C₆-Alkenylresten, C₁-C₆-Alkoxyresten, C₅-C₁₀-Arylresten, C₅-C₁₀-Heteroarylresten, C₃-C₈-Cycloalkylresten, C₂-C₈-Heterocycloalkylresten, Halogenatomen, Hydroxylgruppen, Oxogruppen, Cyanogruppen und Nitrogruppen, substituiert sein kann, und n eine ganze Zahl von 1 bis 3 darstellt.

23. Verfahren nach Anspruch 22, bei dem die Reste R⁵ und R⁶ unabhängig voneinander aus Wasserstoff und C₁-C₆-Alkylresten ausgewählt sind.

24. Verfahren nach Anspruch 23, bei dem nur einer der Reste R⁵ und R⁶ von einem Wasserstoffatom verschieden ist.

25. Verfahren nach Anspruch 24, bei dem alle Reste R⁵ und R⁶ Wasserstoffatome darstellen.

26. Verfahren nach einem der Ansprüche 22 bis 25, bei dem der Index n 1 oder 2 ist.

27. Verfahren nach einem der Ansprüche 22 bis 26, bei dem der Rest R³ einen C₁-C₆-Alkyl-, C₅-C₁₀-Aryl-, C₅-C₁₀-Heteroaryl-, C₂-C₈-Heterocycloalkyl- oder C₃-C₈-Cycloalkylrest darstellt.

28. Verfahren nach Anspruch 27, bei dem der Rest R³ einen C₁-C₆-Alkylrest darstellt.

29. Verfahren nach einem der Ansprüche 22 bis 28, bei dem der Rest R⁴ ein Wasserstoffatom darstellt.

30. Verfahren nach Anspruch 22, bei dem der Rest R³ mit dem Rest R² zu einem Ring verbunden ist, der 5 bis 10 Ringatome aufweist und der neben dem Stickstoffatom, an das der Rest R² gebunden ist, noch 1 bis 3 weitere Heteroatome, ausgewählt aus Sauerstoff-, Stickstoff- und Schwefelatomen, aufweisen kann, und der gesättigt oder einfach oder zweifach ungesättigt sein kann und der 1 bis 3 Substituenten aufweisen kann, ausgewählt aus C₁-C₆-Alkylresten, C₅-C₁₀-Arylresten, C₅-C₁₀-Heteroarylresten, C₃-C₈-Cycloalkylresten, C₂-C₈-Heterocycloalkylresten und Halogenatomen.

31. Verfahren nach Anspruch 20 oder 21, bei dem der Rest HO-A* einen Rest der Formel 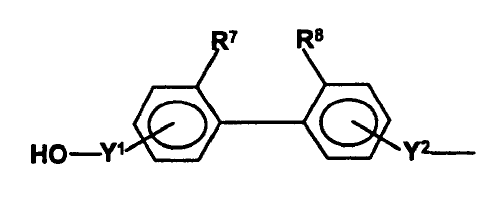 oder 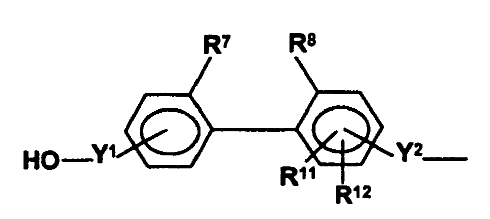 darstellt, wobei Y¹ einen Rest 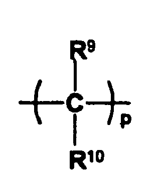 und Y² einen Rest 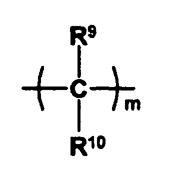 darstellt, in dem R⁹ und R¹⁰ unabhängig voneinander aus Wasserstoffatomen und C₁-C₆-Alkylresten ausgewählt sind und die Reste R⁷ und R⁸ Gruppen darstellen, die die freie Drehbarkeit der beiden Phenylgruppen relativ zueinander verhindern, R¹¹ und R¹² unabhängig voneinander Wasserstoffatome, C₁-C₄-Alkoxyreste, Halogenatome, Cyanoreste, C₁-C₈-Alkoxycarbonylreste, C₁-C₆-Alkylreste darstellen oder R¹¹ und R¹² zusammen mit dem Benzolring, an den sie gebunden sind, eine kondensierte Ringstruktur bilden, die ausgewählt ist aus einer 1,3-Benzodioxolyl-, α-Naphthyl-, β-Naphthyl-, Tetrahydronaphthyl-, Benzimidazolyl- und Benztriazolylstruktur, m eine ganze Zahl von 0 bis 2 ist und p 1 oder 2 darstellt.

32. Verfahren nach Anspruch 31, bei dem einer der Reste R⁷ und R⁸ ausgewählt ist aus einer C₁-C₆-Alkylgruppe, einem Halogenatom und einer Cyanogruppe und der zweite ausgewählt ist aus einer C₃-C₆-verzweigten Alkylgruppe, bevorzugt eine tert.-Butylgruppe, einem Halogenatom und einer Cyanogruppe.

33. Verfahren nach Anspruch 20, bei dem die Verbindung der Formel (III) 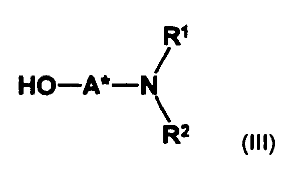 eine Verbindung der Formel 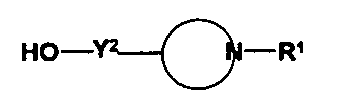 ist, in der Y² wie in Anspruch 31 definiert ist, R¹ wie in Anspruch 20 definiert ist und die Gruppe 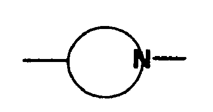 einen 5- bis 1 0-gliedrigen gesättigten oder ungesättigten Ring darstellt, der neben dem Stickstoffatom gegebenenfalls noch 1 bis 3 weitere Heteroatome, ausgewählt aus Stickstoff-, Sauerstoff- und Schwefelatomen, als Ringglieder enthält, und der mit bis zu 5 Substituenten, ausgewählt aus C₁-C₆-Alkylresten, C₂-C₆-Alkenylresten, C₁-C₆-Alkoxyresten, C₅-C₁₀-Arylresten, C₅-C₁₀-Heteroarylresten, C₃-C₈-Cycloalkylresten, C₂-C₈-Heterocycloalkylresten, Halogenatomen, Hydroxylgruppen, Oxogruppen, Cyanogruppen und Nitrogruppen, substituiert sein kann.

34. Verfahren nach Anspruch 33, bei dem die Gruppe 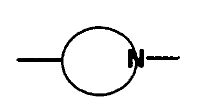 eine Gruppe 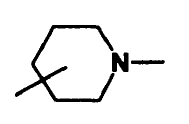 darstellt.

35. Gemisch der Diastereomeren (IVa) 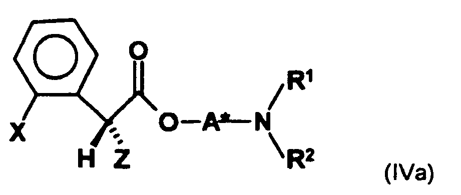 und (IVb) 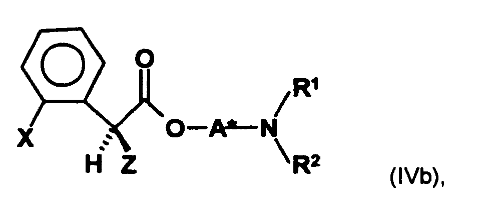 wobei X und Z wie in Anspruch 1 definiert sind und A*, R¹ und R² wie in einem der Ansprüche 20 bis 33 definiert sind.

36. Gemisch nach Anspruch 35, in dem das Verhältnis zwischen dem Diastereomer (IVa) und dem Diastereomer (IVb) 2:1 oder größer ist.

37. Gemisch nach Anspruch 36, in dem das Verhältnis zwischen dem Diastereomer (IVa) und dem Diastereomer (IVb) 3:1 oder größer ist.

38. Verbindung der Formel (IVa) 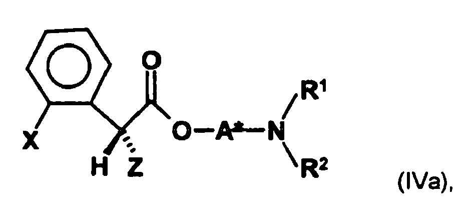 wobei die Reste X, Z, A*, R¹ und R² wie in Anspruch 35 definiert sind.

39. Gemisch der Diastereomeren (VIa) 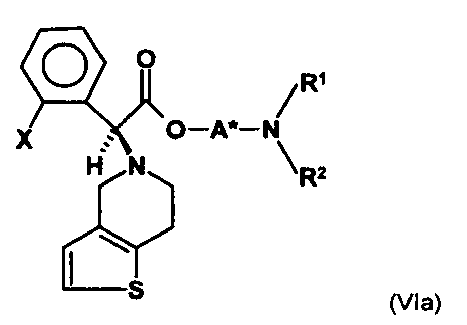 und (VIb) 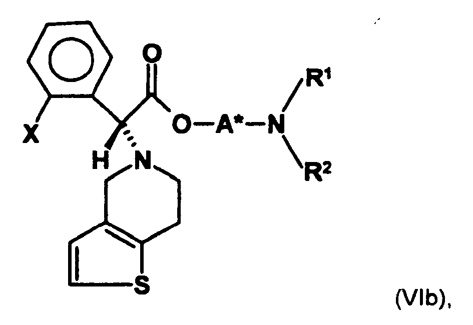 wobei der Rest X wie in Anspruch 1 definiert ist und die Reste A*, R¹ und R² wie in einem der Ansprüche 20 bis 34 definiert sind oder der Salze davon.

40. Gemisch nach Anspruch 39, wobei die Diastereomeren als Maleatsalz, Fumaratsalz oder Oxalatsalz vorliegen.

41. Gemisch nach Anspruch 40, in dem das Verhältnis zwischen dem Diastereomer (VIa) und dem Diastereomer (VIb) 3:1 oder größer ist.

42. Gemisch nach Anspruch 41, das 98% oder mehr an Diastereomer (VIa) und 2% oder weniger an Diastereomer (VIb) enthält.

43. Verbindung der Formel (VIa) 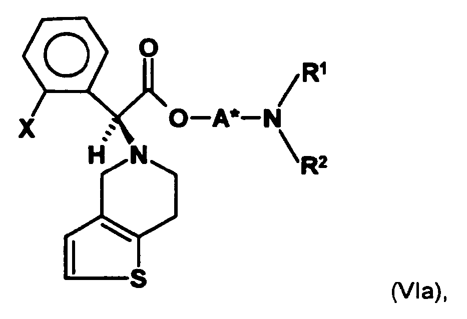 wobei die Reste X, A*, R¹ und R² wie in Anspruch 39 definiert sind, oder ein Salz davon.

44. Verbindung nach Anspruch 43 in Form des Maleatsalzes, Fumaratsalzes oder Oxalatsalzes.

45. Gemisch der Diastereomeren (VIIIa) 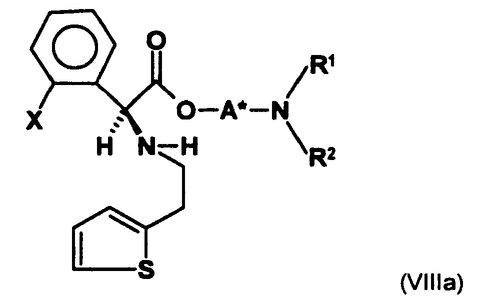 und (Vlllb) 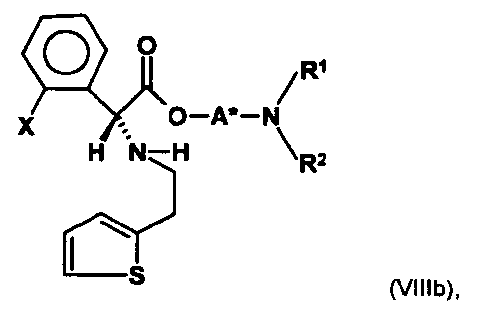 wobei der Rest X wie in Anspruch 1 definiert ist und die Reste A*, R¹ und R² wie in einem der Ansprüche 20 bis 34 definiert sind, oder der Salze davon.

46. Gemisch nach Anspruch 45, wobei die Diastereomere als Maleatsalz, Fumaratsalz oder Oxalatsalz vorliegen.

47. Gemisch nach Anspruch 46, in dem das Verhältnis zwischen den Diastereomeren (VIIIa) und (VIIIb) 3:1 oder größer ist.

48. Gemisch nach Anspruch 47, in dem das Diastereomer (VIIIa) zu 98% oder mehr und das Diastereomer (VIIIb) zu 2% oder weniger vorhanden ist.

49. Verbindung der Formel (VIIIa) 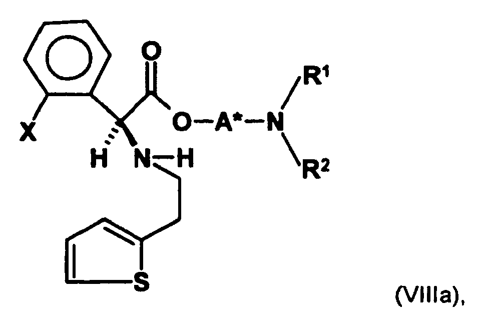 wobei die Reste X, A*, R¹ und R² wie in Anspruch 45 definiert sind, oder eines Salzes davon.

50. Verbindung nach Anspruch 49 in Form des Maleatsalzes, Fumaratsalzes oder Oxalatsalzes.

## Claims

1. A process for preparing a compound of the general formula (Ia) 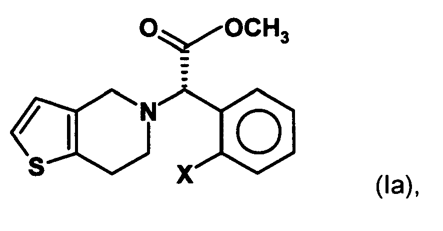 wherein X is a halogen atom, or a pharmaceutically acceptable salt thereof, comprising the process step that a compound of the formula (II) 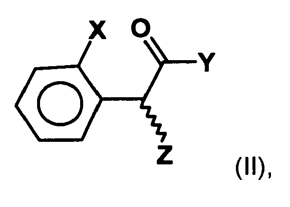 wherein X is as defined above and Y and Z each independently represent a leaving group is reacted with an optically active amino alcohol to form a first mixture of diastereomers.

2. A process according to claim 1, wherein the first mixture of diastereomers is further reacted with a compound of the formula (V) 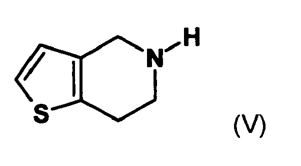 or a compound of the formula (VII) 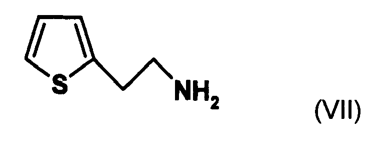 to form a second mixture of diastereomers which is further reacted after optional separation of one diastereomer to form the compound of the formula (Ia).

3. A process according to claim 2, wherein the reaction of the second mixture of diastereomers to form the compound of the formula (Ia) comprises a transesterification in the presence of a Ti- or Si-catalyst.

4. A process according to claim 3, wherein the Ti-catalyst is a product of the reaction of ethylene glycol and a titanium(IV) alkoxide and the Si-catalyst is a chlorinated silica.

5. A process according to claim 2, wherein the reaction of the second mixture of diastereomers to form the compound of the formula (la) comprises a transesterification in the presence of a catalyst which is a halide of a transition metal of the first or second sub-group of the periodic table of elements.

6. A process according to claim 5, wherein the catalyst is a catalyst selected from ZnX₂, Cu₂X₂, CuX₂, AgX, AuX, AuX₃, CdX₂, Hg₂X₂, CoX₂ and HgX₂, wherein X is a halide ion selected from fluoride, chloride, bromide and iodide.

7. A process according to claim 6, wherein the catalyst is zinc chloride.

8. A process according to any of the claims 5 to 7, wherein the transesterification occcurs in the presence of an organic base.

9. A process according to any of the claims 1 to 8, wherein the ratio of the one diastereomer to the second diastereomer in the first mixture of diastereomers is 2 : 1 or higher.

10. A process according to claim 9, wherein the ratio of the one diastereomer to the second diastereomer in the first mixture of diastereomers is 3 : 1 or higher.

11. A process according to claim 9 or 10, wherein the reaction product of the compound of the formula II 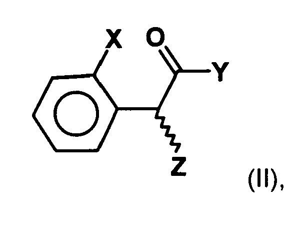 and the optically active amino alcohol is heated with a ketone at reflux.

12. A process according to any of the claims 2 to 11, wherein the ratio of the one diastereomer to the second diastereomer in the second diastereomer mixture is 3 : 1 or higher.

13. A process according to claim 12, wherein the ratio of the one diastereomer to the second diastereomer in the second diastereomer mixture is 9 : 1 or higher.

14. A process according to any of the claims 2 to 13, wherein a diastereomer is separated from the second mixture of diastereomers which is then further reacted to form the compound of the formula (Ia).

15. A process according to any of the claims 2 to 13, wherein the second mixture of diastereomers is reacted without prior separation of a diastereomer to form a mixture of the compounds (Ia) 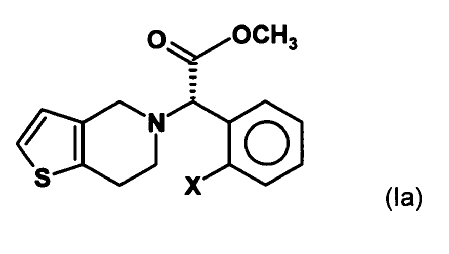 and (Ib) 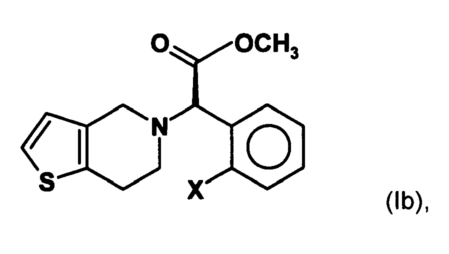 wherein X is as defined above, and the compound of the formula (Ia) is subsequently isolated from the mixture of the compounds of the formulae (Ia) and (Ib) and, optionally, converted to a pharmaceutically acceptable salt thereof.

16. A process according to claim 15, wherein the isolation of the compound of the formula (Ia) from the mixture of the compound of the formula (Ia) and (Ib) comprises the addition of an inorganic acid to the mixture, the precipitation and separation of the salt of the racemate of the compounds of the formula (Ia) and (Ib), the addition of additional inorganic acid to the mother liquor and the precipitation and separation of the salt of the compound of the formula (Ia).

17. A process according to any of the claims 2 to 16, wherein the diastereomers of the second diastereomer mixture are converted to the salt of a dicarboxylic acid which is then subjected to transesterification to form a mixture of the compounds of the formula (Ia) and (Ib).

18. A process according to claim 17, wherein the dicarboxylic acid is maleic acid.

19. A process according to any of the claims 1 to 18, wherein X is a chlorine atom.

20. A process according to any of the claims 1 to 19, wherein the optically active amino alcohol has the formula (III) 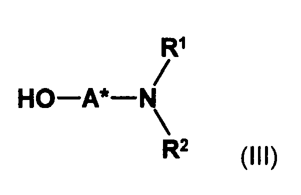 wherein A* represents a hydrocarbon radical with 1 to 30 carbon atoms which may contain up to 5 heteroatoms selected from nitrogen, oxygen, sulfur and halogen atoms and which may be substituted with up to 5 substituents selected from hydroxyl groups, oxo groups, cyano groups and nitro groups and which has one or more optically active units, and
R¹ and R² independently represent hydrogen atoms or hydrocarbon radicals with 1 to 20 carbon atoms, each of which may comprise up to 4 hetero atoms selected from nitrogen, oxygen, sulfur and halogen atoms and which may be substituted with up to 5 substituents selected from hydroxyl groups, oxo groups, cyano groups and nitro groups, or
one or both of the radicals R¹ and R² form(s) a 5- to 10-membered saturated or unsaturated ring with a carbon atom or a heteroatom of the radical A* which, in addition to the nitrogen atom, may optionally contain 1 to 3 additional hetero atoms selected from nitrogen, oxygen and sulfur atoms as ring members and which may be substituted with up to 5 substituents selected from C₁-C₆-alkyl radicals, C₂-C₆-alkenyl radicals, C₁-C₆-alkoxy radicals, C₅-C₁₀-aryl radicals, C₅-C₁₀-heteroaryl radicals, C₃-C₈-cycloalkyl radicals, C₂-C₈-heterocycloalkyl radicals, halogen atoms, hydroxyl groups, oxo groups, cyano groups and nitro groups.

21. A process according to claim 20, wherein the radicals R¹ and R² independently represent a C₁-C₆-alkyl radical, a C₅-C₁₀-aryl radical, a C₅-C₁₀-heteroaryl radical, a C₃-C₈-cycloalkyl radical or a C₂-C₈-heterocycloalkyl radical, or, together with the nitrogen atom to which they are bound, represent a saturated or mono-unsaturated ring with 3 to 8 carbon atoms which, optionally, is substituted with a C₁-C₆-alkyl group or a halogen atom and which, in addition to the nitrogen atom may contain 1 or 2 additional heteroatoms selected from sulfur atoms, nitrogen atoms and oxygen atoms.

22. A process according to claim 20 or 21, wherein the radical HO-A* represents a radical of the formula 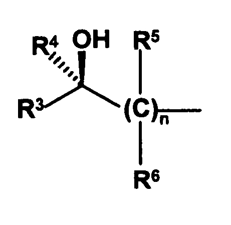 wherein each of the radicals R³ to R⁶ independently is a hydrogen atom or a hydrocarbon radical with 1 to 20 carbon atoms, each of which may have up to 4 heteroatoms selected from nitrogen, oxygen, sulfur and halogen atoms and may have up to 5 substituents selected from hydroxyl groups, oxo groups, cyano groups and nitro groups, or
one or two of the radicals R³ to R⁶ may form a 5- to 10-membered saturated or unsaturated ring with the radical R¹ or the radical R² which, in addition to the nitrogen atom, optionally contains 1 to 3 additional heteroatoms selected from nitrogen, oxygen and sulfur atoms as ring members and which may be substituted with up to 5 substituents selected from C₁-C₆-alkyl radicals, C₂-C₆-alkenyl radicals, C₁-C₆-alkoxy radicals, C₅-C₁₀-aryl radicals, C₅-C₁₀-heteroaryl radicals, C₃-C₈-cycloalkyl radicals, C₂-C₈-heterocycloalkyl radicals, halogen atoms, hydroxyl groups, oxo groups, cyano groups and nitro groups, and wherein n is an integer from 1 to 3.

23. A process according to claim 22, wherein the radicals R⁵ and R⁶ are independently selected from hydrogen and C₁-C₆-alkyl radicals.

24. A process according to claim 23, wherein only one of the radicals R⁵ and R⁶ is different from a hydrogen atom.

25. A process according to claim 24, wherein all of the radicals R⁵ and R⁶ are hydrogen atoms.

26. A process according to any of the claims 22 to 25, wherein the index n is 1 or 2.

27. A process according to any of the claims 22 to 26, wherein the radical R³ is a C₁-C₆-alkyl, C₅-C₁₀-aryl, C₅-C₁₀-heteroaryl, C₂-C₈-heterocycloalkyl or C₃-C₈-cycloalkyl radical.

28. A process according to claim 27, wherein the radical R³ is a C₁-C₆-alkyl radical.

29. A process according to any of the claims 22 to 28, wherein the radical R⁴ is a hydrogen atom.

30. A process according to claim 22, wherein the radical R³ forms a ring with the radical R², which ring has 5 to 10 ring atoms and which, in addition to the nitrogen atom which the radical R² is bonded to, may comprise 1 to 3 additional hetero atoms selected from oxygen, nitrogen and sulfur atoms and which may be saturated or mono- or bi-unsaturated and which may have 1 to 3 substituents selected from C₁-C₆-alkyl radicals, C₅-C₁₀-aryl radicals, C₅-C₁₀-heteroaryl radicals, C₃-C₈-cycloalkyl radicals, C₂-C₈-heterocycloalkyl radicals and halogen atoms.

31. A process according to claim 20 or 21, wherein the radical HO-A* represents a radical of the formula 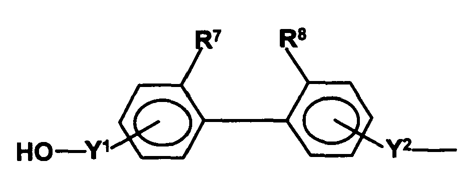 or 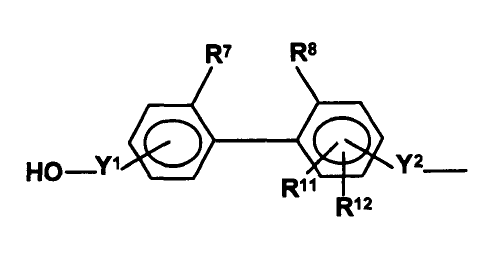 Y¹ representing a radical 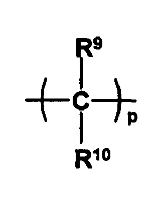 and Y² representing a radical 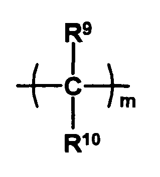 wherein R⁹ and R¹⁰ are independently selected from hydrogen atoms and C₁-C₆-alkyl radicals and the radicals R⁷ and R⁸ are groups preventing free rotatability of the two phenyl groups relatively to each other, R¹¹ and R¹² independently are hydrogen atoms, C₁-C₄-alkoxy radicals, halogen atoms, cyano radicals, C₁-C₆-alkoxycarbonyl radicals or C₁-C₆-alkyl radicals, or R¹¹ and R¹², together with the benzene ring to which they are bonded, form a condensed ring structure which is selected from a 1,3-benzodioxolyl, α-naphthyl, β-naphthyl, tetrahydronaphthyl, benzimidazolyl and benztriazolyl structure, m is an integer from 0 to 2 and p is 1 or 2.

32. A process according to claim 31, wherein one of the radicals R⁷ and R⁸ is selected from a C₁-C₆-alkyl group, a halogen atom and a cyano group and the second one is selected from a C₃-C₆ branched alkyl group, preferably a tert.-butyl group, a halogen atom and a cyano group.

33. A process according to claim 20, wherein the compound of the formula (III) 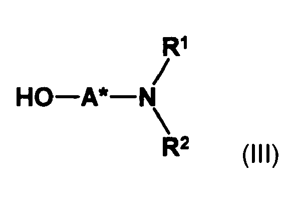 is a compound of the formula 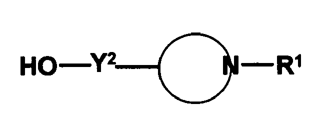 wherein Y² is as defined in claim 31, R¹ is as defined in claim 20 and the group 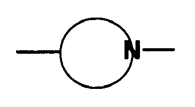 is a 5- to 10-membered saturated or unsaturated ring which, in addition to the nitrogen atom, optionally contains 1 to 3 hetero atoms selected from nitrogen, oxygen and sulfur atoms as ring members and which may be substituted with up to 5 substituents selected from C₁-C₆-alkyl radicals, C₂-C₆-alkenyl radicals, C₁-C₆-alkoxy radicals, C₅-C₁₀-aryl radicals, C₅-C₁₀-heteroaryl radicals, C₃-C₈-cycloalkyl radicals, C₂-C₈-heterocycloalkyl radicals, halogen atoms, hydroxyl groups, oxo groups, cyano groups and nitro groups.

34. A process according to claim 33, wherein the group 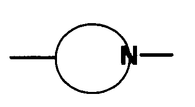 is a group

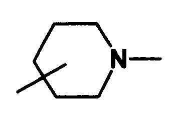

35. A mixture of the diastereomers (IVa) 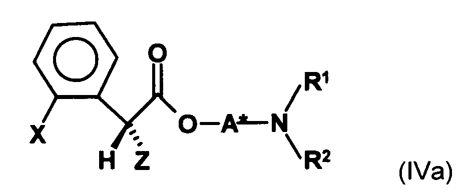 and (IVb) 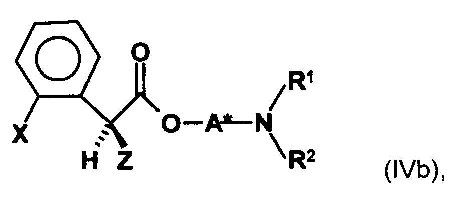 wherein X and Z are as defined in claim 1 and A*, R¹ and R² are as defined in any of the claims 20 to 33.

36. A mixture according to claim 35, wherein the ratio between the diastereomer (IVa) and the diastereomer (IVb) is 2 : 1 or higher.

37. A mixture according to claim 36, wherein the ratio between the diastereomer (IVa) and the diastereomer (IVb) is 3 : 1 or higher.

38. A compound of the formula (IVa) 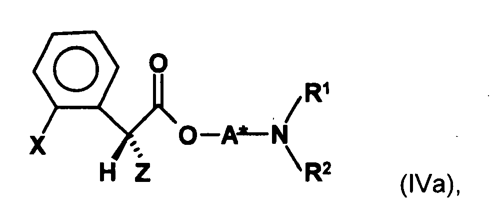 wherein the radicals X, Z, A*, R¹ and R² are as defined in claim 35.

39. A mixture of the diastereomers (VIa) 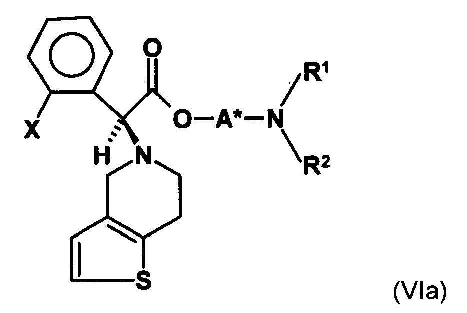 and (VIb) 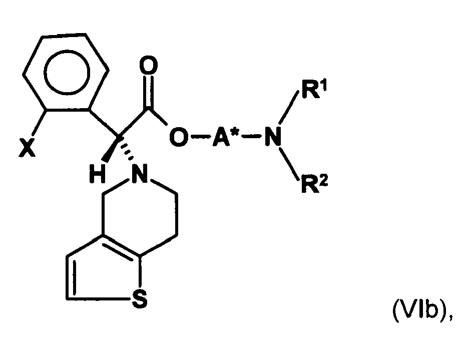 wherein the radical X is as defined in claim 1 and the radicals A*, R¹ and R² are as defined in any of the claims 20 to 34 or of the salts thereof.

40. A mixture according to claim 39, wherein the diastereomers are present as the maleate salt, fumarate salt or oxalate salt.

41. A mixture according to claim 40, wherein the ratio between the diastereomer (VIa) and the diastereomer (Vlb) is 3 : 1 or higher.

42. A mixture according to claim 41 which contains 98 % or more of the diastereomer (VIa) and 2 % or less of the diastereomer (Vlb).

43. A compound of the formula (VIa) 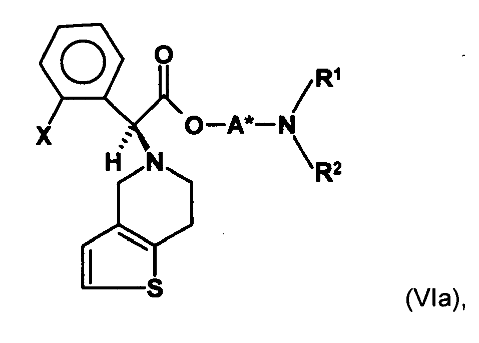 wherein the radicals X, A*, R¹ and R² are as defined in claim 39 or a salt thereof.

44. A compound according to claim 43 as the maleate salt, fumarate salt or oxalate salt.

45. A mixture of the diastereomers (VIIIa) 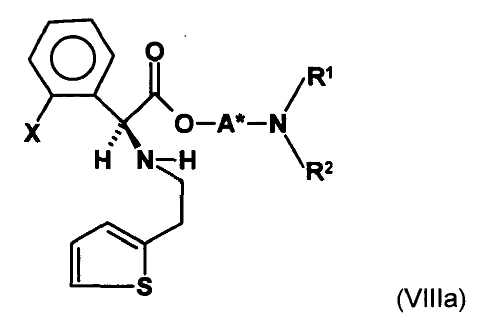 and (Vlllb) 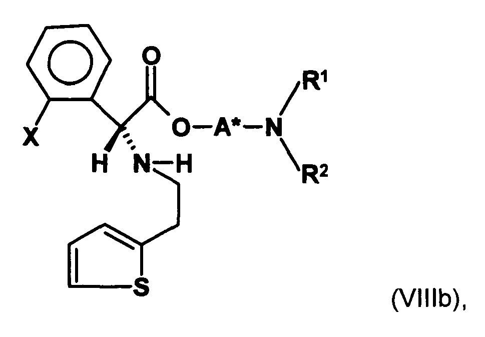 wherein the radical X is as defined in claim 1 and the radicals A*, R¹ and R² are as defined in any of the claims 20 to 34 or of the salts thereof.

46. A mixture according to claim 45, wherein the diastereomers are present as the maleate salt, fumarate salt or oxalate salt.

47. A mixture according to claim 46, wherein the ratio between the diastereomers (VIIIa) and (VIIIb) is 3 : 1 or higher.

48. A mixture according to claim 47, wherein 98 % or more of the diastereomer (VIIIa) and 2 % or less of the diastereomer (VIIIb) are present.

49. A compound of the formula (VIIIa) 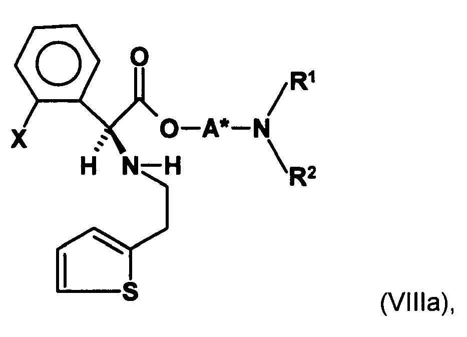 wherein the radicals X, A*, R¹ and R² are as defined in claim 45 or a salt thereof.

50. A compound according to claim 49 in the form of the maleate salt, fumarate salt or oxalate salt.

## Revendications

1. Procédé de préparation d'un composé de formule générale (Ia) 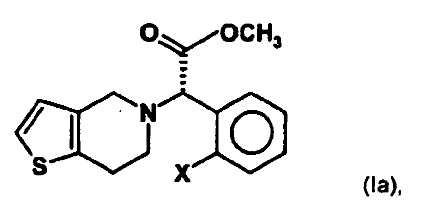 dans laquelle X représente un atome d'halogène, ou un sel compatible sur le plan pharmaceutique de celui-ci, comprenant l'étape de procédé selon lequel un composé de formule (II) 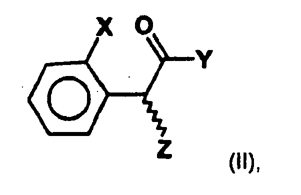 dans laquelle X est comme défini ci-dessus et Y et Z représentent indépendamment les uns des autres chaque fois un groupe de départ, est mis à réagir avec un aminoalcool optiquement actif, pour obtenir un premier mélange de diastéréomères.

2. Procédé selon la revendication 1, dans lequel le premier mélange de diastéréomères est plus loin mis à réagir avec un composé de formule (V) 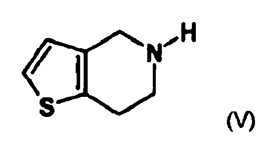 ou avec un composé de formule (VII), 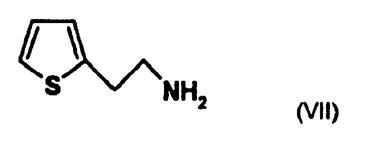 pour obtenir un deuxième mélange de diastéréomères, qui peut éventuellement être mis à réagir après séparation d'un diastéréomère encore pour obtenir le composé de formule (Ia).

3. Procédé selon la revendication 2, dans lequel la réaction du deuxième mélange de diastéréomères pour obtenir le composé de formule (Ia) comprend la transestérification en présence d'un catalyseur de Ti ou de Si.

4. Procédé selon la revendication 3, dans lequel le catalyseur de Ti est un produit réactionnel fait d'éthylèneglycol et d'un alcoxyde de titane (IV) et le catalyseur de Si est un dioxyde de silicium chloré.

5. Procédé selon la revendication 2, dans lequel la réaction du deuxième mélange de diastéréomères pour obtenir le composé de formule (Ia) comprend une transestérification en présence d'un catalyseur, pour lequel il s'agit d'un halogénure d'un métal de transition du premier ou deuxième sous-groupe du système périodique des éléments.

6. Procédé selon la revendication 5, dans lequel, pour le catalyseur, il s'agit un catalyseur choisi parmi les ZnX₂, Cu₂X₂, CuX₂, AgX, AuX, AuX₃, CdX₂, Hg₂X₂, CoX₂ et HgX₂, dans lesquels X représente un ion d'halogénure choisi parmi un fluorure, un chlorure, un bromure et un iodure.

7. Procédé selon la revendication 6, dans lequel il s'agit, pour le catalyseur, de chlorure de zinc.

8. Procédé selon l'une des revendications 5 à 7, dans lequel la transestérification se fait en présence d'une base organique.

9. Procédé selon l'une des revendications 1 à 8, dans lequel, dans le premier mélange de diastéréomères, le rapport du premier diastéréomère au deuxième diastéréomère est de 2:1 ou plus élevé.

10. Procédé selon la revendication 9, dans lequel, dans le premier mélange de diastéréomères, le rapport du premier diastéréomère au deuxième diastéréomère est de 3:1 ou plus élevé.

11. Procédé selon la revendication 9 ou 10, dans lequel le produit réactionnel du composé de formule II 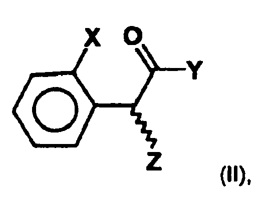 et de l'aminoalcool optiquement actif est chauffé avec une cétone au reflux.

12. Procédé selon l'une des revendications 2 à 11, dans lequel, dans le deuxième mélange de diastéréomères, le rapport du premier diastéréomère au deuxième diastéréomère est de 3:1 ou plus élevé.

13. Procédé selon la revendication 12, dans lequel, dans le deuxième mélange de diastéréomères, le rapport du premier diastéréomère au deuxième diastéréomère est de 9:1 ou plus élevé.

14. Procédé selon l'une des revendications 2 à 13, dans lequel du deuxième mélange de diastéréomères, un diastéréomère est séparé, qui est ensuite mis à réagir pour obtenir le composé de formule (Ia).

15. Procédé selon l'une des revendications 2 à 13, dans lequel le deuxième mélange de diastéréomères est mis à réagir sans séparation préalable d'un diastéréomère pour obtenir un mélange des composés (Ia) 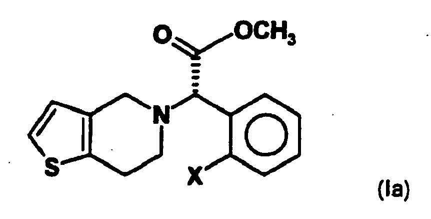 et (Ib) 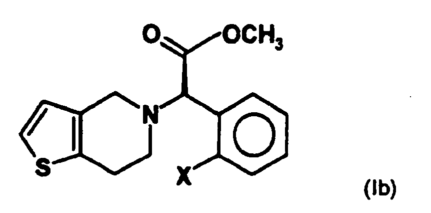 X étant comme défini ci-dessus, et ensuite, du mélange des composés de formules (Ia) et (Ib), le composé de formule (Ia) est isolé, lequel est transformé éventuellement en un sel acceptable sur le plan pharmaceutique de celui-ci.

16. Procédé selon la revendication 15, dans lequel l'isolement du composé de formule (Ia) du mélange du composé de formule (Ia) et (Ib) comprend l'addition d'un acide inorganique au mélange, la précipitation et la séparation du sel de racémate des composés de formule (Ia) et (Ib), l'addition d'un autre acide inorganique à la lessive-mère et la précipitation et la séparation du sel du composé de formule (Ia).

17. Procédé selon l'une des revendications 2 à 16, dans lequel les diastéréomères du deuxième mélange de diastéréomères sont transformés en le sel d'un acide dicarboxylique, lequel est alors soumis à une transestérification pour l'obtention d'un mélange fait des composés de formule (Ia) et (Ib).

18. Procédé selon la revendication 17, dans lequel l'acide dicarboxylique est l'acide maléique.

19. Procédé selon l'une des revendications 1 à 18, dans lequel X représente un atome de chlore.

20. Procédé selon l'une des revendications 1 à 19, dans lequel l'aminoalcool optiquement actif présente la formule (III), 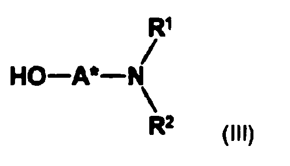 dans laquelle
A* représente un radical hydrocarbure avec 1 à 30 atomes de carbone qui peut contenir jusqu'à 5 hétéroatomes, choisis parmi les atomes d'azote, d'oxygène, de soufre et d'halogène, qui peut être substitué par jusque 5 substituants, choisis parmi les groupes hydroxyle, les groupes oxo, les groupes cyano et les groupes nitro, et qui présente une ou plusieurs unités optiquement actives, et
R¹ et R² représentent chaque fois indépendamment les uns des autres des atomes d'hydrogène ou des radicaux hydrocarbure avec 1 à 20 atomes de carbone, qui peuvent présenter chaque fois jusque 4 hétéroatomes, choisis parmi les atomes d'azote, d'oxygène, de soufre et d'halogène, et jusque 5 substituants, choisis parmi les groupes hydroxyle, les groupes oxo, les groupes cyano et les groupes nitro, ou
un ou les deux des radicaux R¹ et R² forme avec un atome de carbone ou avec un hétéroatome du radical A*, un cycle de 5 à 10 membres saturé ou insaturé, qui contient, outre l'atome d'azote, éventuellement encore 1 à 3 autres hétéroatomes, choisis parmi les atomes d'azote, d'oxygène et de soufre, comme membres de cycle, et qui peut être substitué par jusque 5 substituants, choisis parmi les radicaux alkyle en C₁-C₆, les radicaux alcényle en C₂-C₆, les radicaux alcoxy en C₁-C₆, les radicaux aryle en C₅-C₁₀, les radicaux hétéroaryle en C₅-C₁₀, les radicaux cycloalkyle en C₃-C₈, les radicaux hétérocycloalkyle en C₂-C₈, les atomes d'halogène, les groupes hydroxyle, les groupes oxo, les groupes cyano et les groupes nitro.

21. Procédé selon la revendication 20, dans lequel les radicaux R¹ et R² représentent indépendamment les uns des autres un radical alkyle en C₁-C₆, un radical aryle en C₅-C₁₀, un radical hétéroaryle en C₅-C₁₀, un radical cycloalkyle en C₃-C₈ ou un radical hétérocycloalkyle en C₂-C₈, ou forment ensemble avec l'atome d'azote, auquel ils sont liés, un cycle saturé ou simplement insaturé avec 3 à 8 atomes de carbone, qui peut éventuellement être substitué par un groupe alkyle en C₁-C₆ ou un atome d'halogène et peut contenir, à côté de l'atome d'azote, 1 ou 2 autres hétéroatomes, choisis parmi les atomes de soufre, les atomes d'azote et les atomes d'oxygène.

22. Procédé selon la revendication 20 ou 21, dans lequel le radical HO-A* représente un radical de formule 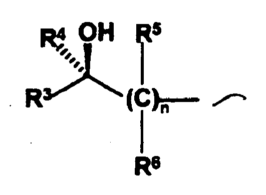 dans laquelle
R³ à R⁶ représentent chaque fois indépendamment les uns des autres des atomes d'hydrogène ou des radicaux hydrocarbure avec 1 à 20 atomes de carbone, qui peuvent présenter chaque fois jusque 4 hétéroatomes, choisis parmi les atomes d'azote, d'oxygène, de soufre et d'halogène, et jusque 5 substituants, choisis parmi les groupes hydroxyle, les groupes oxo, les groupes cyano et les groupes nitro, ou
un ou deux des radicaux R³ à R⁶ forme avec le radical R¹ ou, selon le cas, le radical R² un cycle de 1 à 10 membres saturé ou insaturé, qui contient, outre l'atome d'azote, éventuellement encore 1 à 3 autres hétéroatomes, choisis parmi les atomes d'azote, d'oxygène et de soufre, comme membres de cycle, et qui peut être substitué par jusque 5 substituants, choisis parmi les radicaux alkyle en C₁-C₆, les radicaux alcényle en C₂-C₆, les radicaux alcoxy en C₁-C₆, les radicaux aryle en C₅-C₁₀, les radicaux cycloalkyle en C₃-C₈, les radicaux hétérocycloalkyle en C₂-C₈, les atomes d'halogène, les groupes hydroxyle, les groupes oxo, les groupes cyano et les groupes nitro, et n représente un nombre entier de 1 à 3.

23. Procédé selon la revendication 22, dans lequel les radicaux R⁵ et R⁶ sont choisis, indépendamment l'un de l'autre, parmi l'hydrogène et les radicaux alkyle en C₁-C₆.

24. Procédé selon la revendication 23, dans lequel seulement un des radicaux R⁵ et R⁶ est différent d'un atome d'hydrogène.

25. Procédé selon la revendication 24, dans lequel tous les radicaux R⁵ et R⁶ représentent des atomes d'hydrogène.

26. Procédé selon l'une des revendications 22 à 25, dans lequel l'indice n est 1 ou 2.

27. Procédé selon l'une des revendications 22 à 26, dans lequel le radical R³ représente un radical alkyle en C₁-C₆, aryle en C₅-C₁₀, hétéroaryle en C₅-C₁₀, hétérocyloalkyle en C₂-C₈ ou cycloalkyle en C₃-C₈.

28. Procédé selon la revendication 27, dans lequel le radical R³ représente un radical alkyle en C₁-C₆.

29. Procédé selon l'une des revendications 22 à 28, dans lequel le radical R⁴ représente un atome d'hydrogène.

30. Procédé selon la revendication 22, dans lequel le radical R³ est lié au radical R² en un cycle qui présente 5 à 10 atomes de cycle et qui peut présenter, à côté de l'atome d'azote, auquel le radical R² est lié, encore 1 à 3 autres hétéroatomes, choisis parmi les atomes d'oxygène, d'azote et de soufre, et qui peut être saturé ou une fois ou deux fois insaturé, et qui peut présenter 1 à 3 substituants, choisis parmi les radicaux alkyle en C₁-C₆, les radicaux aryle en C₅-C₁₀, les radicaux hétéroaryle en C₅-C₁₀, les radicaux cycloalkyle en C₃-C₈, les radicaux hétérocycloalkyle en C₂-C₈, et des atomes d'halogène.

31. Procédé selon la revendication 20 ou 21, dans lequel le radical HO-A* représente un radical de formule 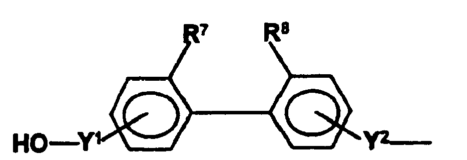 ou 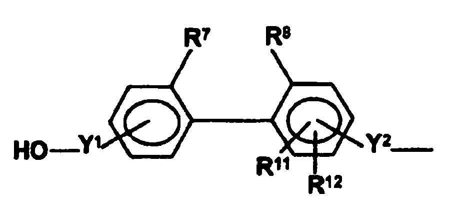 dans laquelle Y¹ représente un radical 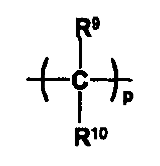 et Y² représente un radical 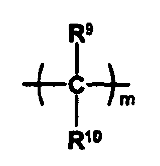 dans lequel R⁹ et R¹⁰ sont choisis indépendamment les uns des autres parmi des atomes d'hydrogène et des radicaux alkyle en C₁-C₆ et les radicaux R⁷ et R⁸ représentent des groupes qui empêchent la libre rotation des deux groupes phényle l'un par rapport à l'autre, R¹¹ et R¹² représentent, indépendamment les uns des autres des atomes d'hydrogène, des radicaux alcoxy en Cₗ-C₄, des atomes d'halogène, des radicaux cyano, des radicaux alcoxycarbonyle en C₁-C₈, des radicaux alkyle en C₁-C₆, ou R¹¹ et R¹² forment, avec le cycle benzène, auquel ils sont liés, une structure cyclique condensée, qui est sélectionnée parmi une structure, 1,3-benzodioxolyle, α-naphtyle, β-naphtyle, tétrahydronaphtyle, benzimidazolyle et benzotriazolyle, m est un nombre entier de 0 à 2 et p représente 1 ou 2.

32. Procédé selon la revendication 31, dans lequel un des radicaux R⁷ et R⁸ est choisi parmi un groupe alkyle en C₁-C₆, un atome d'halogène et un groupe cyano et le deuxième est choisi parmi un groupe alkyle ramifié en C₃-C₆, de préférence un groupe t-butyle, un atome d'halogène et un groupe cyano.

33. Procédé selon la revendication 20, dans lequel le composé de formule (III) 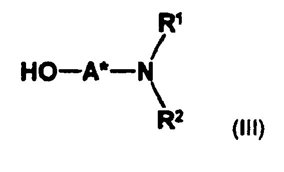 est un composé de formule 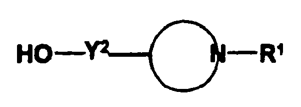 dans laquelle Y² est comme défini à la revendication 31, R¹ est comme défini à la revendication 20 et le groupe 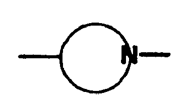 représente un cycle de 5 à 10 membres saturé ou insaturé, qui contient, outre l'atome d'azote, éventuellement encore 1 à 3 autres hétéroatomes, choisis parmi les atomes d'azote, d'oxygène et de soufre, comme membres de cycle, et qui peut être substitué par jusque 5 substituants, choisis parmi les radicaux alkyle en C₁-C₆, les radicaux alcényle en C₂-C₆, les radicaux alcoxy en C₁-C₆, les radicaux aryle en C₅-C₁₀, les radicaux hétéroaryle en C₅-C₁₀, les radicaux cycloalkyle en C₃-C₈, les radicaux hétérocycloalkyle en C₂-C₈, les atomes d'halogène, les groupes hydroxyle, les groupes oxo, les groupes cyano et les groupes nitro.

34. Procédé selon la revendication 33, dans lequel le groupe 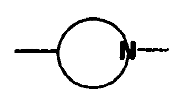 représente un groupe

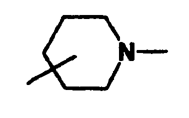

35. Mélange des diastéréomères (IVa) 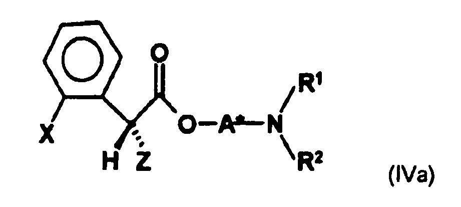 et (IVb) 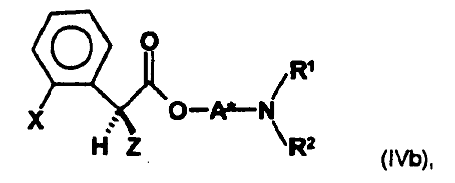 dans lesquelles X et Z sont comme définis à la revendication 1 et A*, R¹ et R² sont comme définis dans une des revendications 20 à 33.

36. Mélange selon la revendication 35, dans lequel le rapport entre le diastéréomère (IVa) et le diastéréomère (IVb) est de 2:1 ou plus grand.

37. Mélange selon la revendication 36, dans lequel le rapport entre le diastéréomère (IVa) et le diastéréomère (IVb) est de 3:1 ou plus grand.

38. Composé de formule (IVa) 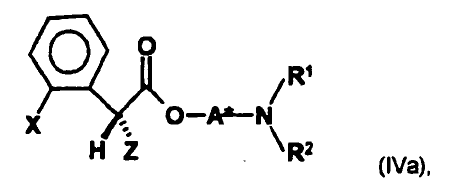 dans lesquelles les radicaux X, Z, A*, R¹ et R² sont comme définis à la revendication 35.

39. Mélange des diastéréomères (VIa) 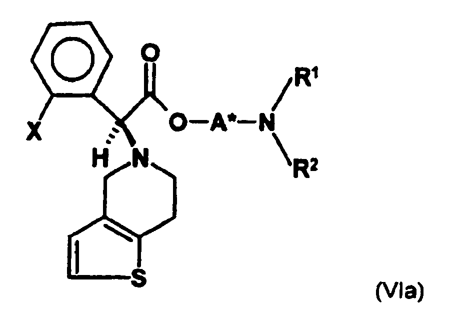 et (VIb) 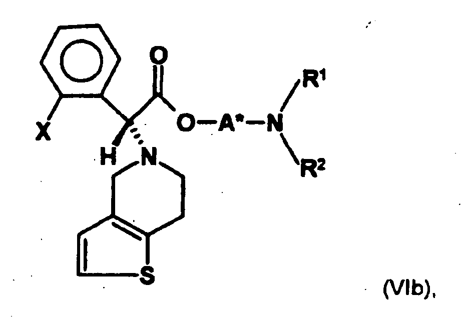 dans lesquelles le radical X est comme défini à la revendication 1 et les radicaux A*, R¹ et R² sont comme définis dans une des revendications 20 à 34 ou des sels de ceux-ci.

40. Mélange selon la revendication 39, dans lequel les diastéréomères sont présents sous forme de sel de maléate, de sel de fumarate ou de sel d'oxalate.

41. Mélange selon la revendication 40, dans lequel le rapport entre le diastéréomère (VIa) et le diastéréomère (VIb) est de 3:1 ou plus grand.

42. Mélange selon la revendication 41, qui contient 98% ou plus de diastéréomère (VIa) et 2% ou moins de diastéréomère (VIb).

43. Composé de formule (VIa) 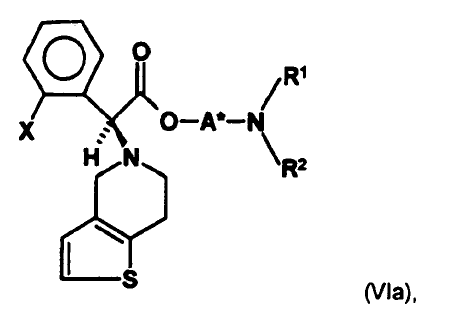 dans laquelle les radicaux X, A*, R¹ et R² sont comme définis à la revendication 39 ou un sel de ceux-ci.

44. Composé selon la revendication 43 sous forme du sel de maléate, du sel de fumarate ou du sel d'oxalate.

45. Mélange des diastéréomères (VIIIa) 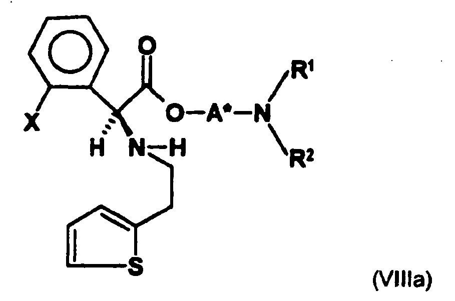 et (VIIIb) 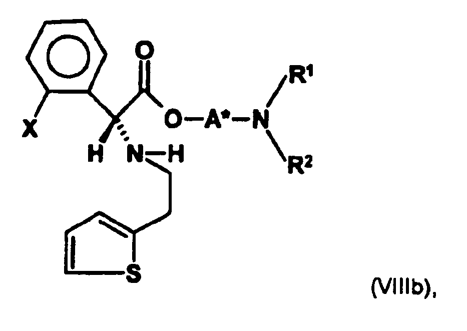 dans lesquelles le radical X est comme défini à la revendication 1 et les radicaux A*, R¹ et R² sont comme définis dans une des revendications 20 à 34 ou des sels de ceux-ci.

46. Mélange selon la revendication 45, dans lequel les diastéréomères sont présents sous forme de sel de maléate, de sel de fumarate ou de sel d'oxalate.

47. Mélange selon la revendication 46, dans lequel le rapport entre les diastéréomères (VIIIa) et (VIIIb) est de 3:1 ou plus grand.

48. Mélange selon la revendication 47, dans lequel le diastéréomère (VIIIa) est présent à 98% ou plus et le diastéréomère (VIIIb) l'est à 2% ou moins.

49. Composé de formule (VIIIa) 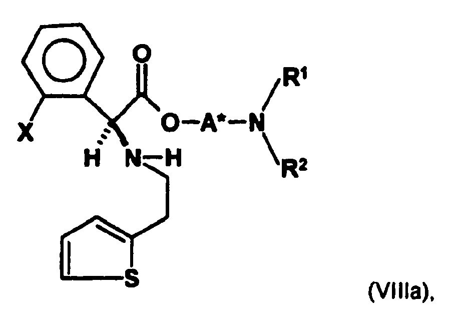 dans laquelle les radicaux X, A*, R¹ et R² sont comme définis à la revendication 45 ou un sel de ceux-ci.

50. Composé selon la revendication 49 sous forme du sel de maléate, du sel de fumarate ou du sel d'oxalate.
